# EUROPEAN PATENT APPLICATION

(11) **EP 4 074 700 A1**
(43) Date of publication of application: **19.10.2022**
(21) Application number: 20900486.0
(22) Date of filing: 09.12.2020
(51) Int. Cl.: C07D 401/04

(54) **HISTONE DEACETYLASE INHIBITOR HAVING NITROGEN-CONTAINING AROMATIC HETEROCYCLIC GROUP**

(30) Priority: 10.12.2019 JP 2019222560
(71) Applicant: Shionogi & Co., Ltd, Osaka-shi, Osaka 541-0045 (JP)
(72) Inventor: ANAN Kosuke, Toyonaka-shi, Osaka 561-0825 (JP); YAMAKAWA Hidekuni, Toyonaka-shi, Osaka 561-0825 (JP); YOSHIHARA Ken, Toyonaka-shi, Osaka 561-0825 (JP); NAKAHARA Kenji, Toyonaka-shi, Osaka 561-0825 (JP); TAKAYA Kenji, Toyonaka-shi, Osaka 561-0825 (JP); MIZOTE Keisuke, Toyonaka-shi, Osaka 561-0825 (JP); KAI Hiroyuki, Toyonaka-shi, Osaka 561-0825 (JP)
(74) Representative: Vossius & Partner Patentanwälte Rechtsanwälte mbB
(86) International application number: PCT/JP2020/045829
(87) International publication number: WO 2021/117759

(57) **Abstract**

A novel compound having HDAC2 inhibitory activity is provided.

A compound represented by Formula (I): 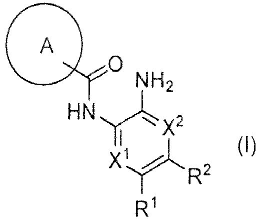 wherein the group represented by Formula: 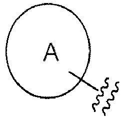 is a group represented by Formula: 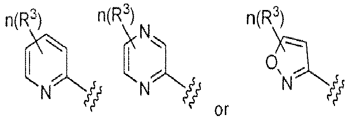 or the like, wherein R³ is each independently amino or the like, and n is an integer of 0 to 4, X¹ is N or the like, X² is N or the like, R⁴ and R⁵ are each independently a hydrogen atom or the like, R¹ is substituted with Substituent group C or unsubstituted non-aromatic carbocyclyl or the like, Substituent group C is halogen or the like, and R² is a hydrogen atom,
or a pharmaceutically acceptable salt thereof.

## Description

### [TECHNICAL FIELD]

The present invention relates to a compound having an HDAC2 inhibitory activity and useful as a therapeutic or prophylactic agent for a disease associated with HDAC2 or a pharmaceutically acceptable salt thereof, and a pharmaceutical composition containing them.

### [BACKGROUND ART]

In recent years, there have been many reports suggesting that the contribution of epigenetic regulation accompanied by DNA methylation and chemical modification of histones is important in the process of synaptic plasticity and memory formation.

Histone acetylation, which is known as a typical example of epigenetic regulation, is regulated by the function of histone acetyltransferase (HAT) and histone deacetylase (HDAC).

There are 18 subtypes of human HDACs, which are roughly classified into 5 groups (class I, class IIa, class IIb, class III, class IV). It is known that HDACs use various proteins such as histones as substrates, and HDACs using histones as substrates are generally considered to negatively regulate the expression of specific gene regions by deacetylation of histones.

It has been suggested that HDACs regulate different gene clusters for each subtype. It is known that HDAC2 belonging to class I is mainly involved in the regulation of genes related to neural function. Non-clinical studies have revealed that inhibition of HDAC2 enhances transcription of genes related to neural function, resulting in enhanced neural function (Non-Patent Document 1).

HDAC2 is also known to be upregulated in patients and model mice with Alzheimer-type dementia. Therefore, it has been suggested that HDAC2 may be involved in the formation of the pathological condition by excessively suppressing gene transcription in the nervous system (Non-Patent Document 2). Furthermore, since it has been reported that specific inhibition of HDAC2 in Alzheimer's disease model mice has an effect of improving impaired cognitive function, HDAC2 is attracting attention as a promising drug discovery target for Alzheimer-type dementia (Non-Patent Document 3). Furthermore, in recent years, there are reports suggesting that HDAC2 is also associated with other neurological disorders such as schizophrenia and depression, and it is expected that HDAC2 inhibitors can be widely applied to neurodegenerative diseases and psychiatric disorders (Non-Patent Document 4).

Most of the HDAC inhibitors currently used clinically are non-selective HDAC inhibitors that are indicated for cancer and inhibit multiple HDAC subtypes. These are known to cause side effects commonly observed with other anticancer agents, including thrombocytopenia. A plurality of nonclinical studies have been conducted to clarify the mechanism of such side effects, and it has been clarified that inhibition of HDAC1 and 2 at the same time causes suppression of cell proliferation (Non-Patent Document 5). The inhibitory effect on cell proliferation by simultaneous inhibition of HDAC1 and 2 is considered to be related to the clinical side effects of known HDAC inhibitors. Therefore, it is required to create a compound that selectively inhibits HDAC2, but it is assumed that HDAC1 and HDAC2 have extremely high amino acid homology, and it is extremely difficult to create a compound that selectively inhibits HDAC2.

Patent Documents 1 to 4, 16, 20 and 21 and Non-Patent Documents 6 to 8 describe HDAC inhibitors, but the compounds substantially disclosed have a structure different from that of the compound of the present invention.

Patent Documents 17 and 18 describe RAF inhibitors having a different mechanism of action from the compounds of the present application.

### [PRIOR ART REFERENCES]

### [Patent Document]

[Patent Document 1] International Publication WO2012/149540 1
[Patent Document 2] International Publication WO2010/065117 2
[Patent Document 3] International Publication WO2017/004522 3
[Patent Document 4] JP2013-170164 4
[Patent Document 5] US Patent Application Publication US2019/0076552 5
[Patent Document 6] International Publication WO2018/098296 6
[Patent Document 7] International Publication WO2017/136451 7
[Patent Document 8] US Patent Publication US9603950 8
[Patent Document 9] International Publication WO2017/007756 9
[Patent Document 10] International Publication WO2017/007755 1 0
[Patent Document 11] International Publication WO2016/057779 1 1
[Patent Document 12] US Patent Application Publication US2014/0128391 1 2
[Patent Document 13] International Publication WO2012/149540 1 3
[Patent Document 14] International Publication WO2009/037001 1 4
[Patent Document 15] International Publication WO2007/118137 1 5
[Patent Document 16] International Publication WO2005/030705 1 6
[Patent Document 17] International Publication WO2005/030704 1 7
[Patent Document 18] International Publication WO2009/006389 1 8
[Patent Document 19] International Publication WO2009/006404 1 9
[Patent Document 20] International Publication WO2020/076951 2 0
[Patent Document 21] International Publication WO2020/068950 2 1

### [Non-Patent Document]

0 0 0 1
[Non-Patent Document 1] The Journal of Neuroscience 2013, Vol. 33, No. 14, pp. 5924-5929 1
[Non-Patent Document 2] Nature 2012, Vol. 483, No. 7388, pp. 222-226 2
[Non-Patent Document 3] Nature 2009, Vol. 459, No. 7243, pp. 55-60 3
[Non-Patent Document 4] Nature Neuroscience 2012, Vol. 15, No. 9, pp. 1245-12544
[Non-Patent Document 5] The EMBO Journal 2010, Vol. 29, No. 15, pp. 2586-2597 5
[Non-Patent Document 6] Chemical Science 2015, Volume 6, pp. 804-815 6
[Non-Patent Document 7] Bioorganic & Medicinal Chemistry Letters 2010, Vol. 20, pp. 3142-3145 7
[Non-Patent Document 8] 6th CSJ Chemistry Festa 2016 Proceedings Challenge intractable diseases with the power of chemistry -Epigenetics Medicinal Chemistry- 8
[Non-Patent Document 9] Cell Reports 2018, Vol. 22, No. 13, pp. 3393-3400 9

### [SUMMARY OF THE INVENTION]

### [PROBLEMS TO BE SOLVED BY THE INVENTION]

An object of the present invention is to provide a compound having HDAC2 inhibitory activity and preferably having a high HDAC2/HDAC1 selectivity and useful as a therapeutic or prophylactic agent for a disease related to HDAC2 or its pharmaceutically acceptable salt, and a pharmaceutical composition comprising thereof.

### [MEANS FOR SOLVING THE PROBLEM]

The present invention relates to the following.
(1) A compound represented by Formula (I): 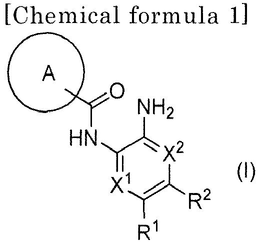 wherein the group represented by Formula: 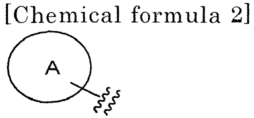 is a group represented by Formula: 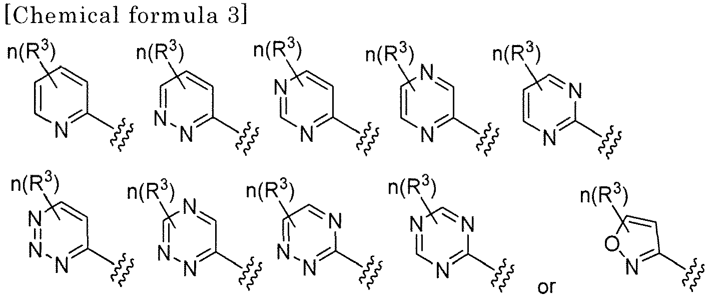 wherein
   R³ is each independently amino, alkylamino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, or substituted with Substituent group A or unsubstituted alkylcarbonyloxy,
   substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl, or
   two R³s may be taken together with an adjacent carbon atom to form non-aromatic carbocyclyl optionally substituted with halogen, non-aromatic heterocyclyl, optionally substituted with halogen, aromatic carbocyclyl optionally substituted with halogen, or aromatic heterocyclyl optionaly substituted with halogen,
   Substituent group A: halogen, cyano, hydroxy, amino, alkylamino, alkyloxy, and non-aromatic carbocyclyl,
   Substituent group B: oxo, halogen, cyano, hydroxy, alkyl, haloalkyl, oxo non-aromatic heterocyclyalkyl, alkenyl, alkynyl, alkyloxy, haloalkyloxy, non-aromatic carbocyclylalkyloxy, amino, and alkylamino, and
   n is an integer from 0 to 4,
   X¹ is N or CR⁴,
   X² is N or CR⁵,
   R⁴ and R⁵ are each independently a hydrogen atom, halogen, alkyl, haloalkyl, alkyloxy or haloalkyloxy,
   R¹ is substituted with Substituent group C or unsubstituted non-aromatic carbocyclyl, substituted with Substituent group C or unsubstituted non-aromatic heterocyclyl, substituted with Substituent group C or unsubstituted aromatic carbocyclyl, or substituted with Substituent group C or unsubstituted aromatic heterocyclyl,
   provided that when R¹ is substituted with Substituent group C or unsubstituted aromatic carbocyclyl, either X¹ or X² is N,
   Substituent group C: halogen, cyano, hydroxy, alkyl, haloalkyl, alkyloxy, haloalkyloxy, amino, and alkylamino, and
   R² is a hydrogen atom, halogen, alkyl, haloalkyl, alkyloxy, or haloalkyloxy,
   when R¹ is substituted with Substituent group C or unsubstituted aromatic carbocyclyl, a substituent on the aromatic carbocyclyl and R² may be taken together to form a substituted or unsubstituted carbocycle or a substituted or unsubstituted heterocycle,
   provided that the following compounds are excluded: 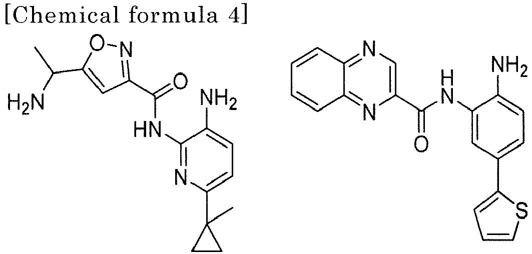 or a pharmaceutically acceptable salt thereof.
   (1') A compound represented by Formula (I): 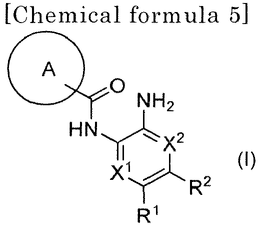 wherein the group represented by Formula: 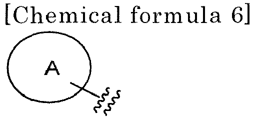 is a group represented by Formula: 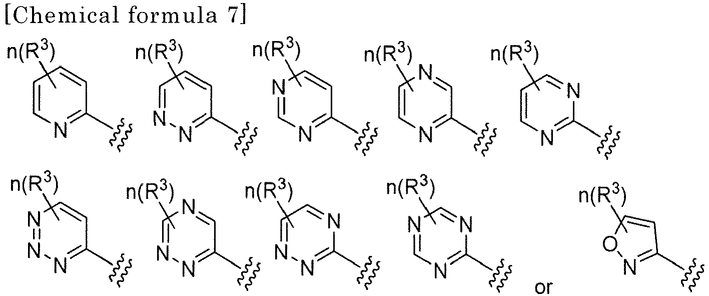 wherein R³ is each independently substituted with Substituent group D or unsubstituted amino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, substituted with Substituent group A or unsubstituted alkyloxycarbonyl, or substituted with Substituent group A or unsubstituted alkylcarbonyloxy,
   substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl,
   n is an integer from 0 to 4, and
   two R³s may be taken together with an adjacent carbon atom to form (a) a non-aromatic carbocycle optionally substituted with halogen, (b) a non-aromatic heterocycle optionally substituted with one or more substituents selected from the group consisting of halogen; alkyl; and oxo, (c) an aromatic carbocycle optionally substituted with halogen, or (d) an aromatic heterocycle optionally substituted with halogen,
   provided that when R¹ is substituted with Substituent group C or unsubstituted thienyl, (i) R³ is not alkyloxyalkyl and hydroxyalkyl, or (ii) two R³s bonded to adjacent carbon atoms together is not taken together to form the rings (a) to (d) above,
   Substituent group A: halogen, cyano, hydroxy, amino, alkylamino, alkyloxy, a non-aromatic carbocycle, and a non-aromatic heterocycle optionally substituted with oxo,
   Substituent group B: oxo, halogen, cyano, hydroxy, alkyl, haloalkyl, oxo non-aromatic heterocyclylalkyl, alkenyl, alkynyl, alkyloxy, haloalkyloxy, non-aromatic carbocyclylalkyloxy, non-aromatic heterocyclyloxy, amino, and alkylamino,
   Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl,
   or the group shown below: 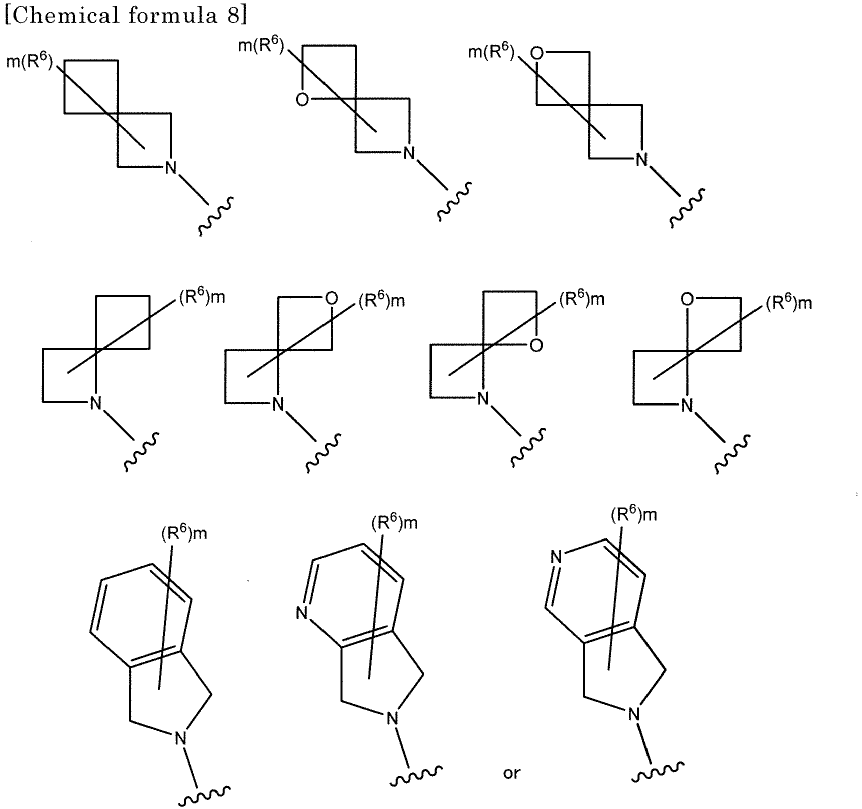 wherein R⁶ is each independently halogen, cyano, hydroxy, alkyl, haloalkyl, alkyloxy, haloalkyloxy, amino, or alkylamino, and
   m is an integer of 0 to 2,
   X¹ is N or CR⁴,
   X² is N or CR⁵,
   R⁴ and R⁵ are each independently a hydrogen atom, halogen, alkyl, haloalkyl, alkyloxy, or haloalkyloxy,
   R¹ is substituted with Substituent group C or unsubstituted non-aromatic carbocyclyl, substituted with Substituent group C or unsubstituted non-aromatic heterocyclyl, substituted with Substituent group C or unsubstituted aromatic carbocyclyl, or substituted with Substituent group C or unsubstituted aromatic heterocyclyl,
   provided that when R¹ is substituted with Substituent group C or unsubstituted aromatic carbocyclyl, either X¹ or X² is N,
   Substituent group C: halogen, cyano, hydroxy, alkyl, haloalkyl, alkyloxy, haloalkyloxy, amino, and alkylamino, and
   R² is a hydrogen atom, halogen, alkyl, haloalkyl, alkyloxy or haloalkyloxy,
   when R¹ is substituted with Substituent group C or unsubstituted aromatic carbocyclyl, a substituent on the aromatic carbocyclyl and R² may be taken together to form a substituted or unsubstituted carbocycle or a substituted or unsubstituted heterocycle,
   provided that the following compound is excluded: 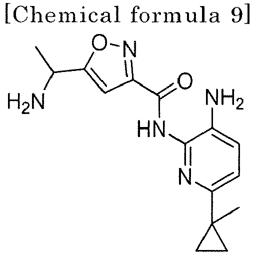 or a pharmaceutically acceptable salt thereof.
   (1") A compound represented by Formula (I): 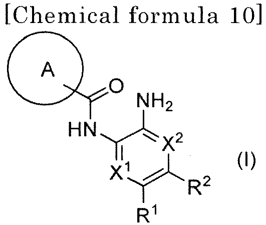 wherein the group represented by Formula: 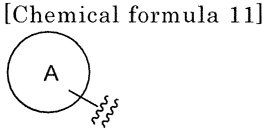 is a group represented by Formula: 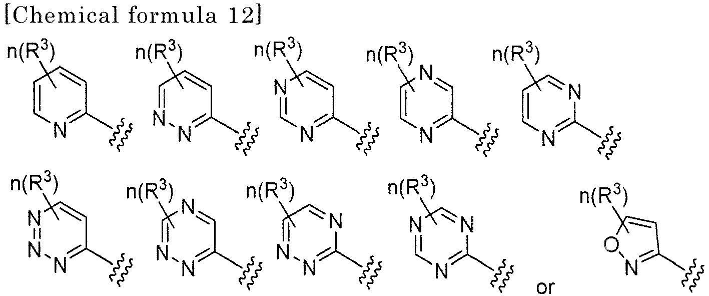 wherein R³ is each independently substituted with Substituent group D or unsubstituted amino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, substituted with Substituent group A or unsubstituted alkyloxycarbonyl, substituted with Substituent group A or unsubstituted alkylcarbonyloxy, substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl,
   n is an integer from 0 to 4, and
   two R³s may be taken together with an adjacent carbon atom to form (a) a non-aromatic carbocycle optionally substituted with halogen, (b) a non-aromatic heterocycle optionally substituted with one or more substituents selected from the group consisting of halogen; alkyl; and oxo, (c) an aromatic carbocycle optionally substituted with halogen, or (d) an aromatic heterocycle optionally substituted with halogen,
   provided that when R¹ is substituted with Substituent group C or unsubstituted thienyl, (i) R³ is not alkyloxyalkyl and hydroxyalkyl, or (ii) two R³s bonded to adjacent carbon atoms is not taken together to form the rings (a) to (d) above,
   Substituent group A: halogen, cyano, hydroxy, amino, alkylamino, alkyloxy, a non-aromatic carbocycle, and a non-aromatic heterocycle optionally substituted with oxo,
   Substituent group B: oxo, halogen, cyano, hydroxy, alkyl, haloalkyl, hydroxy alkyl, oxo non-aromatic heterocyclylalkyl, alkenyl, alkynyl, alkyloxy, haloalkyloxy, non-aromatic carbocyclylalkyloxy, non-aromatic heterocyclyloxy, amino, and alkylamino,
   Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl,
   or the group shown below: 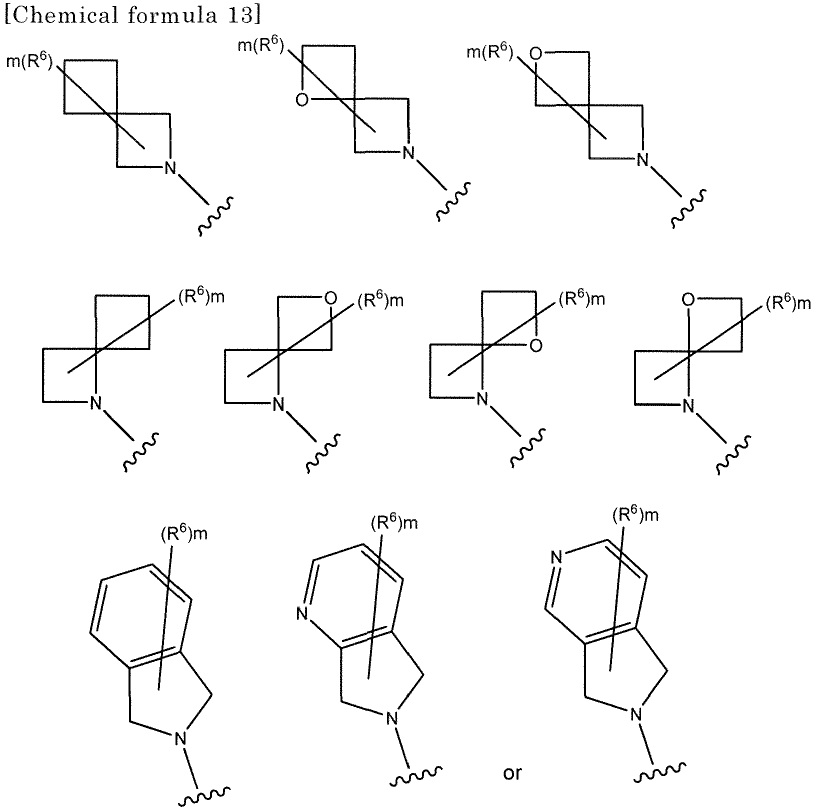 wherein R⁶ is each independently halogen, cyano, hydroxy, alkyl, haloalkyl, alkyloxy, haloalkyloxy, amino, or alkylamino, and
   m is an integer of 0 to 2,
   X¹ is N or CR⁴,
   X² is N or CR⁵,
   R⁴ and R⁵ are each independently a hydrogen atom, halogen, alkyl, haloalkyl, alkyloxy, or haloalkyloxy,
   R¹ is substituted with Substituent group C or unsubstituted non-aromatic carbocyclyl, substituted with Substituent group C or unsubstituted non-aromatic heterocyclyl, substituted with Substituent group C or unsubstituted aromatic carbocyclyl, substituted with Substituent group C or unsubstituted aromatic heterocyclyl,
   provided that when R¹ is substituted with Substituent group C or unsubstituted aromatic carbocyclyl, either X¹ or X² is N,
   Substituent group C: halogen, cyano, hydroxy, alkyl, haloalkyl, alkyloxy, haloalkyloxy, amino and alkylamino, and
   R² is a hydrogen atom, halogen, alkyl, haloalkyl, alkyloxy or haloalkyloxy,
   when R¹ is substituted with Substituent group C or unsubstituted aromatic carbocyclyl, a substituent on the aromatic carbocyclyl and R² may be taken together to form a substituted or unsubstituted carbocycle or a substituted or unsubstituted carbocycle heterocycle,
   provided that the following compound is excluded: 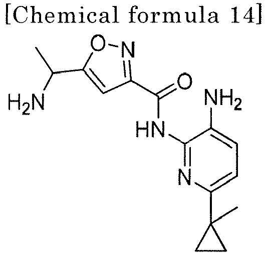 or a pharmaceutically acceptable salt thereof.
(2) The compound according to any one of the above (1), (1'), and (1"), wherein R¹ is substituted with Substituent group C or unsubstituted aromatic carbocyclyl or substituted with Substituent group C or unsubstituted aromatic heterocyclyl,
   or a pharmaceutically acceptable salt thereof.
(3) The compound according to any one of the above (1), (1'), and (1"), wherein R¹ is substituted with Substituent group C or unsubstituted aromatic heterocyclyl,
   or a pharmaceutically acceptable salt thereof.
(4) The compound according to any one of the above (1), (1'), and (1"), wherein R¹ is substituted with Substituent group C or unsubstituted pyridyl or substituted with Substituent group C or unsubstituted imidazolyl,
   or a pharmaceutically acceptable salt thereof.
(5) The compound according to any one of the above (1) to (4), (1'), and (1"), wherein R² is a hydrogen atom,
   or a pharmaceutically acceptable salt thereof.
(6) The compound according to any one of the above (1) to (5), (1'), and (1"), wherein X² is CR⁵,
   or a pharmaceutically acceptable salt thereof.
(7) The compound according to any one of the above (1) to (6), (1'), and (1"), wherein X¹ is CR⁴,
   or a pharmaceutically acceptable salt thereof.
(8) The compound according to any one of the above (1) to (7), (1'), and (1"), wherein each of R⁴ and R⁵ is a hydrogen atom,
   or a pharmaceutically acceptable salt thereof.
(9) The compound according to any one of the above (1) to (8), (1'), and (1"), wherein n is 0 or 1,
   or a pharmaceutically acceptable salt thereof.
(10) The compound according to any one of the above (1) to (8), wherein the group represented by Formula: 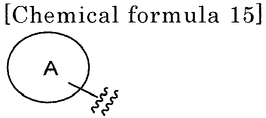 is a group represented by Formula: 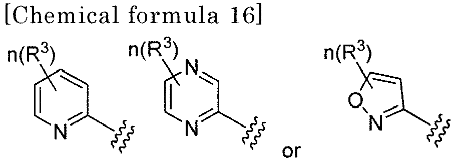 wherein n is an integer of 0 or 1, and the other symbols have the same meaning as the above (1),
   or a pharmaceutically acceptable salt thereof.
   (10') The compound according to any one of the above (1) to (8), and (1'), wherein the group represented by Formula: 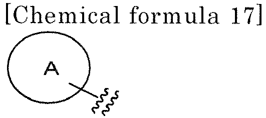 is a group represented by Formula: 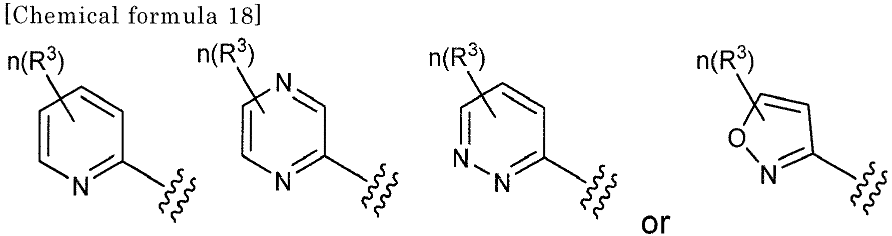 wherein n is an integer of 0 or 1, and the other symbols have the same meaning as the above (1'), or a pharmaceutically acceptable salt thereof.
   (10") The compound according to any one of the above (1) to (8), (1'), and (1"), wherein the group represented by Formula: 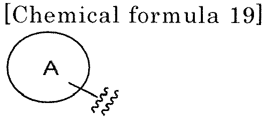 is a group represented by Formula: 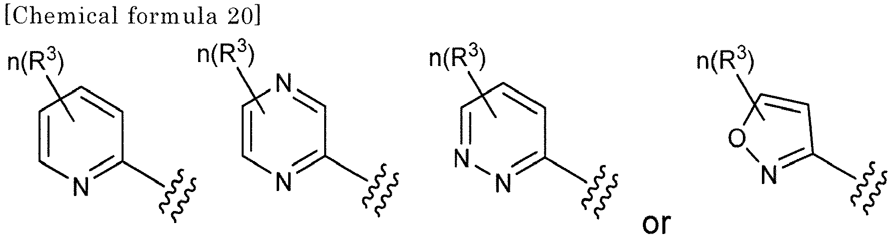 wherein n is an integer of 0 or 1, and the other symbols have the same meaning as the above (1"), or a pharmaceutically acceptable salt thereof.
(11) The compound according to any one of the above (1) to (8), wherein the group represented by Formula: 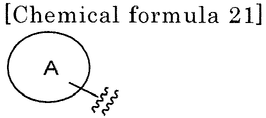 is a group represented by Formula: 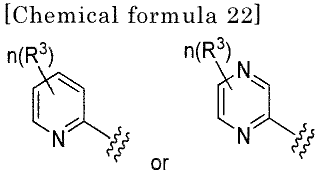 wherein n is an integer of 0 or 1, and the other symbols have the same meaning as the above (1),
   or a pharmaceutically acceptable salt thereof.
   (11') The compound according to any one of the above (1) to (8), and (1'), wherein the group represented by Formula: 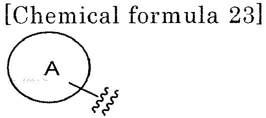 is a group represented by Formula: 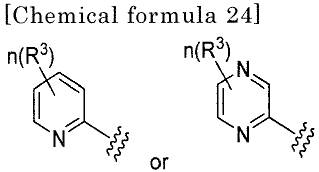 wherein n is an integer of 0 or 1, and the other symbols have the same meaning as the above (1'), or a pharmaceutically acceptable salt thereof.
   (11") The compound according to any one of the above (1) to (8), (1'), and (1"), wherein the group represented by Formula: 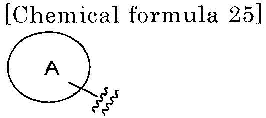 is a group represented by Formula: 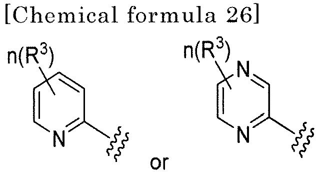 wherein n is an integer of 0 or 1, and the other symbols have the same meaning as the above (1"), or a pharmaceutically acceptable salt thereof.
(12) The compound according to any one of the above (1) to (8), wherein the group represented by Formula: 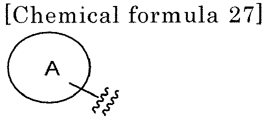 is a group represented by Formula: 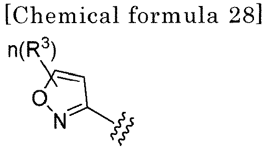 wherein n is an integer of 0 or 1, and the other symbols have the same meaning as the above (1),
   or a pharmaceutically acceptable salt thereof.
   (12') The compound according to any one of the above (1) to (8) and (1'), wherein the group represented by Formula: 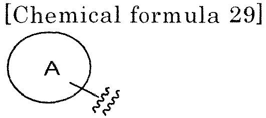 is a group represented by Formula: 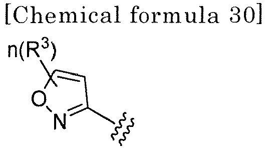 wherein n is an integer of 0 or 1, and the other symbols have the same meaning as the above (1'),
   or a pharmaceutically acceptable salt thereof.
   (12") The compound according to any one of the above (1) to (8), (1') and (1"), wherein the group represented by Formula: 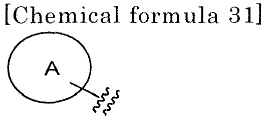 is a group represented by Formula: 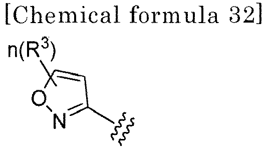 wherein n is an integer of 0 or 1, and the other symbols have the same meaning as the above (1"),
   or a pharmaceutically acceptable salt thereof.
(13) The compound according to any one of the above (1) to (12), (1'), (10') to (12'), (1") and (10") to (12"), wherein R³ is each independently cyano, alkyl, haloalkyl, alkyloxy, haloalkyloxy, alkyloxyalkyloxy, non-aromatic carbocyclyl, non-aromatic heterocyclyl, or alkyl aromatic heterocyclyl,
   or a pharmaceutically acceptable salt thereof.
(14) The compound according to any one of the above (1) to (13), (1'), (10') to (12'), (1") and (10") to (12"), wherein R³ is each independently cyano, alkyl, haloalkyl, alkyloxy, haloalkyloxy, alkyloxyalkyloxy, non-aromatic carbocyclyl, or alkyl aromatic heterocyclyl,
   or a pharmaceutically acceptable salt thereof.
   (14') The compound according to the above (1), (1'), and (1"), wherein the compound is selected from the group consisting of Examples I-030, I-080, 1-130, 1-178, 1-179, I-216, 1-218, 1-221, 1-237, 1-246, 1-249, 1-250, 1-253, 1-262, 1-265, 1-277, 1-280, 1-281, I-285 and I-303
   or a pharmaceutically acceptable salt thereof.
(15) A pharmaceutical composition, comprising the compound according to any one of the above (1) to (14), (1'), (10') to (12'), (14'), (1") and (10") to (12"), or a pharmaceutically acceptable salt thereof.
(16) The pharmaceutical composition according to the above (15), wherein the composition is an HDAC2 inhibitor.
(17) An HDAC2 inhibitor comprising the compound according to any one of the above (1) to (14), (1'), (10') to (12'), (14'), (1") and (10") to (12"), or a pharmaceutically acceptable salt thereof.
(18) A method for treating or preventing a disease related to HDAC2 comprising administering the compound according to any one of the above (1) to (14), (1'), (10') to (12'), (14'), and (10") to (12"), or a pharmaceutically acceptable salt thereof.
(19) Use of the compound according to any one of the above items (1) to (14), (1'), (10') to (12'), (14'), (1") and (10") to (12"), or a pharmaceutically acceptable salt thereof, for the manufacture of a medicament for treating or preventing a disease related to HDAC2.
(20) The compound according to any one of the above (1) to (14), (1'), (10') to (12'), (14'), (1") and (10") to (12"), or a pharmaceutically acceptable salt thereof, for use in treating or preventing a disease related to HDAC2.

### [EFFECT OF THE INVENTION]

The compound according to the present invention has HDAC2 inhibitory activity, and are useful as a therapeutic agent and/or prophylactic agent.

### [MODE FOR CARRYING OUT THE INVENTION]

Terms used in this description are explained below. Each term, unless otherwise indicated, has the same meaning when it is used alone or together with other terms.

The term of "consisting of" means having only components.

The term of "comprising" means not restricting with components and not excluding undescribed factors.

Hereinafter, the present invention is described with reference to embodiments. It should be understood that, throughout the present specification, the expression of a singular form includes the concept of its plural form unless specified otherwise. Accordingly, it should be understood that an article in singular form (for example, in the English language, "a," "an," and "the") includes the concept of its plural form unless specified otherwise. Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a contradiction, the present specification (including definitions) precedes.

The term "halogen" includes a fluorine atom, a chlorine atom, a bromine atom and an iodine atom. A fluorine atom and a chlorine atom are especially preferable.

The term "alkyl" includes a C1 to C15, preferably C1 to C10, more preferably C1 to C6 and further preferably C1 to C4 linear or branched hydrocarbon group. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

A preferred embodiment of "alkyl" is methyl, ethyl, n-propyl, isopropyl, n-butyl, isobutyl, sec-butyl, tert-butyl or n-pentyl. A more preferred embodiment is methyl, ethyl, n-propyl, isopropyl or tert-butyl.

The term "alkenyl" includes a C2 to C15, preferably a C2 to C10, more preferably a C2 to C6 and further preferably a C2 to C4 linear or branched hydrocarbon group having one or more double bond(s) at any position(s). Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl.

A preferred embodiment of "alkenyl" is vinyl, allyl, propenyl, isopropenyl or butenyl.

The term "alkynyl" includes a C2 to C10, preferably a C2 to C8, more preferably a C2 to C6 and further preferably a C2 to C4 linear or branched hydrocarbon group having one or more triple bond(s) at any position(s). Furthermore, it may have double bond(s) at any position(s). Examples include ethynyl, propynyl, butynyl, pentynyl, hexynyl, heptynyl, octynyl, nonynyl, and decynyl.

A preferred embodiment of "alkynyl" is ethynyl, propynyl, butynyl or pentynyl.

The term "aromatic carbocyclyl" means a cyclic aromatic hydrocarbon group which is monocyclic or polycyclic having two or more rings. Examples include phenyl, naphthyl, anthryl, and phenanthryl.

A preferred embodiment of "aromatic carbocyclyl" is phenyl.

The term "aromatic carbocycle" means a ring derived from the above "aromatic carbocyclyl".

A preferred embodiment of "aromatic carbocycle" is a benzene ring. a ring derived from the above "aromatic carbocyclyl group".

The term "non-aromatic carbocyclyl" means a cyclic saturated hydrocarbon group or a cyclic unsaturated non-aromatic hydrocarbon group, which is monocyclic or polycyclic having two or more rings. The "non-aromatic carbocyclyl" which is polycyclic having two or more rings includes a fused ring group wherein a non-aromatic carbocyclyl, which is monocyclic or polycyclic having two or more rings, is fused with a ring of the above "aromatic carbocyclyl".

In addition, examples of the "non-aromatic carbocyclyl" also include a group having a bridge or a group to form a spiro ring as follows:

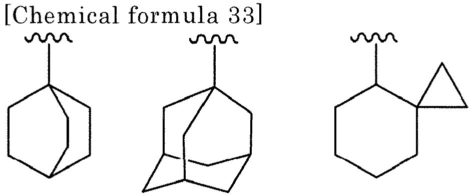

The non-aromatic carbocyclyl which is monocyclic is preferably C3 to C16, more preferably C3 to C12 and further preferably C4 to C8 carbocyclyl. Examples include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, and cyclohexadienyl.

The non-aromatic carbocyclyl which is polycyclic having two or more rings is preferably C8 to C20, more preferably C8 to C16 Examples include indanyl, indenyl, acenaphthyl, tetrahydronaphthyl, and fluorenyl.

The term "non-aromatic carbocycle" means a ring derived from the above "non-aromatic carbocyclyl".

The term "aromatic heterocyclyl" means an aromatic cyclyl, which is monocyclic or polycyclic having two or more rings, containing one or more, same or different heteroatom(s) selected independently from O, S and N.

The aromatic heterocyclyl, which is polycyclic having two or more rings, includes a fused ring group wherein an aromatic heterocyclyl, which is monocyclic or polycyclic having two or more rings, is fused with a ring of the above "aromatic carbocyclyl", the bond may be held in any ring.

The aromatic heterocyclyl, which is monocyclic, is preferably a 5- to 8-membered ring and more preferably a 5- to 6- membered ring. Examples of the 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of the 6-membered aromatic heterocyclyl include pyridyl, pyridazinyl, pyrimidinyl, pyrazinyl, triazinyl. The aromatic heterocyclyl, which is bicyclic, is preferably a 8- to 10-membered ring and more preferably a 9- to 10-membered ring. Examples of aromatic heterocyclyl, which is bicyclic, include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl.

The aromatic heterocyclyl, which is polycyclic having three or more rings, is preferably a 13- to 15-membered ring. Examples of aromatic heterocyclyl, which is polycyclic having three or more rings, include carbazolyl, acridinyl, xanthenyl, phenothiazinyl, phenoxathiinyl, phenoxazinyl, and dibenzofuryl.

The term "aromatic heterocycle" means a ring derived from the above "aromatic heterocyclyl".

The aromatic heterocycle, which is monocyclic, is preferably a 5- to 8-membered ring and more preferably a 5- to 6- membered ring. Examples of the 5-membered aromatic heterocycle include a pyrroline ring, an imidazoline ring, a pyrazoline ring, a triazole ring, a tetrazole ring, a furan, a thiophene ring, an isoxazole ring, an oxazole ring, an oxadiazole ring, an isothiazole ring, a thiazole ring, and a thiadiazole ring. Examples of the 6-membered aromatic heterocycle include a pyridine ring, a pyridazine ring, a pyrimidine ring, a pyrazine ring, a triazine ring. The aromatic heterocycle, which is bicyclic, is preferably a 8- to 10-membered ring and more preferably a 9- to 10- membered ring. Examples of aromatic heterocyclyl, which is bicyclic, include an indole ring, an isoindole ring, an indazole ring, an indolizine ring, a quinoline ring, an isoquinoline ring, a cinnoline ring, a phthalazine ring, a quinazoline ring, a naphthyridine ring, a quinoxaline ring, a purine ring, a pteridine ring, a benzimidazole ring, a benzisoxazole ring, a benzoxazole ring, a benzoxadiazole ring, a benzisothiazole ring, a benzothiazole ring, a benzothiadiazole ring, a benzofuran ring, an isobenzofuran ring, a benzothiophene ring, a benzotriazole ring, an imidazopyridine ring, a triazolopyridine ring, an imidazothiazole ring, a pyrazinopyridazine ring, an oxazolopyridine ring, and athiazolopyridine ring.

The aromatic heterocyclyl, which is polycyclic having three or more rings, is preferably a 13- to 15-membered ring. Examples of aromatic heterocyclyl, which is polycyclic having three or more rings, include a carbazole ring, an acridine ring, a xanthene ring, phenothiazine ring, a phenoxathiine ring, a phenoxazine ring, and a dibenzofuran ring.

As the "aromatic heterocyclyl" in R¹, pyridyl, imidazolyl, pyrazolyl, oxazolyl, oxadiazolyl, triazolyl, thiazolyl, and fryl are preferable, and pyridyl, imidazolyl, and pyrazolyl are more preferable.

The term "non-aromatic heterocyclyl" means a non-aromatic cyclyl, which is monocyclic or polycyclic having two or more rings, containing one or more, same or different heteroatom(s) selected independently from O, S and N. The "non-aromatic heterocyclyl", which is polycyclic having two or more rings, includes a non-aromatic heterocyclyl fused with a ring of the above "aromatic carbocyclyl", "non-aromatic carbocyclyl" and/or "aromatic heterocyclyl", and further includes a non-aromatic carbocyclyl, which is monocyclic or polycyclic having two or more rings, fused with a ring of the above "aromatic heterocyclyl", the bond may be held in any ring.

In addition, examples of the "non-aromatic heterocyclyl" also include a group having a bridge or a group to form a spiro ring as follows:

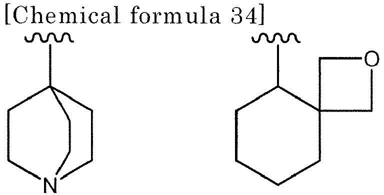

The non-aromatic heterocyclyl, which is monocyclic, is preferably a 3- to 8-membered and more preferably a 5- to 6- membered ring. Examples of the 3-membered non-aromatic heterocyclyl include thiiranyl, oxiranyl and aziridinyl. Examples of the 4-membered non-aromatic heterocyclyl include oxetanyl and azetidinyl. Examples of the 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, and thiolanyl. Examples of the 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydrooxazinyl, tetrahydropyridazinyl, hexahydropyrimidinyl, dioxazinyl, thiinyl, and thiazinyl. Examples of the 7-membered non-aromatic heterocyclyl include hexahydroazepinyl, tetrahydrodiazepinyl, and oxepanyl.

The non-aromatic heterocyclyl, which is polycyclic having two or more rings, is preferably a 8- to 20-membered and more preferably a 8- to 10- membered ring. Examples of non-aromatic heterocyclyl, which is polycyclic having two or more rings, include indolinyl, isoindolinyl, chromanyl, and isochromanyl.

The term "non-aromatic heterocycle" means a ring derived from the above "non-aromatic heterocyclyl".

The alkyl moiety in "alkyloxy", "haloalkyloxy", "alkylcarbonyloxy", "alkylcarbonyl", "alkyloxycarbonyl", "alkylsulfanyl", "alkylsulfinyl", "alkylsulfonyl", "alkyloxyalkyloxy", "alkyloxyalkyl", and the like is synonymous with the above "alkyl".

The alkenyl moiety in "alkenyloxy", "alkenylcarbonyloxy", "alkenylcarbonyl", "alkenyloxycarbonyl", "alkenylsulfanyl", "alkenylsulfinyl", "alkenylsulfonyl", and the like are synonymous with the above "alkenyl".

The alkynyl moiety in "alkynyloxy", "alkynylcarbonyloxy", "alkynylcarbonyl", "alkynyloxycarbonyl", "alkynylsulfanyl", "alkynylsulfinyl", "alkynylsulfonyl", and the like is synonymous with the above "alkynyl".

0 0 0 2

In the present specification, "substituted with Substituent group A or unsubstituted" means "may be substituted with one or more groups selected from the substituent group A". The same applies to the Substituent groups B, C, D and the like.

When "non-aromatic carbocycle", "non-aromatic heterocycle", "non-aromatic carbocyclyl " and "non-aromatic heterocyclyl" are substituted with "oxo", it means a ring in which two hydrogen atoms on a carbon atom are substituted as follows.

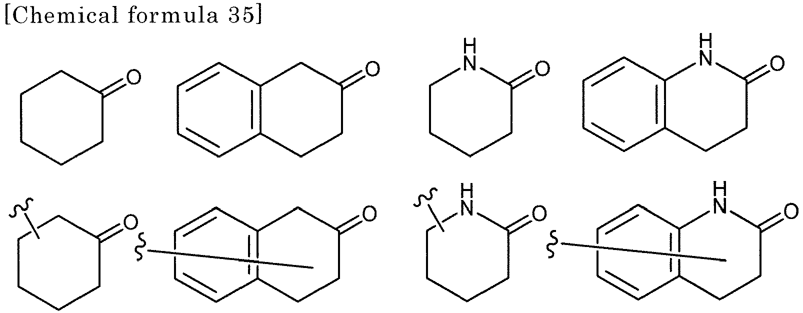

In the compound represented by the Formula (I), preferred embodiments of the group represented by Formula: 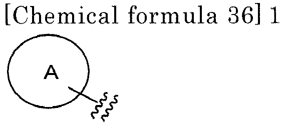 R¹, R², R³, R⁴, R⁵, n, X¹ and X² are shown below. The embodiment of compounds represented by Formula (I) includes the compounds indicated by all possible combination of the specific examples shown below.

The group represented by Formula: 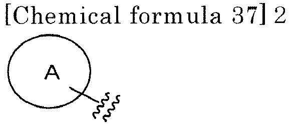 is a group represented by Formula: 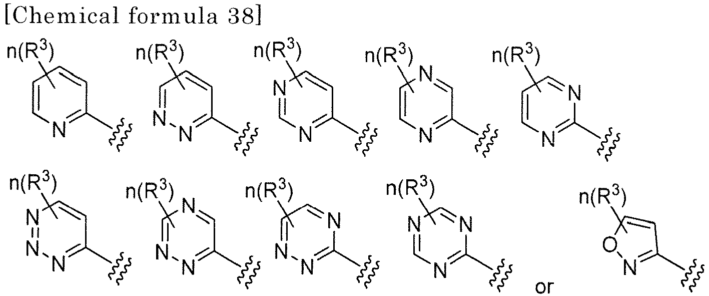 (referred to as A-1).

The group represented by Formula: 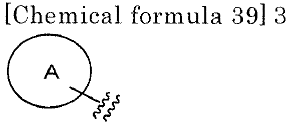 is a group represented by Formula: 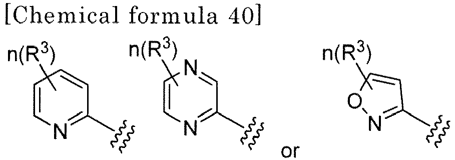 (referred to as A-2).

The group represented by Formula: 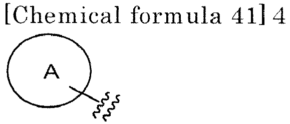 is a group represented by Formula: 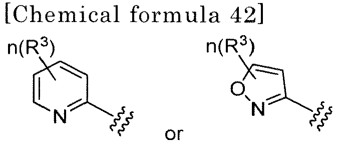 (referred to as A-3).

The group represented by Formula: 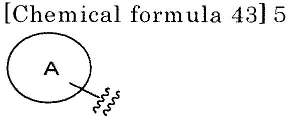 is a group represented by Formula: 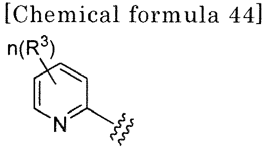 (referred to as A-4).

The group represented by Formula: 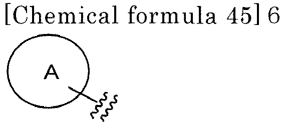 is a group represented by Formula: 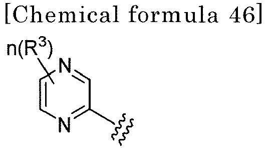 (referred to as A-5).

The group represented by Formula: 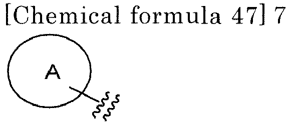 is a group represented by Formula: 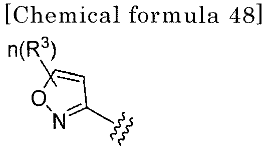 (referred to as A-6).

The group represented by Formula: 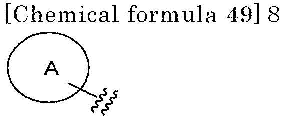 is a group represented by Formula: 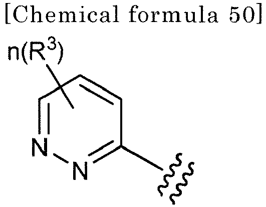 (referred to as A-7).

The group represented by Formula: 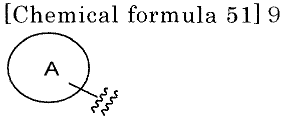 is a group represented by Formula: 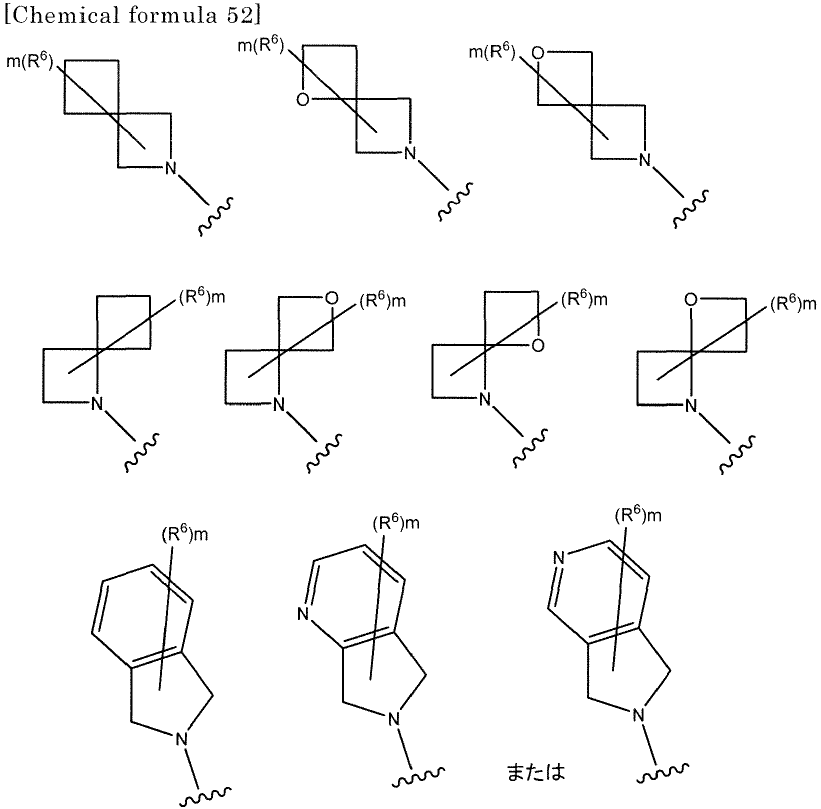 wherein R⁶ is each independently halogen, cyano, hydroxy, alkyl, haloalkyl, alkyloxy, haloalkyloxy, amino, or alkylamino, and
m is an integer of 0 to 2 (referred to as A-8).

R³ is each independently amino, alkylamino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, or substituted with Substituent group A or unsubstituted alkylcarbonyloxy,
substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl, or
two R³s may be taken together with an adjacent carbon atom to form non-aromatic carbocyclyl optionally substituted with halogen, non-aromatic heterocyclyl optionally substituted with halogen, aromatic carbocyclyl optionally substituted with halogen, and aromatic heterocyclyl optionally substituted with halogen,
Substituent group A: halogen, cyano, hydroxy, amino, alkylamino, alkyloxy and non-aromatic carbocyclyl,
Substituent group B: oxo, halogen, cyano, hydroxy, alkyl, haloalkyl, oxo non-aromatic heterocyclylalkyl, alkenyl, alkynyl, alkyloxy, haloalkyloxy, non-aromatic carbocyclylalkyloxy, amino, and alkylamino (referred to as B-1).

R³ is each independently amino, alkylamino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, or substituted with Substituent group A or unsubstituted alkylcarbonyloxy,
substituted with Substituent group B or unsubstituted monocyclic non-aromatic carbocyclyl, substituted with Substituent group B or unsubstituted morpholinyl, substituted with Substituent group B or unsubstituted monocyclic aromatic carbocyclyl, or substituted with Substituent group B or unsubstituted monocyclic aromatic heterocyclyl,
Substituent group A: halogen, cyano, hydroxy, amino, alkylamino, alkyloxy, and non-aromatic carbocyclyl,
Substituent group B: oxo, halogen, cyano, hydroxy, alkyl, haloalkyl, oxo non-aromatic heterocyclylalkyl, alkenyl, alkynyl, alkyloxy, haloalkyloxy, non-aromatic carbocyclylalkyloxy, amino, and alkylamino (referred to as B-2).

R³ is each independently amino, alkylamino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, or substituted with Substituent group A or unsubstituted alkylcarbonyloxy,
substituted with Substituent group B or unsubstituted monocyclic non-aromatic carbocyclyl, substituted with Substituent group B or unsubstituted morpholinyl, substituted with Substituent group B or unsubstituted monocyclic aromatic carbocyclyl, or substituted with Substituent group B or unsubstituted monocyclic aromatic heterocyclyl,
Substituent group A: halogen, cyano, hydroxy, amino, alkylamino, alkyloxy, and non-aromatic carbocyclyl,
Substituent group B: oxo, halogen, cyano, hydroxy, alkyl, haloalkyl, oxo non-aromatic heterocyclylalkyl, alkenyl, alkynyl, alkyloxy, haloalkyloxy, non-aromatic carbocyclylalkyloxy, amino, and alkylamino (referred to as B-3).

R³ is each independently cyano, alkyl, haloalkyl, alkyloxy, haloalkyloxy, alkyloxyalkyloxy, non-aromatic carbocyclyl, non-aromatic heterocyclyl, or alkyl aromatic heterocyclyl (referred to as B-4).

R³ is each independently cyano, alkyl, haloalkyl, alkyloxy, haloalkyloxy, alkyloxyalkyloxy, non-aromatic carbocyclyl, or alkyl aromatic heterocyclyl (referred to as B-5).

R³ is each independently substituted with Substituent group D or unsubstituted amino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, substituted with Substituent group A or unsubstituted alkyloxycarbonyl, or substituted with Substituent group A or unsubstituted alkylcarbonyloxy,
substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl,
Substituent group A: halogen, cyano, hydroxy, amino, alkylamino, alkyloxy, non-aromatic carbocyclyl, and non-aromatic heterocyclyl optionally substituted with oxo,
Substituent group B: oxo, halogen, cyano, hydroxy, alkyl, haloalkyl, hydroxyalkyl, oxo non-aromatic heterocyclylalkyl, alkenyl, alkynyl, alkyloxy, haloalkyloxy, non-aromatic carbocyclylalkyloxy, non-aromatic heterocyclyloxy, amino and alkylamino,
Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl, and alkylsulfonyl (referred to as B-6).

n is an integer from 0 to 4 (referred to as C-1).

n is 0 or 1 (referred to as C-2).

n is 2 (referred to as C-3).

X¹ is N or CR⁴ (referred to as D-1).

X¹ is CR⁴ (referred to as D-2).

X¹ is N (referred to as D-3).

X² is N or CR⁵ (referred to as E-1).

X² is CR⁵ (referred to as E-2).

X² is N (referred to as E-3).

R⁴ and R⁵ are each independently a hydrogen atom, halogen, alkyl, haloalkyl, alkyloxy or haloalkyloxy (referred to as F-1).

R⁴ and R⁵ are each independently a hydrogen atom, halogen or alkyl (referred to as F-2).

R⁴ and R⁵ are each independently a hydrogen atom (referred to as F-3).

R¹ is substituted with Substituent group C or unsubstituted non-aromatic carbocyclyl, substituted with Substituent group C or unsubstituted non-aromatic heterocyclyl, substituted with Substituent group C or unsubstituted aromatic carbocyclyl, or substituted with Substituent group C or unsubstituted aromatic heterocyclyl,
provided that when R¹ is substituted with Substituent group C or unsubstituted aromatic carbocyclyl, either X¹ or X² is N, Substituent group C: halogen, cyano, hydroxy, alkyl, haloalkyl, alkyloxy, haloalkyloxy, amino, and alkylamino (referred to as G-1).
R¹ is substituted with Substituent group C or unsubstituted non-aromatic heterocyclyl, substituted with Substituent group C or unsubstituted aromatic carbocyclyl, or substituted with Substituent group C or unsubstituted aromatic heterocyclyl,
provided that when R¹ is substituted with Substituent group C or unsubstituted aromatic carbocyclyl, either X¹ or X² is N, Substituent group C: halogen, cyano, alkyl, and haloalkyl (referred to as G-2).
R¹ is substituted with Substituent group C or unsubstituted aromatic carbocyclyl, or substituted with Substituent group C or unsubstituted aromatic heterocyclyl,
provided that when R¹ is substituted with Substituent group C or unsubstituted aromatic carbocyclyl, either X¹ or X² is N, Substituent group C: halogen, cyano, alkyl, and haloalkyl (referred to as G-3).
R¹ is substituted with Substituent group C or unsubstituted aromatic carbocyclyl,
provided that when R¹ is substituted with Substituent group C or unsubstituted aromatic carbocyclyl, either X¹ or X² is N,

Substituent group C: halogen, cyano, alkyl, and haloalkyl (referred to as G-4).
   R¹ is substituted with Substituent group C or unsubstituted aromatic heterocyclyl,
Substituent group C: halogen, cyano, alkyl, and haloalkyl (referred to as G-5).
   R¹ is substituted with Substituent group C or unsubstituted 6-membered aromatic heterocyclyl,
Substituent group C: halogen, cyano, alkyl, and haloalkyl (referred to as G-6).
   R¹ is substituted with Substituent group C or unsubstituted 5-membered aromatic heterocyclyl,
Substituent group C: halogen, cyano, alkyl, and haloalkyl (referred to as G-7).
   R¹ is substituted with Substituent group C or unsubstituted pyridyl, substituted with Substituent group C or unsubstituted imidazolyl,
Substituent group C: halogen, cyano, alkyl, and haloalkyl (referred to as G-8).
   R¹ is substituted with Substituent group C or unsubstituted pyridyl,
Substituent group C: halogen, cyano, alkyl, and haloalkyl (referred to as G-9).
   R¹ is substituted with Substituent group C or unsubstituted imidazolyl, Substituent group C: halogen, cyano, alkyl, and haloalkyl (referred to as G-10).

R² is a hydrogen atom, halogen, alkyl, haloalkyl, alkyloxy, or haloalkyloxy (referred to as H-1).

R² is a hydrogen atom, halogen, alkyl, or haloalkyl (referred to as H-2).

R² is a hydrogen atom, or halogen (referred to as H-3).

R² is a hydrogen atom (referred to as H-4).

When R¹ is substituted or unsubstituted aromatic carbocyclyl, a substituent on the aromatic carbocyclyl and R² may be taken together to form a substituted or unsubstituted carbocycle, or a substituted or unsubstituted heterocycle (referred to as I-1).

When R¹ is substituted or unsubstituted aromatic carbocyclyl, a substituent on the aromatic carbocyclyl and R² are may be taken together to form a substituted or unsubstituted carbocycle (referred to as I-2).

When R¹ is substituted or unsubstituted aromatic carbocyclyl, a substituent on the aromatic carbocyclyl and R² are may be taken together to form a substituted or unsubstituted heterocycle (referred to as I-3).

In particular, the following embodiments are preferable.
(i) A compound represented by Formula (I): 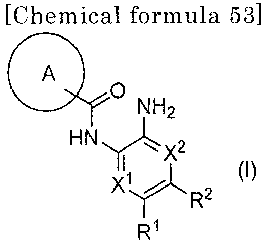 wherein the group represented by Formula: 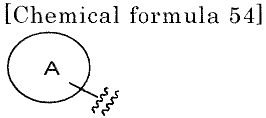 is a group represented by Formula: 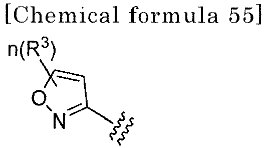 wherein
   R³ is each independently aromatic heterocyclyl substituted with alkyl, and n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted aromatic heterocyclyl, and
   R² is a hydrogen atom,
   or a pharmaceutically acceptable salt thereof.
   (i') A compound represented by Formula (I): 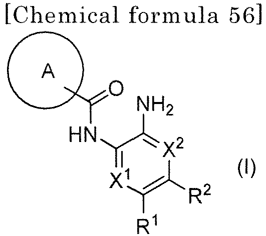 wherein the group represented by Formula: 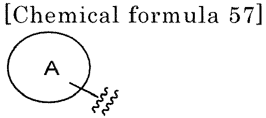 is a group represented by Formula: 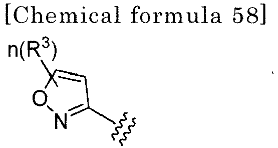 wherein
   R³ is each independently aromatic heterocyclyl substituted with alkyl, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl or substituted with Substituent group C1 or unsubstituted pyrazolyl, and
   R² is a hydrogen atom,
   or a pharmaceutically acceptable salt thereof.
(ii) A compound represented by Formula (I): 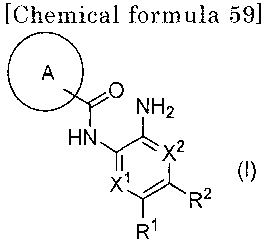 wherein the group represented by Formula: 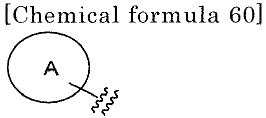 is a group represented by Formula: 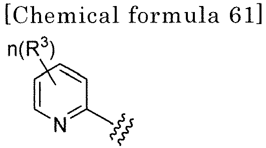 wherein
   R³ is each independently cyano, alkyl, alkyloxy, alkyloxyalkyloxy, cycloalkyl, morpholinyl, aromatic heterocyclyl substituted with alkyl, or alkylamino, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted aromatic heterocyclyl, and
   R² is a hydrogen atom,
   or a pharmaceutically acceptable salt thereof.
   (ii') A compound represented by Formula (I): 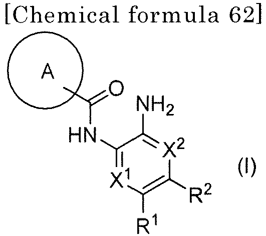 wherein the group represented by Formula: 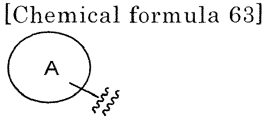 is a group represented by Formula: 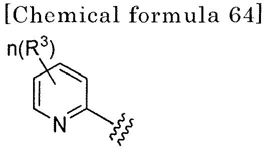 wherein
   R³ is each independently cyano, alkyl, alkyloxy, alkyloxyalkyloxy, cycloalkyl, morpholinyl, aromatic heterocyclyl substituted with alkyl, or alkylamino, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl, or substituted with Substituent group C1 or unsubstituted pyrazolyl, and
   R² is a hydrogen atom,
   or a pharmaceutically acceptable salt thereof.
(iii) A compound represented by Formula (I): 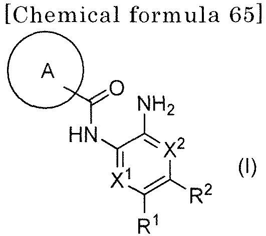 wherein the group represented by Formula: 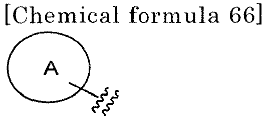 is a group represented by Formula: 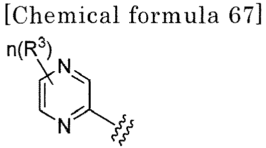 wherein
   R³ is each independently alkyl, haloalkyl, alkyloxy, alkyloxyalkyloxy, haloalkyloxy, cycloalkyl, substituted with alkyl or unsubstituted aromatic heterocyclyl, or alkylamino, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted aromatic heterocyclyl, and
   R² is a hydrogen atom,
   or a pharmaceutically acceptable salt thereof.
   (iii') A compound represented by Formula (I): 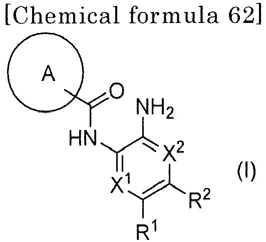 wherein the group represented by Formula: 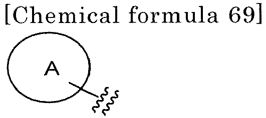 is a group represented by Formula: 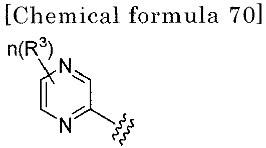 wherein
   R³ is each independently alkyl, haloalkyl, alkyloxy, alkyloxyalkyloxy, haloalkyloxy, cycloalkyl, substituted with alkyl or unsubstituted aromatic heterocyclyl, or alkylamino, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl, or substituted with Substituent group C1 or unsubstituted pyrazolyl, and
   R² is a hydrogen atom,
   or a pharmaceutically acceptable salt thereof.
(iv) A compound represented by Formula (I): 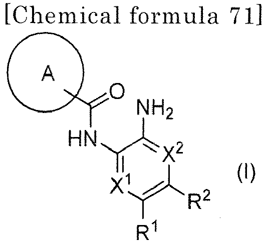 wherein the group represented by Formula: 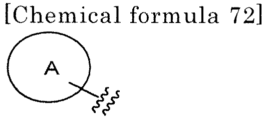 is a group represented by Formula: 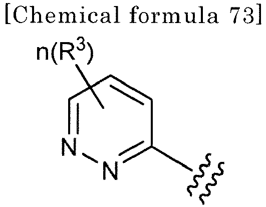 wherein
   R³ is each independently substituted with Substituent group D or unsubstituted amino, alkyl, aromatic hetelocyclylalkyl, alkyloxyalkyl, alkyloxy, non-aromatic heterocyclyl, substituted with alkyl or unsubstituted pyrazolyl, or non-aromatic carbocyclyl,
      Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl, substituted with Substituent group C1 or unsubstituted pyrazolyl, substituted with Substituent group C1 or unsubstituted pyrrolyl, or substituted with Substituent group C1 or unsubstituted phenyl, and
   R² is a hydrogen atom,
   or a pharmaceutically acceptable salt thereof.
(v) A compound represented by Formula (I): 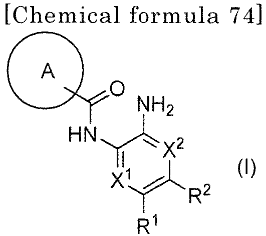 wherein the group represented by Formula: 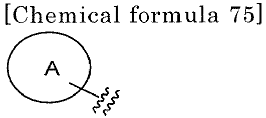 is a group represented by Formula: 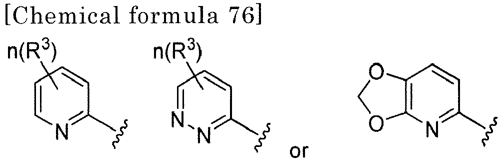 wherein
   R³ is each independently substituted with Substituent group D or unsubstituted amino, alkyl, aromatic hetelocyclylalkyl, alkyloxyalkyl, alkyloxy, non-aromatic heterocyclyl, substituted with alkyl or unsubstituted pyrazolyl, or non-aromatic carbocyclyl,
      Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl, substituted with Substituent group C1 or unsubstituted pyrazolyl, substituted with Substituent group C1 or unsubstituted pyrrolyl, or substituted with Substituent group C1 or unsubstituted phenyl, and
   R² is a hydrogen atom,
   or a pharmaceutically acceptable salt thereof.
   (v') A compound represented by Formula (I): 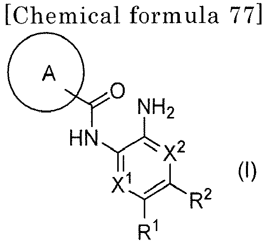 wherein the group represented by Formula: 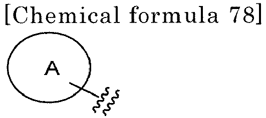 is a group represented by Formula: 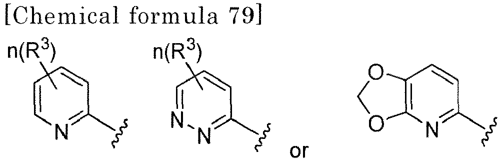 wherein
   R³ is each independently substituted with Substituent group D or unsubstituted amino, alkyl, aromatic hetelocyclylalkyl, alkyloxyalkyl, alkyloxy, non-aromatic heterocyclyl, substituted with alkyl or unsubstituted pyrazolyl, or non-aromatic carbocyclyl,
      Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl, and
   n is an integer of 0 or 1,
   X¹ is CR⁴,
   X² is CR⁵,
   R⁴ and R⁵ are hydrogen atoms,
   R¹ is substituted with Substituent group C1 (Substituent group C1: halogen and alkyl) or unsubstituted pyridyl, or substituted with Substituent group C1 or unsubstituted pyrazolyl, and
   R² is a hydrogen atom,
   or a pharmaceutically acceptable salt thereof.

The compounds of Formula (I) are not limited to specific isomers but include all possible isomers (e.g., keto-enol isomers, imine-enamine isomers, diastereoisomers, enantiomers, or rotamers), racemates or mixtures thereof.

One or more hydrogen, carbon and/or other atom(s) in the compounds of Formula (I) may be replaced with isotopes of hydrogen, carbon and/or other atoms respectively. Examples of isotopes include hydrogen, carbon, nitrogen, oxygen, phosphorus, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl respectively. The compounds of Formula (I) include the compounds replaced with these isotopes. The compounds replaced with the above isotopes are useful as medicines and include all of radiolabeled compounds of the compound of Formula (I). A "method of radiolabeling" in the manufacture of the "radiolabeled compounds" is encompassed by the present invention, and the "radiolabeled compounds" are useful for studies on metabolized drug pharmacokinetics, studies on binding assay and/or diagnostic tools.

A radiolabeled compound of the compounds of Formula (I) can be prepared using well-known methods in this field of the invention. For example, a tritium-labeled compound of Formula (I) can be prepared by introducing a tritium to a certain compound of Formula (I) through a catalytic dehalogenation reaction using a tritium. This method comprises reacting an appropriately-halogenated precursor of the compound of Formula (I) with tritium gas in the presence of an appropriate catalyst, such as Pd/C, and in the presence or absent of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a raw material having ¹⁴C.

The pharmaceutically acceptable salts of the compounds of Formula (I) include, for example, salts with alkaline metal (e.g., lithium, sodium, or potassium), alkaline earth metal (e.g., calcium or barium), magnesium, transition metal (e.g., zinc or iron), ammonia, organic bases (e.g., trimethylamine, triethylamine, dicyclohexylamine, ethanolamine, diethanolamine, triethanolamine, meglumine, ethylenediamine, pyridine, picoline, or quinoline), or amino acids, or salts with inorganic acids (e.g., hydrochloric acid, sulfuric acid, nitric acid, carbonic acid, hydrobromic acid, phosphoric acid, or hydroiodic acid) or organic acids (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, or ethanesulfonic acid). Especially, salts with hydrochloric acid, sulfuric acid, phosphoric acid, tartaric acid, methanesulfonic acid and the like are included. These salts can be formed by the usual methods.

The compounds of Formula (I) or pharmaceutically acceptable salts thereof may form solvates (e.g., hydrates), co-crystal and/or crystal polymorphs. The present invention encompasses those various solvates, co-crystal and crystal polymorphs. "Solvates" may be those wherein any numbers of solvent molecules (e.g., water molecules) are coordinated with the compounds of Formula (I). When the compounds of Formula (I) or pharmaceutically acceptable salts thereof are allowed to stand in the atmosphere, the compounds may absorb water, resulting in attachment of adsorbed water or formation of hydrates. Recrystallization of the compounds of Formula (I) or pharmaceutically acceptable salts thereof may produce crystal polymorphs. "Co-crystal" means that a compound of Formula (I) or a salt thereof and a counter molecule exist in the same crystal lattice, and it can include any number of counter molecules.

The compounds of Formula (I) or pharmaceutically acceptable salts thereof may form prodrugs. The present invention also encompasses such various prodrugs. Prodrugs are derivatives of the compounds of the present invention that have chemically or metabolically degradable groups, and compounds that are converted to the pharmaceutically active compounds of the present invention through solvolysis or under physiological conditions in vivo. Prodrugs include compounds that are converted to the compounds of Formula (I) through enzymatic oxidation, reduction, hydrolysis or the like under physiological conditions in vivo, compounds that are converted to the compounds of Formula (I) through hydrolysis by gastric acid etc., and the like. Methods for selecting and preparing suitable prodrug derivatives are described in, for example, "Design of Prodrugs, Elsevier, Amsterdam, 1985". Prodrugs themselves may have some activity.

When the compounds of Formula (I) or pharmaceutically acceptable salts thereof have hydroxyl group(s), prodrugs include acyloxy derivatives and sulfonyloxy derivatives that are prepared by, for example, reacting compounds having hydroxyl group(s) with suitable acyl halide, suitable acid anhydride, suitable sulfonyl chloride, suitable sulfonyl anhydride and mixed anhydride, or with a condensing agent. For example, they include CH₃COO-, C₂H₅COO-, tert-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO-, CH₃SO₃-, CH₃CH₂SO₃-, CF₃SO₃-, CH₂FSO₃-, CF₃CH₂SO₃-, p-CH₃O-PhSO₃-, PhSO₃- and p-CH₃PhSO₃-.

### (Production method for compound of the present invention)

The compounds of Formula (I) can be prepared, for example, by a general synthetic method shown below. Starting materials and reaction reagents used in such synthesis are commercially available or can be prepared according to methods well known in the art using compounds commercially available. Further, extraction, purification and the like may be performed in accordance with the methods carried out in a normal organic chemistry experiment.

The compound of the present invention can be synthesized with reference to a method known in the art.

In the following steps, when a substituent that impedes the reaction (e.g., hydroxy, mercapto, amino, formyl, carbonyl, or carboxyl) is possessed, the substituent may be protected by the method described in Protective Groups in Organic Synthesis, Theodora W Greene (John Wiley & Sons) or the like in advance, and the protecting group may be removed at a desirable stage.

In addition, in the following all steps, an order of steps to be implemented may be appropriately changed, and each intermediate may be isolated, and used in a next step. All of reaction time, reaction temperature, solvents, reagents, protecting groups, etc. are mere exemplification and not limited as long as they do not cause an adverse effect on a reaction.

0 0 0 3

The general method for synthesizing the compound of the present invention is shown below. Both the starting materials and reaction reagents used in these synthesis are either commercially available or can be prepared using commercially available compounds according to methods well known in the art.

For example, the compounds represented by Formula (I) of the present invention can be prepared by the general synthetic methods described below.

### [Method A]

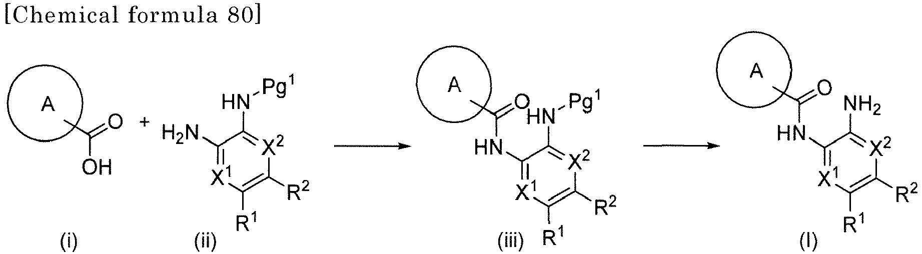

wherein Pg¹ is an appropriate protecting group for the amino group, and the other symbols have the same meaning as described above.

### (First step)

Compound (iii) can be prepared by reacting Compound (i) or a sodium salt or a potassium salt thereof and the like and Compound (ii) in the presence of a condensing agent such as 1-hydroxybenzotriazole, HOAt, 1-ethyl-3- (3-dimethylaminopropyl) carbodiimide hydrochloride, HATU, PyBOP, etc. and a base such as triethylamine, diisopropylethylamine, sodium carbonate etc. in a solvent such as THF, DMF, NMP, etc.

Further, Compound (iii) can be prepared by reacting the acid halide of the Compound (i) and Compound (ii) in the presence of a base such as pyridine, triethylamine, etc. in a solvent such as dichloromethane, THF, DMF, NMP, etc.

### (Second step)

Compound of Formula (I) is obtained by deprotecting compound (iii) in a solvent such as methanol, acetonitrile, acetone, dichloromethane, ethyl acetate, dioxane, etc. in the presence of an acid such as hydrochloric acid, trifluoroacetic acid, trimethylsilyl trifluoromethanesulfonate, etc.

### [Method B]

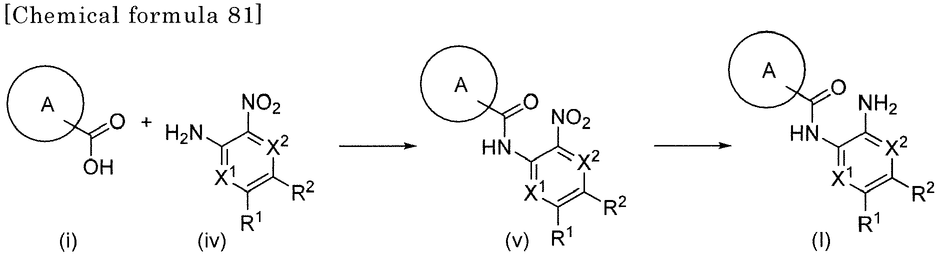

wherein the symbols have the same meaning as described above.

### (First step)

Compound (v) can be prepared by reacting Compound (i) or a sodium salt or a potassium salt thereof and the like and Compound (iv) in the presence of a condensing agent such as 1-hydroxybenzotriazole, HOAt, 1-ethyl-3- (3-dimethylaminopropyl) carbodiimide hydrochloride, HATU, PyBOP, etc. and a base such as triethylamine, diisopropylethylamine, sodium carbonate etc. in a solvent such as THF, DMF, NMP, etc.

Further, Compound (v) can be prepared by reacting the acid halide of the Compound (i) and Compound (iv) in the presence of a base such as pyridine, triethylamine, etc. in a solvent such as dichloromethane, THF, DMF, NMP, etc.

### (Second step)

Compound of Formula (I) is obtained by catalytically reducting Compound (v) or reducing under general reducing conditions such as iron ,tin, etc. in the presence of an acid.

The compound (i), compound (ii) and compound (iv) used as intermediates is commercially available products or can be prepared according to the methods described in the literature listed below, or the following examples.
International Publication WO2011/044181
International Publication WO2015/191630
International Publication WO2007/136605
Journal of Pesticide Science 25, 24-30 (2000)
HETEROCYCLES 57, 2299-2308 (2002)

The compound of the present invention has HDAC2 inhibitory activity and it can be available for therapeutic agent and/or prophylactic agent for a disease related to HDAC2.

In the present invention, the term "therapeutic agent and/or prophylactic agent" also includes a symptom improving agent.

Diseases related to HDAC2 include cancer and neurological disorders, preferably neurological disorders.

Examples of neurological diseases include central nervous system diseases (for example, neurodegenerative diseases). Central nervous system diseases include, for example, Alzheimer's disease, Alzheimer's dementia, Alzheimer's senile dementia, mild cognitive impairment (MCI), memory loss, attention deficit symptoms related to Alzheimer's disease, neurodegeneration related to Alzheimer's disease, and dementia of mixed vascular origin, dementia of degenerative origin, presenile dementia, senile dementia, dementia related to Parkinson's disease, vascular dementia, frontotemporal dementia, stroke, progressive supranuclear palsy, corticobasal degeneration, schizophrenia, delirium, attention defect disorder (ADD), Schizoaffective disorder, Rubinstein-Tevi syndrome, depression, manic disease, attention defect disorder, drug indulgence, dementia, autism, agitation, emotional dullness, anxiety, post-traumatic stress disorder (PTSD), mental illness, personality disorder, bipolar disorder, unipolar emotional disorder, obsessive compulsive disorder, eating disorder, post-traumatic stress disorder, hypersensitivity, adolescent behavioral disorder, disinhibition and the like, and Alzheimer's disease is particularly preferable.

The compound of the present invention has not only HDAC2 inhibitory activity but also are useful as a medicine and has any or all of the following excellent characteristics:
a) The compound is highly selective for HDAC2.
b) The compound is highly selective for HDAC2 with respect to HDAC1.
c) The compound is a weak inhibitor of CYP enzymes (e.g., CYP1A2, CYP2C9, CYP2C19, CYP2D6, and CYP3A4).
d) The compound demonstrates good pharmacokinetics, such as a high bioavailability, and moderate clearance.
e) The compound has a high metabolic stability.
f) The compound has no irreversible inhibitory action against CYP enzymes (e.g., CYP3A4) when the concentration is within the range described in the present description as the measurement conditions.
g) The compound has no mutagenicity.
h) The compound is associated with a low cardiovascular risk.
i) The compound is associated with a low hematological toxicity risk.
j) The compound has a high solubility.
k) The compound has high brain distribution ability.

A pharmaceutical composition of the present invention can be administered orally or parenterally. Methods for parenteral administration include dermal, subcutaneous, intravenous, intraarterial, intramuscular, intraperitoneal, transmucosal, inhalation, transnasal, ophthalmic, and inner ear or vaginal administration.

In the case of oral administration of pharmaceutical composition of the present invention, any forms, which are usually used, such as oral solid formulations (e.g., tablets, powders, granules, capsules, pills, or films), and oral liquid formulations (e.g., suspension, emulsion, elixir, syrup, lemonade, spirit, aromatic water, extract, decoction, or tincture) may be prepared according to the usual method and administered. The tablets can be sugar-coated tablets, film-coated tablets, enteric-coating tablets, sustained-release tablets, troche tablets, sublingual tablets, buccal tablets, chewable tablets or orally disintegrating tablets. Powders and granules can be dry syrups. Capsules can be soft capsules, micro capsules or sustained-release capsules.

In the case of parenteral administration of pharmaceutical composition of the present invention, any forms, which are usually used, such as injections, drips, and external preparations (e.g., ophthalmic drops, nasal drops, ear drops, aerosols, inhalations, lotion, infusion, liniment, mouthwash, enema, ointment, plaster, jelly, cream, patch, cataplasm, external powder, or suppository) can be preferably administered. Injections can be emulsions whose type is O/W, W/O, O/W/O, W/O/W or the like.

The pharmaceutical composition may be manufactured by mixing an effective amount of the compound of the present invention with various pharmaceutical additives suitable for the formulation, such as excipients, binders, disintegrants, and lubricants. Furthermore, the pharmaceutical composition can be for pediatric patients, geriatric patients, serious cases or operations by appropriately changing the effective amount of the compound of the present invention, formulation and/or various pharmaceutical additives. The pediatric pharmaceutical compositions are preferably administered to patients under 12 or 15 years old. In addition, the pediatric pharmaceutical compositions can be administered to patients who are under 27 days old after the birth, 28 days to 23 months old after the birth, 2 to 11 years old, 12 to 17 years old, or 18 years old. The geriatric pharmaceutical compositions are preferably administered to patients who are 65 years old or over.

Although the dosage of a pharmaceutical composition of the present invention should be determined in consideration of the patient's age and body weight, the type and degree of diseases, the administration route and the like, a usual oral dosage is 0.05 to 100 and preferably 0.1 to 10 mg/kg/day. For parenteral administration, although the dosage highly varies with administration routes, a usual dosage is 0.005 to 10 and preferably 0.01 to 1 mg/kg/day. The dosage may be administered in one to several divisions per day.

The compound of the present invention can be used in combination of therapeutic agents to increase the activity of the compound or reduce the dose of the compound, or the like. In this case, the timing of administration for a compound of the present invention and the co-administered drug is not limited. They can be administered to the subjects to be treated, at a time or at different times.

### EXAMPLES

The present invention will be described in more detail with reference to, but not limited to, the following Examples, Reference Examples and Test Examples.

In this description, meaning of each abbreviation is as follows:
TMS: trimethylsilane
DMSO: dimethylsulfoxide
DMA: dimethylacetamide
DMF: dimethylformamide
THF: tetrahydrofuran
HOAt: 1-hydroxy-7-azabenzotriazole
HATU: 2-(7-azabenzotriazol-1-yl)-1,1,3,3-tetramethyluronium hexafluorophosphate

0 0 0 4

Proton nuclear magnetic resonance spectra (¹H NMR) and carbon nuclear magnetic resonance spectra (¹³C NMR) were recorded using a BURKER AVANCE 300 spectrometer in the described solvent. Chemical shifts (δ) are described in parts per million compared to the internal standard tetramethylsilane. Electrospray ionization (ESI) mass spectra were recorded on a Bruker HCTplus mass spectrometer. Reagents and solvents were purchased from Aldrich, Merck, Nacalai Tesque, Tokyo Chemical Industry, Wako Pure Chemical Industries, Kishida Chemical, Kanto Chemical and used without purification. Flash column chromatography was performed using silica gel purchased from Merck silica gel. Further, when showing NMR data, there are cases where all the measured peaks are not described.

"MS" represents a measured value by LC/MS (liquid chromatography/mass spectrometry), and was measured under the following conditions.

0 0 0 5

### Measurement condition

Column: Sim-pack XR-ODS (2.2 *µ* m, i.d. 3.0×50mm) (Shimadzu)
Flow rate: 1.6 mL/min; UV detection wavelength: 254nm;
Mobile phase: [A] is a 0.1% formic acid-containing aqueous solution, and [B] is a 0.1% formic acid-containing acetonitrile solution.
Gradient: Linear gradient of 10% to 100% solvent [B] was performed for 3 minutes and the 100% solvent [B] was maintained for 0.5 minutes.

### [Example 1]

### Example 1 Synthesis of Compound 1-006

### Step 1 Synthesis of Compound 3

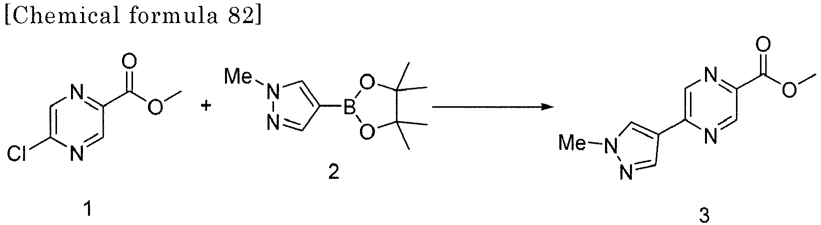

A suspension of Compound 1 (1.726 g, 10 mmol), Compound 2 (2.497 g, 12 mmol), Tripotassium Phosphate (6.37 g, 30 mmol), [1,1'-bis (diphenylphosphino) ferrocene] palladium (II) dichloride dichloromethane adduct (0.408 g, 0.5 mmol), 1,2-dimethoxyethane (30 mL) and water (6 mL) was heated under reflux for 3 hours under nitrogen atmosphere. Water (20 mL) and ethyl acetate (20 mL) were added to the reaction mixture. The precipitated solid was collected by filtration to give Compound 3 (1.71 g, yield: 78.4%) as a gray powder.

1H-NMR (δ ppm TMS / d6-DMSO): 3.91 (3H, s), 3.93 (3H, s), 8.22 (1H, s), 8.57 (1H, s), 9.09 (2H, s).

### Step 2 Synthesis of Compound 4

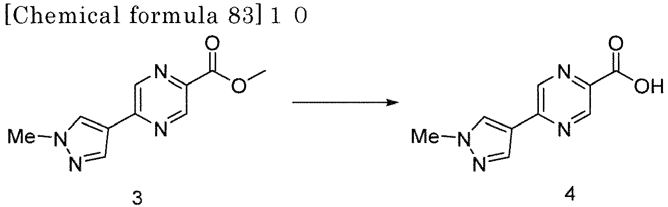

To a suspension of Compound 3 (1.637 g, 7.5 mmol) and DMSO (15 mL) was added 2 mol/L sodium hydroxide aqueous solution (7.5 mL, 15 mmol) under ice-cooling, and the mixture was stirred at room temperature for 1 hour. The reaction was neutralized with 2 mol/L hydrochloric acid. The precipitated solid was collected by filtration to give Compound 4 (1.50 g, yield: 97.9%) as a gray powder.

1H-NMR (δ ppm TMS / DMSO-d6): 3.93 (3H, s), 8.21 (1H, s), 8.56 (1H, s), 9.08 (2H, s), 13.50 (1H, s).

### Step 3 Synthesis of Compound 6

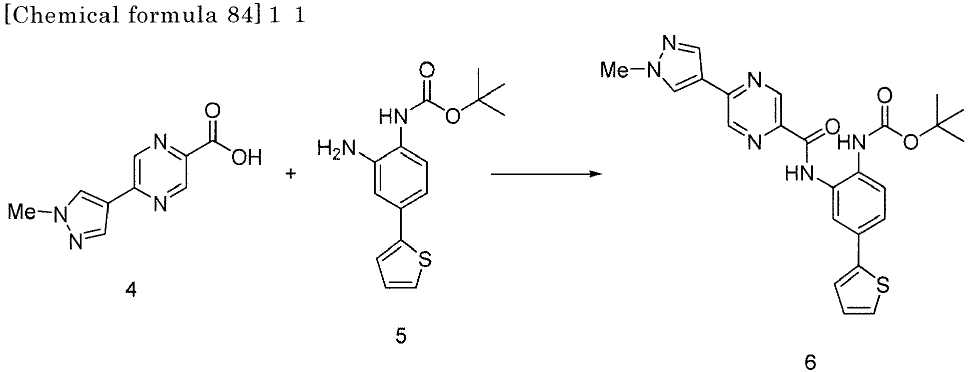

To a suspension of Compound 4 (1.067 g, 3.68 mmol) and DMF (14 mL) were added HATU (1.73 g, 4.55 mmol) and DIPEA (0.795 mL, 4.55 mmol), and the mixture was stirred at room temperature for 2 hours. The precipitated solid was collected by filtration to give Compound 6 (1.46 g, yield: 87.5%) as a gray powder.

1H-NMR (δ ppm TMS / d6-DMSO): 1.51 (9H, s), 3.95 (3H, s), 7.16 (1H, t, J = 4.3 Hz), 7.38 (1H, d, J = 8.4 Hz) , 7.48 + 7.52 (2H, m), 7.57 (1H, d, J = 4.9 Hz), 8.24 (1H, s), 8.26 (1H, s), 8.62 (1H, s), 9.02 (1H, s), 9.19 (1H, s), 9.22 (1H, s), 10.35 (1H, s).

### Step 4 Synthesis of Compound 1-006

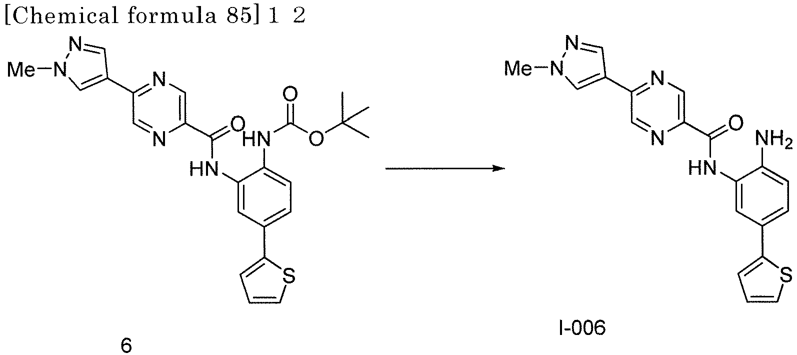

To a suspension of Compound 6 (1.324 g, 2.5 mmol) and methanol (5 mL) was added 4 mol/L hydrochloric acid dioxane solution (10 mL, 40 mmol) under ice-cooling, and the mixture was stirred at room temperature for 6 hours. Then, the reaction solution was left at room temperature overnight. 2 mol/L sodium hydroxide aqueous solution (22 mL) was added dropwise to the reaction solution. The precipitated solid was collected by filtration to give Compound 1-006 (0.55 g, yield: 58.4%) as a yellow powder.

1H-NMR (δ ppm TMS / DMSO-d6): 3.95 (3H, s), 5.20 (2H, s), 6.85 (1H, d, J = 8.4 Hz), 7.06 (1H, dd, J = 5.0, 3.6) Hz), 7.26 (1H, dd, J = 3.5, 0.9 Hz), 7.31 (1H, dd, J = 8.3, 2.1 Hz), 7.37 (1H, dd, J = 5.1, 0.9 Hz), 7.71 (1H, d) , J = 2.0 Hz), 8.25 (1H, s), 8.59 (1H, s), 9.09 (1H, d, J = 1.4 Hz), 9.17 (1H, d, J = 1.4 Hz), 10.03 (1H, s).

0 0 0 6

### Example 2 Synthesis of compound I-176

### Step 1 Synthesis of Compound 9

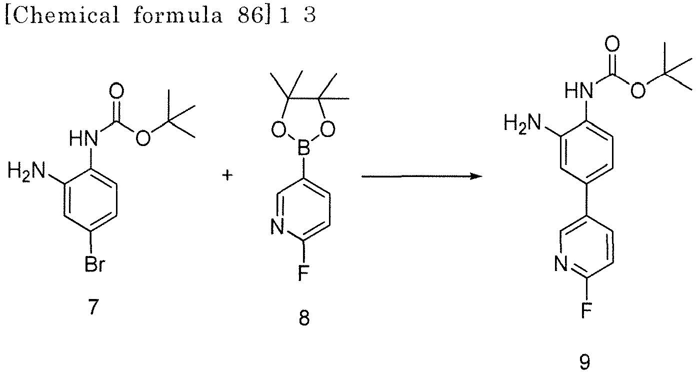

A suspension of Compound 7 (8.8 g, 30.6 mmol), Compound 8 (13.67 g, 61. 3 mmol), 2 mol/L potassium carbonate aqueous solution (16.94 g, 123 mmol), 1,1' -bis (di-t-butylphosphino) ferrocene palladium dichloride (3.99 g, 6.13 mmol) and dioxane (53 mL) was stirred under nitrogen atmosphere under reflux for 2 hours The reaction mixture was filtered through Celite (registered trademark), water was added to the mother liquid, and the mixture was extracted with ethyl acet ate. The organic layer was washed with brine, dried over anhydrous sodium sul fate, and concentrated under reduced pressure. Ethyl acetate and hexane were added to the residue, and the resulting powder was collected by filtration to giv e Compound 9 (6.81 g, yield: 73.3%) as a light brown powder.

1H-NMR (δ ppm TMS / CDCl₃): 1.53 (9H, s), 3.86 (2H, br s), 6.25 (1H, br s), 6.9 2-6.99 (3H, m), 7.40 (1H, d, J) = 8.0 Hz), 7.91 (1H, td, J = 8.0, 2.5 Hz), 8.36 (1 H, br s).

### Step 2 Synthesis of Compound 11

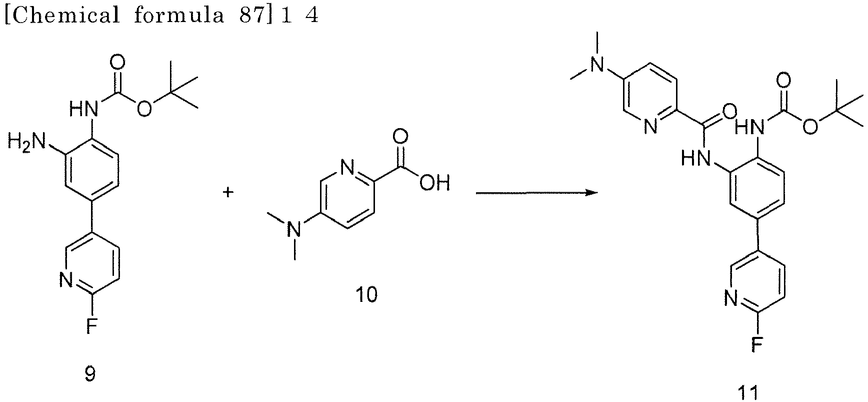

To a suspension of Compound 9 (0.182 g, 0.6 mmol), Compound 10 (0.110 g, 0.66 mmol) and DMF (2 mL) were added HATU (0.297 g, 0.78 mmol) and trie thylamine (0.125 mL, 0.9 mmol). The mixture was stirred at room temperature for 4 hours. Water (100 mL) was added to the reaction mixture, and the mixtu re was extracted with ethyl acetate (100 mL). The organic layer was washed tw ice with brine (100 mL), dried over anhydrous sodium sulfate, and concentrated under reduced pressure. Ethyl acetate and hexane were added to the residue, a nd the resulting powder was collected by filtration to give Compound 11 (0.25 g, yield: 92.3%) as a light brown powder.

1H-NMR (δ ppm TMS / CDCl₃): 1.51 (9H, s), 3.06 (6H, s), 7.22 (1H, dd, J = 8.9, 3.0 Hz), 7.30 (1H, dd, J = 8.5, 2.8) Hz), 7.37 (1H, d, J = 8.4 Hz), 7.47 (1H, dd, J = 8.3, 2.1 Hz), 7.95 (1H, t, J = 4.4 Hz), 8.06 (1H, d, J = 2.9) Hz), 8.26 (1H, td, J = 8.2, 2.6 Hz), 8.33 (1H, d, J = 1.3 Hz), 8.53 (1H, d, J = 2.4 Hz), 9.15 (1 H, s), 10.30 ( 1H, s).

### Step 3 Synthesis of Compound I-176

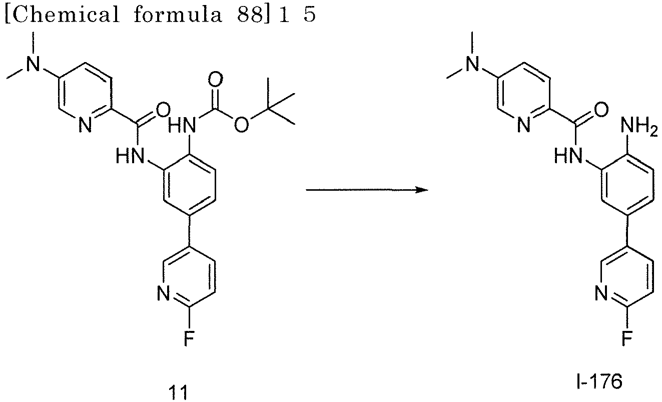

To a suspension of Compound 11 (0.226 g, 0.5 mmol) and dichloromethane (2 mL) were added 2,6-lutidine (0.349 mL, 3 mmol) and trimethylsilyl trifluorom ethanesulfonate (0.452 mL, 2.5 mmol) at room temperature. The mixture was st irred for 1 hour. Water (100 mL) was added to the reaction mixture, and the mixture was extracted with ethyl acetate (100 mL). The organic layer was wash ed twice with brine (100 mL), dried over anhydrous sodium sulfate, and concentr ated under reduced pressure. Ethyl acetate and hexane were added to the resid ue, and the resulting powder was collected by filtration to give Compound I-176 (0.15 g, yield: 85.4%) as a light brown powder.

1H-NMR (δ ppm TMS / d6-DMSO): 3.05 (6H, s), 5.12 (2H, s), 6.91 (1H, d, J = 8. 3 Hz), 7.18-7.22 (2H, m), 7.30 ( 1H, dd, J = 8.2, 2.0 Hz), 7.88 (1H, d, J = 1.9 H z), 7.93 (1H, d, J = 8.8 Hz), 8.11-8.16 (2H, m), 8.41 (1H, d) , J = 1.8 Hz), 9.84 (1H, s).

0 0 0 7

### Example 3 Synthesis of Compound I-018

### Step 1 Synthesis of Compound 14

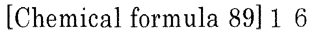

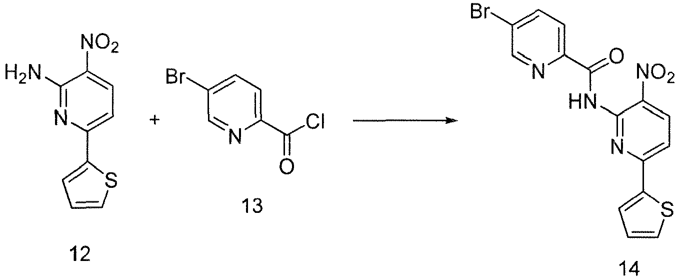

To a solution of Compound 12 (300 mg, 1.356 mmol) and Compound 13 (598 mg, 2.71 mmol) in THF (3 mL) was added pyridine (274 *µ* L, 3.39 mmol), and the mixture was stirred at room temperature for 3 days. Saturated ammonium chloride solution (3 mL) and water (100 mL) were added to the reaction mixture, and the mixture was extracted with ethyl acetate (100 mL). The organic layer was washed once with brine (100 mL), dried over anhydrous sodium sulfate, and concentrated under reduced pressure. Ethyl acetate and hexane were added to the residue, and the resulting powder was collected by filtration to give Compound 14 (262 mg, yield: 47.7%) as a brown powder.

1H-NMR (δ ppm TMS / d6-DMSO): 7.27 (1H, t, J = 4.4 Hz), 7.89 (1H, d, J = 4.8 Hz), 7.97 (1H, d, J = 8.8 Hz), 8.09 (1H, d, J = 3.5 Hz), 8.12 (1H, d, J = 8.3 Hz), 8.39 (1H, dd, J = 8.3, 2.3 Hz), 8.59 (1H, d, J = 8.5 Hz), 8.96 (1H, d, J = 2.0 Hz), 11.89 (1H, s).

### Step 2 Synthesis of Compound 16

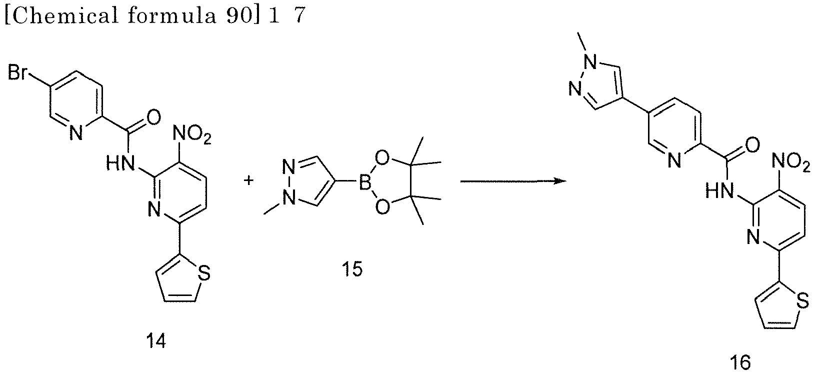

A suspension of Compound 14 (233 mg, 0.575 mmol), Compound 15 (359 mg, 1.725 mmol), 2 mol/L potassium carbonate aqueous solution (6.37 g, 30 mmol), 1,1'-bis (di-t-butylphosphino) ferrocene palladium dichloride (112 mg, 0.172 mmol) and DMF (5 mL) was stirred at 100°C. for 6 hours under nitrogen atmosphere. Water was added to the reaction mixture, and the mixture was extracted with ethyl acetate. The organic layer was washed with brine, dried over anhydrous sodium sulfate, and concentrated under reduced pressure. The residue was purified by silica gel chromatography (ethyl acetate/hexane) to give Compound 16 (20 mg, yield: 8.6%) as a yellow powder.

1H-NMR (δ ppm TMS / d6-DMSO): 3.92 (3H, s), 7.27 (1H, t, J = 4.4 Hz), 7.89 (1H, d, J = 5.3 Hz), 7.97 (1H, d, J = 8.8 Hz), 8.09 (1H, d, J = 2.5 Hz), 8.14-8.20 (2H, m), 8.25-8.29 (1H, m), 8.47 (1H, s), 8.60 (1H, d, J) = 8.8 Hz), 9.07 (1H, s), 11.95 (1H, s).

### Step 3 Synthesis of Compound 1-018

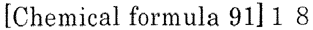

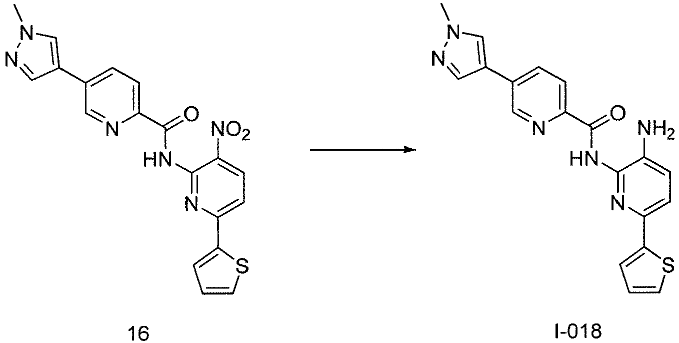

A suspension of Compound 16 (19 mg, 0.047 mmol), 10% Pd-C (38 mg, 0.357 mmol) and methanol (1 mL) was hydrogenated at room temperature for 5 hours. The reaction mixture was filtered through Celite (registered trademark) and concentrated under reduced pressure. The residue was purified by silica gel chromatography (methanol / chloroform) to give Compound I-018 (9.1 mg, yield: 51.7%) as a yellow powder.

1H-NMR (δ ppm TMS / d6-DMSO): 3.92 (3H, s), 5.36 (2H, s), 7.08 (1H, dd, J = 4.9, 3.6 Hz), 7.24 (1H, d, J = 8.3) Hz), 7.45 (1H, d, J = 4.5 Hz), 7.53 (1H, d, J = 2.5 Hz), 7.62 (1H, d, J = 8.3 Hz), 8.11-8.15 (2H, m), 8.23 ( 1H, dd, J = 8.2, 2.1 Hz), 8.44 (1H, s), 9.01 (1H, d, J = 1.5 Hz), 10.27 (1H, s).

0 0 0 8

### Example 4 Synthesis of Compound I-053

### Step 1 Synthesis of Compound 19

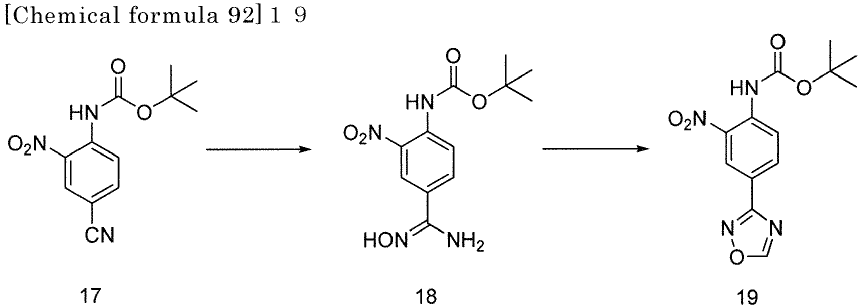

To a suspension of Compound 17 (1.0 g, 3.8 mmol) and methanol (10 mL) was added 50% hydroxylamine aqueous solution (0.447 mL, 7.6 mmol), and the mixture was stirred at 60°C for 2 hours. Water was added to the reaction mixture, and the mixture was extracted with ethyl acetate. The organic layer was washed with brine, dried over anhydrous sodium sulfate, and concentrated under reduced pressure to give crude compound 18 (1.20 g) as an orange solid.

A mixture of compound 18 (300 mg, 1.0 mmol) and ethyl orthoformate (3 mL) was stirred at 150°C for 18 hours. The reaction mixture was purified by silica gel chromatography (ethyl acetate/hexane) to give Compound 19 (71.2 mg, yield: 23%) as a yellow powder.

1H-NMR (δ ppm TMS / CDCl₃): 1.57 (9H, s), 8.33 (1H, dd, J = 8.9, 1.9 Hz), 8.77 (1H, d, J = 11.5 Hz), 8.79 (1H, s) , 8.98 (1H, d, J = 1.8 Hz)

### Step 2 Synthesis of Compound 22

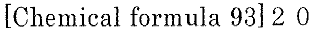

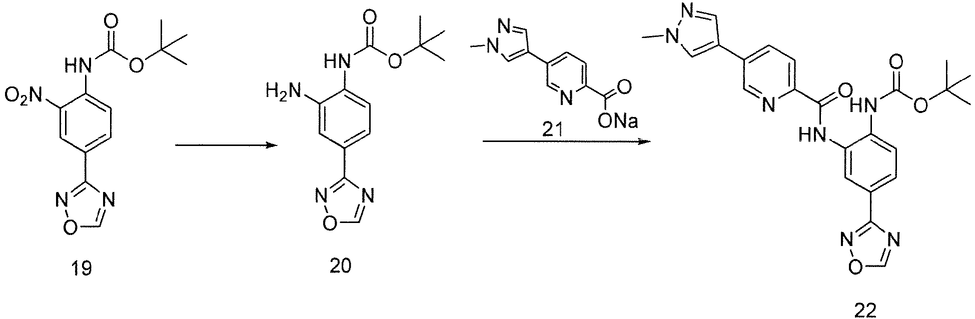

To a solution of Compound 19 (70 mg, 0.23 mmol) and DMF (0.7 mL) was added Tin (II) chloride dihydrate (155 mg, 0.69 mmol), and the mixture was stirred at room temperature for 7 hours. Potassium carbonate aqueous solution was added to the reaction mixture, and the mixture was extracted with ethyl acetate. The organic layer was washed with brine and dried over anhydrous sodium sulfate. The organic layer was concentrated under reduced pressure to give Compound 20 (70 mg) as a yellow amorphous substance.

To a suspension of Compound 20 (70 mg), Compound 21 (62.8 mg, 0.28 mmol) and DMF (1.4 mL) was added HATU (125 mg, 0.329 mmol), and the mixture was stirred overnight at room temperature. Water was added to the reaction mixture, and the mixture was extracted with ethyl acetate. The organic layer was washed with saturated brine and dried over anhydrous sodium sulfate. The organic layer was concentrated under reduced pressure. The residue was purified by silica gel chromatography (ethyl acetate/hexane) to give Compound 22 (67.4 mg, yield: 57.6%) as a white powder.

1H-NMR (δ ppm TMS / d6-DMSO): 1.52 (9H, s), 3.92 (3H, s), 7.53 (1H, d, J = 8.5 Hz), 7.84 (1H, d, J = 8.3 Hz) , 8.12 (1H, s), 8.15 (1H, d, J = 10.0 Hz), 8.24 (1H, d, J = 8.3 Hz), 8.44 (1H, s), 8.72 (1H, s), 8.90 (1H, 1H, s), 9.37 (1H, s), 9.72 (1H, s), 10.53 (1H, s).

### Step 3 Synthesis of Compound 1-053

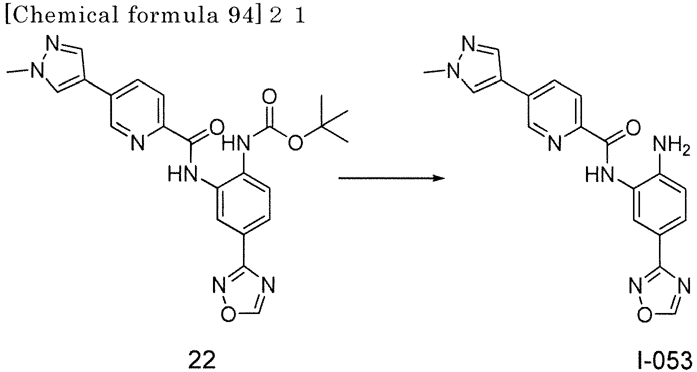

To a suspension of Compound 22 (60 mg, 0.13 mmol) and dichloromethane (0.6 mL) were added 2,6-lutidine (91 *µ* L, 0.78 mmol) and trimethylsilyl trifluoromethanesulfonate (117 *µ* L, 0.65 mmol) under ice-cooling, the mixture was stirred at room temperature for 1 hour. Water (10 mL) was added to the reaction mixture, and the mixture was extracted with chloroform (10 mL). The organic layer was washed twice with brine (10 mL) and dried over anhydrous sodium sulfate. The organic layer was concentrated under reduced pressure. The residue was purified by silica gel chromatography (methanol/chloroform) to give Compound I-053 (36.1 mg, yield: 76.8%) as a yellow powder.

1H-NMR (δ ppm TMS / d6-DMSO): 3.77 (3H, s), 5.47 (2H, s), 6.78 (1H, d, J = 8.3 Hz), 7.50 (1H, d, J = 6.8 Hz) , 7.96 (1H, d, J = 7.0 Hz), 7.97 (1H, s), 8.02 (1H, s), 8.07 (1H, d, J = 8.3 Hz), 8.28 (1H, s), 8.83 (1H, 1H, s), 9.40 (1H, s), 9.90 (1H, s)

0 0 0 9

### Example 5 Synthesis of Compound I-182

### Step 1 Synthesis of Compound 27

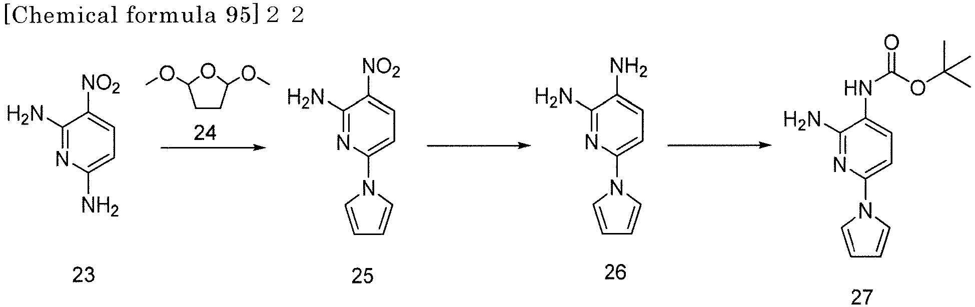

A mixture of Compound 23 (3.11 g, 20.18 mmol), Compound 24 (2.65 mL, 20.58 mmol) and acetic acid (31.1 mL) was stirred at 100°C. for 4 hours. Under ice cooling, the reaction solution was neutralized with 10 mol/L sodium hydroxide aqueous solution. The precipitated solid was collected by filtration to give a crude Compound 25.

To the obtained Compound 25 were added 10% Pd-C (2.27 g, 21.3 mmol) and methanol (49.4 mL), and hydrogenation was performed at room temperature for 5 hours. The reaction mixture was filtered through Celite (registered trademark) and concentrated under reduced pressure. Ethyl acetate was added to the residue, and the resulting powder was collected by filtration to give Compound 26 (3.11 g, yield: 73.8%) as a light brown powder.

To a mixture of Compound 26 (2.5 g, 14.35 mmol), THF (12.5 mL) and water (12.5 mL) were added sodium hydrogen carbonate (3.62 g, 43.1 mmol) and di-t-butyl dicarbonate (4.33 ml, 18.66 mmol), and the mixture was stirred at room temperature for 3 hours. Water was added to the reaction mixture, and the mixture was extracted with ethyl acetate. The organic layer was washed with brine and dried over anhydrous sodium sulfate. The organic layer was concentrated under reduced pressure. Ethyl acetate was added to the residue, and the resulting powder was collected by filtration to give Compound 27 (2.22 g, yield: 56.4%) as a light brown powder.

1H-NMR (δ ppm TMS / d6-DMSO): 1.47 (9H, s), 5.95 (2H, br s), 6.20 (2H, t, J = 2.1 Hz), 6.77 (1H, d, J = 8.3 Hz), 7.49 (2H, t, J = 2.1 Hz), 7.63 (1H, br s), 8.40 (1H, br s).

### Step 2 Synthesis of Compound 29

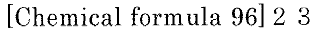

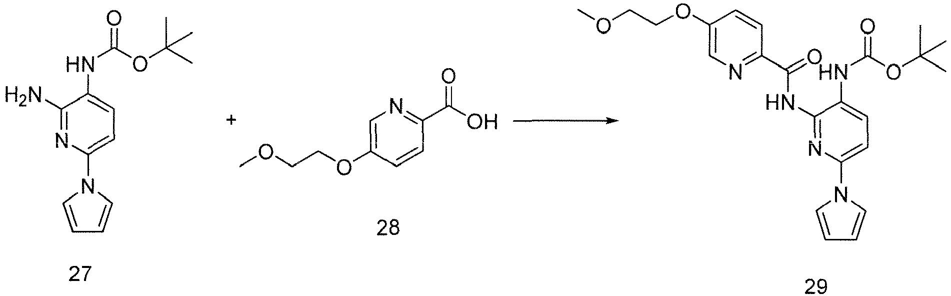

To a suspension of Compound 27 (0.108 g, 0.55 mmol), Compound 28 (0.108 g, 0.55 mmol) and DMF (2 mL) were added HATU (0.247 g, 0.65 mmol) and triethylamine (0.104 mL, 0.75 mmol), the mixture was stirred at room temperature for 4 hours. Water (100 mL) was added to the reaction mixture, and the mixture was extracted with ethyl acetate (100 mL). The organic layer was washed twice with brine (100 mL) and dried over anhydrous sodium sulfate. The organic layer was concentrated under reduced pressure, the residue was purified by silica gel chromatography (ethyl acetate/hexane), ethyl acetate and hexane were added to the concentrate, and the resulting powder was collected by filtration to give Compound 29 (0.08 g, yield: 35.3). %) as a light brown powder.

1H-NMR (δ ppm TMS / d6-DMSO): 1.51 (9H, s), 3.48 (3H, s), 3.81-3.84 (2H, m), 4.28 (2H, dd, J = 5.3, 3.8 Hz), 6.33 (2H, t, J = 2.2 Hz), 7.23 (1H, d, J = 8.7 Hz), 7.39 (1H, dd, J = 8.7, 2.8 Hz), 7.46 (2H, t, J = 2.3 Hz), 8.28 (1H, d, J = 8.7 Hz), 8.33 (1H, s), 8.39 (1H, d, J = 2.6 Hz), 8.71 (1H, s), 10.26 (1H, s).

### Step 3 Synthesis of compound I-182

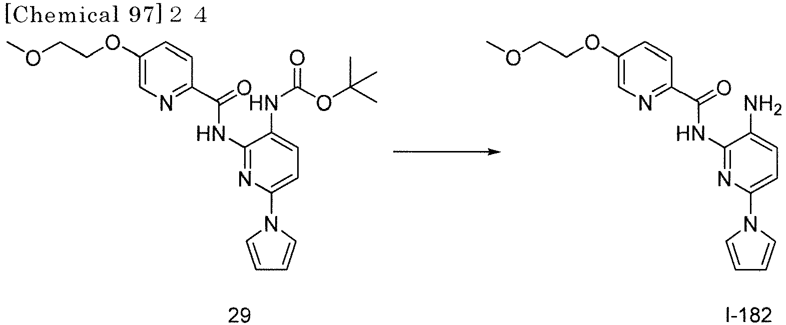

To a suspension of Compound 29 (0.073 g, 0.16 mmol) and dichloromethane (2 mL) were added 2,6-lutidine (0.112 mL, 0.96 mmol) and trimethylsilyl trifluoromethanesulfonate (0.145 mL, 0.800 mmol) at room temperature. The mixture was stirred for 1 hour. Water (100 mL) was added to the reaction mixture, and the mixture was extracted with ethyl acetate (100 mL). The organic layer was washed twice with brine (100 mL), dried over anhydrous sodium sulfate, and concentrated under reduced pressure. The residue was purified by silica gel chromatography (ethyl acetate/hexane), ethyl acetate and hexane were added to the concentrate, the resulting powder was collected by filtration to give Compound I-182 (0.04 g, yield: 70.7%) as a pale yellow powder.

1H-NMR (δ ppm TMS / d6-DMSO): 3.72 (2H, t, J = 4.3 Hz), 4.31 (2H, t, J = 4.4 Hz), 5.11 (2H, s), 6.21 (2H, t, J = 2.0 Hz), 7.35 (1H, d, J = 8.5 Hz), 7.40 (1H, d, J = 8.5 Hz), 7.47 (2H, t, J = 2.1 Hz), 7.64 (1H, dd, J = 8.7, 2.8 Hz), 8.12 (1H, d, J = 8.7 Hz), 8.43 (1H, d, J = 2.8 Hz), 10.24 (1H, s).

0 0 1 0

### Example 6 Synthesis of Compound I-043

### Step 1 Synthesis of Compound 32

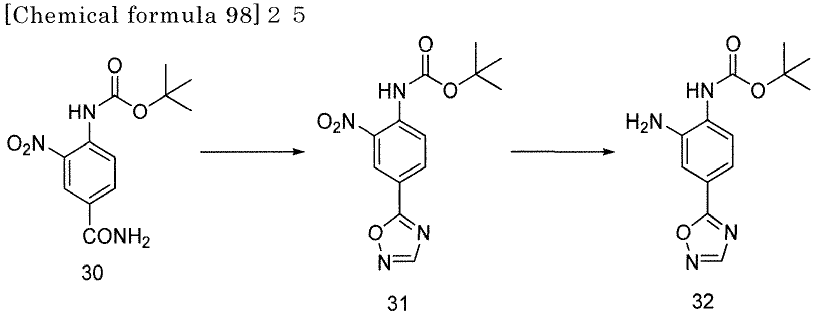

To Compound 30 (0.7 g, 2.489 mmol) was added N, N-dimethylformamide dimethylacetal (1.66 mL, 12.44 mmol), and the mixture was stirred at 60°C for 0.5 hours. The reaction mixture was concentrated under reduced pressure, dioxa ne (0.4 mL), acetic acid (0.4 mL) and 50% hydroxylamine aqueous solution (220 *µ* L, 3.73 mmol) were added to the residue, and the mixture was stirred at 90°C for 4 hours. Potassium carbonate aqueous solution was added to the reaction mixture, and the mixture was extracted with ethyl acetate. The organic layer w as washed with brine and dried over anhydrous sodium sulfate. The organic lay er was concentrated under reduced pressure, and the residue was purified by sili ca gel chromatography (ethyl acetate/hexane) to give Compound 31 (0.443 mg, yi eld: 58.2%) as a pale yellow powder.

To a solution of Compound 31 (440 mg, 1.437 mmol) and DMF (4.4 mL) was added Tin (II) chloride dihydrate (972 mg, 4.31 mmol), and the mixture was stirred at 40°C. for 3 hours. Potassium carbonate aqueous solution was added to the reaction mixture, the mixture was filtered through Celite (registered trademark), and extracted with ethyl acetate. The organic layer was washed with brine and dried over anhydrous sodium sulfate. The reaction mixture was concentrated under reduced pressure, and the residue was purified by silica gel chromatography (ethyl acetate/hexane) to give Compound 32 (0.34 g, yield: 84.9%) as a pale yellow powder. 1H-NMR (δ ppm TMS / CDCl₃): 1.54 (9H, s), 3.77 (2H, s), 6.50 (1H, s), 7.58 (1H, d, J = 1.5 Hz), 7.64 (1H, dd, J = 1.5 Hz, 6.0 Hz), 7.70 (1H, d, 6.0 Hz), 8.43 (1H, s)

### Step 2 Synthesis of Compound I-043

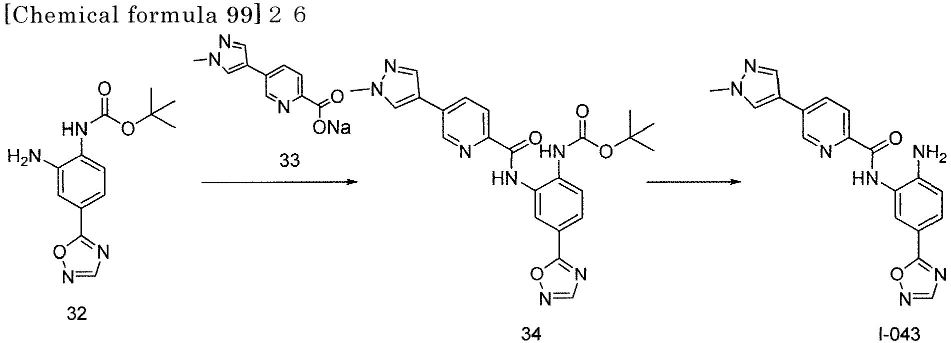

To a suspension of Compound 32 (100 mg, 0.362 mmol), Compound 33 (90 mg, 0.398 mmol) and DMF (2 mL) was added HATU (179 mg, 0.471 mmol), and the mixture was stirred at room temperature overnight. Water was added to the reaction solution, and the precipitated solid was collected by filtration to give Compound 34 (136.4 mg, yield: 81.7%) as a yellow powder.

To a suspension of Compound 34 (120 mg, 0.26 mmol) and dichloromethane (1.2 mL) were added 2,6-Lutidine (0.18 mL, 1.56 mmol) and trimethylsilyl trifluoromethanesulfonate (0.24 mL, 1.3 mmol) under ice-cooling. The mixture was stirred at room temperature for 1 hour. Water was added to the reaction mixture, and the mixture was extracted with chloroform. The organic layer was washed with brine and dried over anhydrous sodium sulfate. The organic layer was concentrated under reduced pressure. Acetone was added to the residue, and the resulting powder was collected by filtration to give Compound I-043 as an orange powder.

1H-NMR (δ ppm TMS / d6-DMSO): 3.92 (3H, s), 5.99 (2H, s), 6.94 (1H, d, J = 8.5 Hz), 7.73 (1H, d, J = 6.8 Hz), 8.11 (1H, d, J = 10.0 Hz), 8.12 (1H, s), 8.17 (1H, d, J = 2.5 Hz), 8.22 (1H, dd, J = 2.5 Hz, 10.5 Hz), 8.43 (1H, s), 8.91 (1H, s), 8.98 (1H, s), 10.1

### (1H, s)

0 0 1 1

### Example 7 Synthesis of Compound I-273

### Step 1 Synthesis of Compound 36

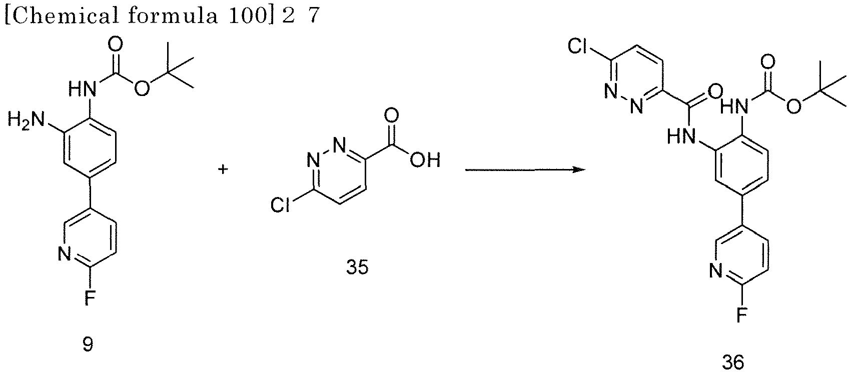

To a suspension of Compound 9 (0.607 g, 2 mmol), Compound 35 (0.349 g, 2.200 mmol) and DMF (6 mL) were added HATU (0.989 g, 2.60 mmol) and triethylamine (0.416 mL, 3.00 mmol), the mixture was stirred at room temperature for 2 hours. Water (100 mL) was added to the reaction mixture, and the mixture was extracted with ethyl acetate (100 mL). The organic layer was washed twice with brine (100 mL), dried over anhydrous sodium sulfate, and concentrated under reduced pressure. Ethyl acetate and hexane were added to the residue, and the resulting powder was collected by filtration to give Compound 36 (0.73 g, yield: 82.2%) as a light brown powder.

1H-NMR (δ ppm TMS / CDCl₃): 1.49 (9H, s), 7.30 (1H, dd, J = 8.3, 2.4 Hz), 7.59 (2H, s), 8.13 (1H, s), 8.20 (1H, d, J = 8.9 Hz), 8.27 (1H, tt, J = 7.7, 2.0 Hz), 8.37 (1H, d, J = 8.9 Hz), 8.54 (1H, d, J = 0.5 Hz), 9.22 (1H, d, J = 0.4 Hz), 10.76 (1H, s).

### Step 2 Synthesis of Compound 37

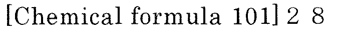

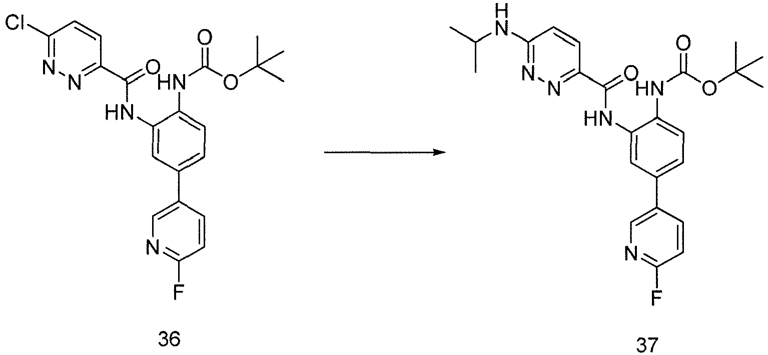

To a suspension of Compound 36 (0.133 g, 0.3 mmol), isopropylamine (0.077 mL, 0.900 mmol) and dioxane (2 mL) was added triethylamine (0.083 mL, 0.600 mmol), and the mixture was stirred at 110°C. for 8 hours. Water (100 mL) was added to the reaction mixture, and the mixture was extracted with ethyl acetate (100 mL). The organic layer was washed twice with brine (100 mL), dried over anhydrous sodium sulfate, and concentrated under reduced pressure. The residue was purified by silica gel chromatography (ethyl acetate/hexane/chloroform) to give Compound 37 (0.07 g, yield: 50%) as a white powder.

1H-NMR (δ ppm TMS / CDCl₃): 1.24 (6H, d, J = 6.4 Hz), 1.48 (9H, s), 4.22 (1H, s), 6.92 (1H, d, J = 9.4 Hz), 7.30 (1H, d, J = 8.5 Hz), 7.46 (1H, d, J = 7.4 Hz), 7.51 (2H, s), 7.84 (1H, d, J = 9.3 Hz), 8.22 (1H, s), 8.26 (1H, t, J = 8.2 Hz), 8.53 (1H, s), 9.13 (1H, s), 10.46 (1H, s).

### Step 3 Synthesis of Compound 1-273

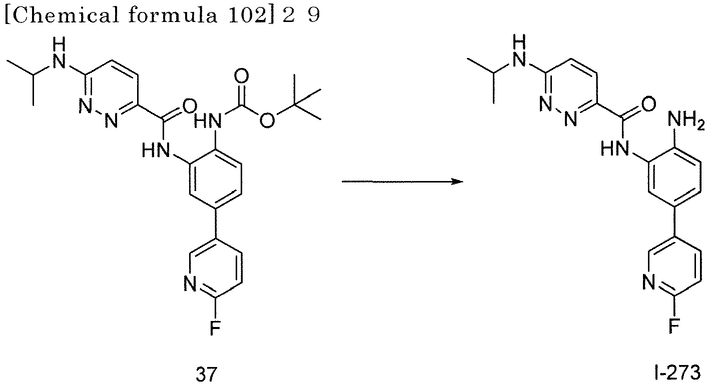

To a suspension of Compound 37 (0.065 g, 0.14 mmol) and dichloromethan e (2 mL) were added 2,6-Lutidine (0.065 mL, 0.560 mmol) and trimethylsilyl trifl uoromethanesulfonate (0.076 mL, 0.420 mmol) under ice-cooling., and the mixture was stirred at 0°C for 0.5 hours. Water (100 mL) was added to the reaction m ixture, and the mixture was extracted with ethyl acetate (100 mL). The organic layer was washed twice with brine (100 mL), dried over anhydrous sodium sulf ate, and concentrated under reduced pressure. Ethyl acetate was added to the r esidue, and the resulting powder was collected by filtration to give Compound I-273 (0.035 g, yield: 68.2%) as a light brown powder.

1H-NMR (δ ppm TMS / d6-DMSO): 1.24 (6H, d, J = 6.4 Hz), 4.24 (1H, sept, J = 6.3 Hz), 5.20 (2H, s), 6.90 (1H, s), 6.92 (1H, s), 7.21 (1H, dd, J = 8.5, 2.6 Hz), 7.34 (1H, d, J = 8.3 Hz), 7.39 (1H, d, J = 7.3 Hz), 7.79 (1H, s), 7.82 (1H, d, J = 9.2 Hz), 8.14 (1H, t, J = 7.0 Hz), 8.42 (1H, s), 10.08 (1H, s).

0 0 1 2

### Example 8 Synthesis of Compound 1-222

### Step 1 Synthesis of Compound 39

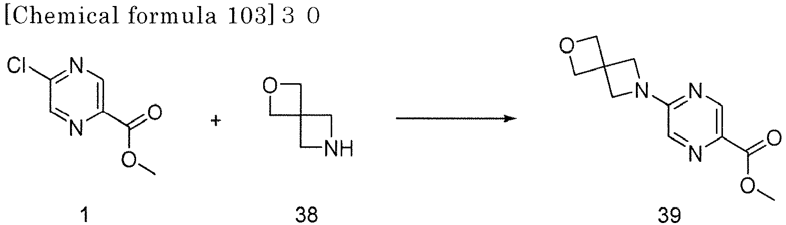

To a suspension of Compound 1 (0.500 g, 2.90 mmol), Compound 38 (0.316 g, 3.19 mmol) and dioxane (2.5 mL) was added triethylamine (0.884 mL, 6.37 mmol), and the mixture was stirred at room temperature for 16 hours. Water (10 mL) was added to the reaction mixture, and the mixture was extracted with ethyl acetate (50 mL). The organic layer was washed with water (10 mL) and brine (10 mL), dried over anhydrous sodium sulfate, and concentrated under reduced pressure to give Compound 39 (0.118 g, yield: 17%) as a brown powder.

1H-NMR (δ ppm TMS / d6-DMSO): 3.81 (3H, s), 4.35 (4H, s), 4.74 (4H, s), 7.89 (1H, s, J = 1.3 Hz), 8.63 (1H, s, J = 1.3 Hz)

### Step 2 Synthesis of Compound 41

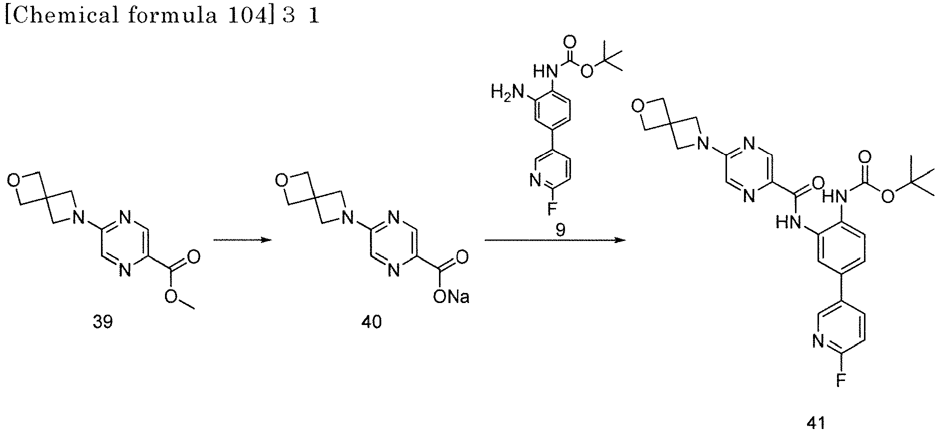

To a solution of Compound 39 (0.118 g, 0.502 mmol), THF (0.590 mL) and methanol (0.590 mL) was added 2 mol/L sodium hydroxide aqueous solution (0.263 mL, 0.527 mmol), and the mixture was stirred for 1 hour. The reaction mixture was concentrated under reduced pressure to give a crude Compound 40 (0.135 g) as a light brown powder.

To a suspension of Compound 40 (0.050 g, 0.206 mmol), Compound 9 (0.048 g, 0.158 mmol) and DMF (0.5 mL) was added HATU (0.078 g, 0.206 mmol), and the mixture was stirred at room temperature for 4 hours. Water (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound 41 (0.073 g, yield 91%) as a light brown powder. 1H-NMR (δ ppm TMS / d6-DMSO): 1.50. (9H, s), 4.36 (4H, s), 4.75 (4H, s), 7.30 (1H, dd, J = 2.7 Hz, 8.5 Hz), 7.39 (1H, d, J = 8.4 Hz), 7.50 (1H, dd, J = 1.9 Hz, 8.2 Hz), 7.80 (1H, s), 8.26 (2H, d, J = 13.1 Hz), 8.52 (1H, s), 8.72 (1H, s), 9.19 (1H, s), 10.10 (1H, s)

### Step 3 Synthesis of Compound I-222

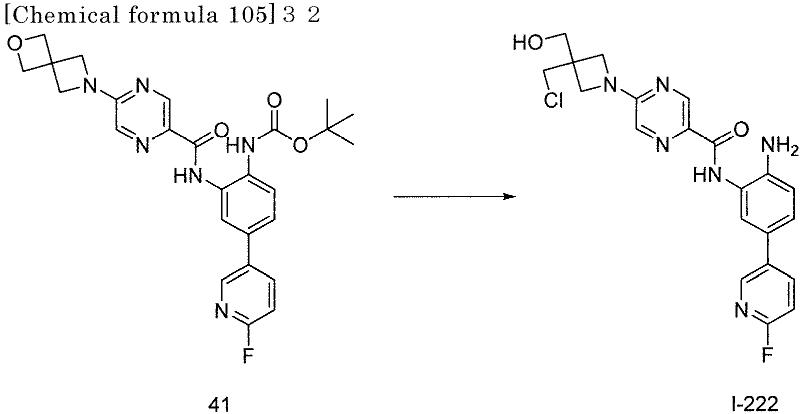

To a solution of Compound 41 (0.065 g, 0.14 mmol) and dioxane (0.7 mL) was added 4 mol/L hydrochloric acid dioxane solution (0.345 mL, 1.382 mmol), and the mixture was stirred at room temperature for 16 hours. Saturated sodium hydrogen carbonate aqueous solution (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound 1-222 (0.051 g, yield 84%) as a brown powder.

1H-NMR (δ ppm TMS / d6-DMSO): 3.57 (1H, s), 3.67 (1H, d, J = 5.1 Hz), 3.92-4.01 (5H, m), 5.16 (2H, s), 5.20 (1H, d, J = 5.3 Hz), 6.91 (1H, d, J = 8.4 Hz), 7.21 (1H, dd, J = 2.6 Hz, 8.5 Hz), 7.34 (1H, dd, J = 1.9 Hz, 8.3 Hz), 7.79 (1H, d, J = 1.6 Hz), 7.96 (1H, s), 8.14 (1H, td, J = 2.5 Hz, 8.2 Hz), 8.41 (1H, s), 8.71 (1H, s), 9.73 (1H, s)

0 0 1 3

### Example 9 Synthesis of Compound 1-224

### Step 1 Synthesis of Compound 43

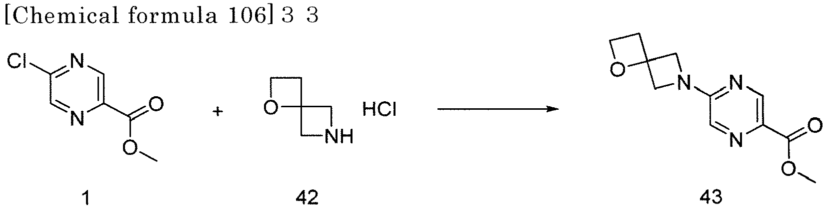

To a suspension of Compound 1 (0.500 g, 2.90 mmol), Compound 42 (0.316 g, 3.19 mmol) and dioxane (2.5 mL) was added triethylamine (1.325 mL, 9.56 mmol), and the mixture was stirred at room temperature for 16 hours. Water (10 mL) was added to the reaction mixture, and the mixture was extracted with ethyl acetate (50 mL). The organic layer was washed with water (10 mL) and brine (10 mL), dried over anhydrous sodium sulfate, and concentrated under reduced pressure to give Compound 43 (0.453 g, yield: 67%) as a light brown powder.

1H-NMR (δ ppm TMS / d6-DMSO): 2.89 (2H, t, J = 7.5 Hz), 3.32 (3H, s), 4.27 (2H, dd, J = 1.4 Hz, 10.7 Hz), 4.44 (4H, m), 7.90 (1H, d, J = 1.3 Hz), 8.63 (1H, d, J = 1.3 Hz)

### Step 2 Synthesis of Compound 45

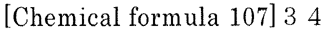

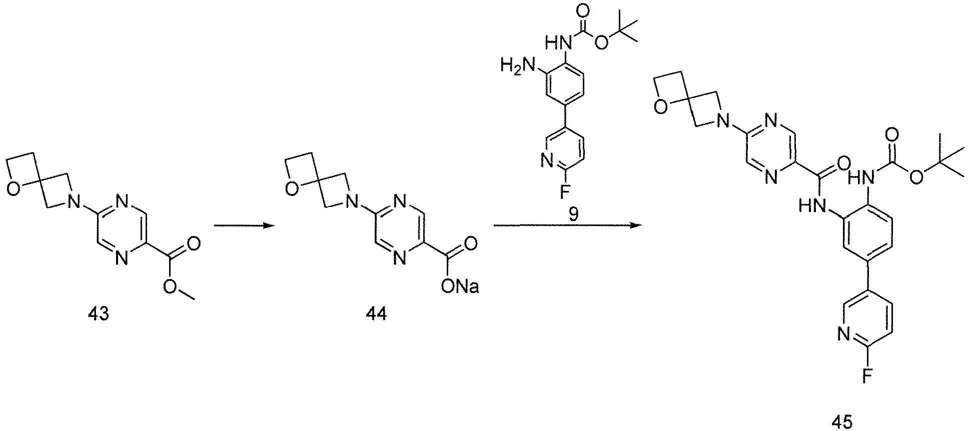

To a solution of Compound 43 (0.453 g, 1.926 mmol), THF (2.3 mL) and methanol (2.3 mL) was added 2 mol/L sodium hydroxide aqueous solution (1.01 mL, 2.022 mmol), and the mixture was stirred for 1 hour. The reaction mixture was concentrated under reduced pressure to give a crude Compound 44 (0.470 g) as a light brown powder.

To a suspension of Compound 44 (0.050 g, 0.429 mmol), Compound 9 (100 mg, 0.330 mmol) and DMF (1.0 mL) was added HATU (0.163 g, 0.429 mmol), and the mixture was stirred at room temperature for 4 hours. Water (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound 45 (0.165 g, yield 99%) as a white powder.

1H-NMR (δ ppm TMS / d6-DMSO): 1.50 (9H, s), 2.91 (2H, t, J = 7.5 Hz), 4.29 (2H, d, J = 10.5 Hz), 4.42-4.50 (4H, m), 7.30 (1H. dd, J = 2.6 Hz, 8.5 Hz), 7.40 (1H, d, J = 8.3 Hz), 7.51 (1H, dd, J = 1.9 Hz, 8.3 Hz), 7,82 (1H, s), 8.23-8.28 (2H, m), 8.52 (1H, s), 8.72 (1H, s), 9.18 (1H, s), 10.11 (1H, s)

### Step 3 Synthesis of Compound I-224

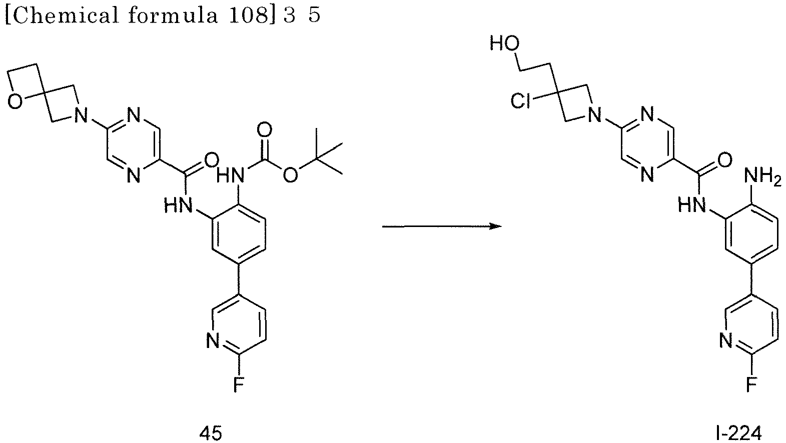

To a solution of Compound 45 (0.200 g, 0.395 mmol) and dioxane (2.0 mL) was added 4 mol/L hydrochloric acid dioxane solution (0.987 mL, 3.95 mmol), and the mixture was stirred at room temperature for 16 hours. Saturated sodium hydrogen carbonate aqueous solution (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound I-224 (0.130 g, 74% yield) as a brown powder.

1H-NMR (δ ppm TMS / d6-DMSO): 2.25 (2H, t, J = 7.3 Hz), 3.76 (2H, t, J = 7.3 Hz), 4.00 (2H, d, J = 9.4 Hz), 4.24 (2H, d, J = 9.4 Hz), 5.16 (2H, s), 6.07 (1H, s), 6.91 (1H, d, J = 8.3 Hz), 7.21 (1H, dd, J = 2.8 Hz, 8.5 Hz), 7.34 (1H, dd, J = 2.0 Hz, 8.3 Hz), 7.78 (1H, d, J = 2.0 Hz), 7.92 (1H, s, J = 1.1 Hz), 8.14 (1H, td, J = 2.6 Hz, 8.3 Hz), 8.41 (1H, d, J = 2.0 Hz), 8.71 (1H, d, J = 1.1 Hz), 9.73 (1H, s)

0 0 1 4

### Example 10 Synthesis of Compound 1-225

### Step 1 Synthesis of Compound 47

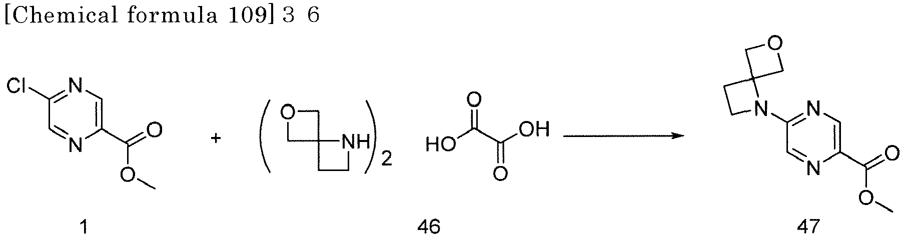

To a suspension of Compound 1 (0.500 g, 2.90 mmol), Compound 46 (0.459 g, 1.594 mmol) and dioxane (2.5 mL) was added triethylamine (1.325 mL, 9.56 mmol), and the mixture was stirred at room temperature for 16 hours. Water (10 mL) was added to the reaction mixture, and the mixture was extracted with ethyl acetate (50 mL). The organic layer was washed with water (10 mL) and brine (10 mL), dried over anhydrous sodium sulfate, and concentrated under reduced pressure to give Compound 47 (0.478 g, yield: 70%) as a brown powder.

1H-NMR (δ ppm TMS / d6-DMSO): 2.67 (2H, t, J = 7.2 Hz), 3.82 (3H, s), 3.98 (2H, t, J = 7.1 Hz), 4.65 (2H, d, J = 7.0 Hz), 5.25 (2H, d, J = 6.9 Hz), 8.01 (1H, s), 8.71 (1H, s)

### Step 2 Synthesis of Compound 49

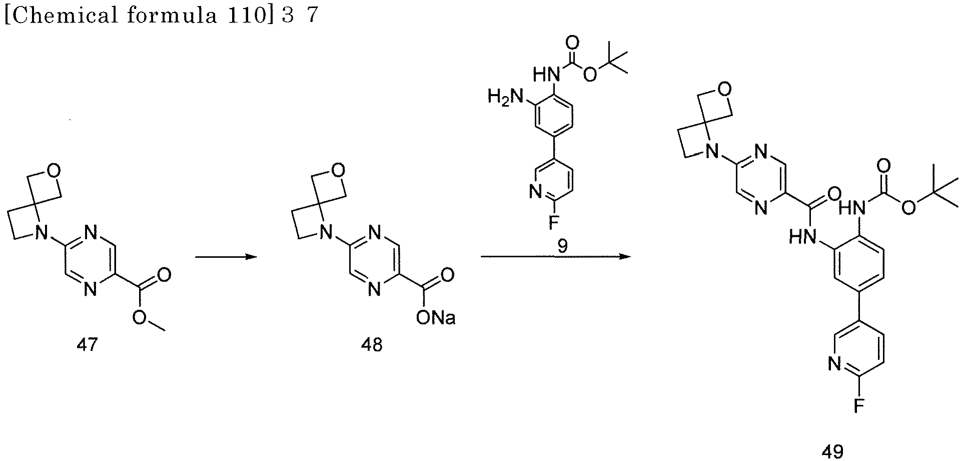

To a solution of Compound 47 (0.478 g, 2.032 mmol), THF (2.5 mL) and methanol (2.5 mL) was added 2 mol/L sodium hydroxide aqueous solution (1.07 mL, 2.134 mmol), and the mixture was stirred for 1 hour. The reaction mixture was concentrated under reduced pressure to give a crude Compound 48 (0.499 g) as a brown powder.

To a suspension of Compound 48 (0.104 g, 0.429 mmol), Compound 9 (100 mg, 0.330 mmol) and DMF (1.0 mL) was added HATU (0.163 g, 0.429 mmol), and the mixture was stirred at room temperature for 4 hours. Water (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound 49 (0.162 g, yield 98%) as a light brown powder. 1H-NMR (δ ppm TMS / d6-DMSO): 1.51 (9H, s), 2.68 (2H, t, 7.3 Hz), 3.98 (2H, t, J = 7.3 Hz), 4.70 (2H, d, J = 7.3 Hz), 5.25 (2H, d, J = 6.9 Hz), 7.30 (1H, dd, J = 2.5 Hz, 8.4 Hz), 7.39 (1H, d, J = 8.4 Hz), 7.51 (1H, d, J = 8.4 Hz), 7.97 (1H, s), 8.25-8.30 (2H, m), 8.53 (1H, s), 8.80 (1H, s), 9.22 (1H, s), 10.16 (1H, s)

### Step 3 Synthesis of compound 1-225

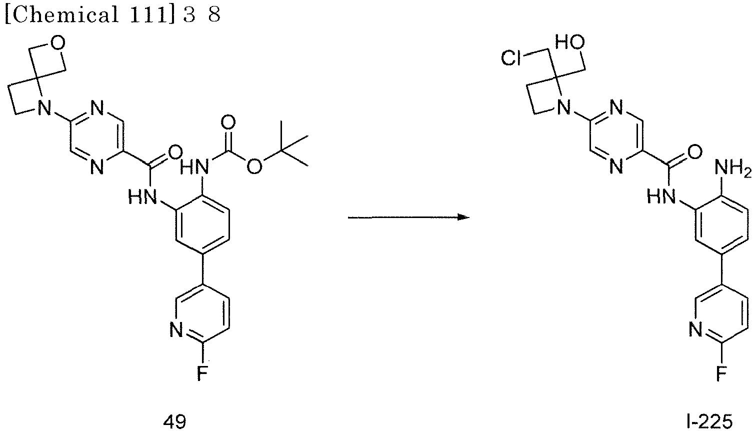

To a solution of Compound 49 (0.170 g, 0.336 mmol) and dioxane (2.0 mL) was added 4 mol/L hydrochloric acid dioxane solution (0.839 mL, 3.36 mmol), and the mixture was stirred at room temperature for 16 hours. Saturated sodium hydrogen carbonate aqueous solution (10 mL) was added to the reaction mixture, and the mixture was extracted with ethyl acetate (10 mL). The organic layer was washed with water (10 mL) and brine (10 mL), dried over anhydrous sodium sulfate, and concentrated under reduced pressure. Ethyl acetate and hexane were added to the residue, and the resulting powder was collected by filtration to give Compound I-225 (0.056 g, 37% yield) as a brown powder.

1H-NMR (δ ppm TMS / d6-DMSO): 1.51 (9H, s),2.41 (2H, m), 3.72 (1H, s), 3.95-4.05 (4H, m), 4.30 (1H, d, J = 11.7 Hz), 5.16 (2H, s), 5.32 (1H, s), 6.91 (1H, d, J = 8.4 Hz), 7.21 (1H, dd, J = 2.3 Hz, 8.5 Hz), 7.33 (1H, d, J = 7.0 Hz), 7.80 (1H, s), 7.93 (1H, br) 8.14 (1H, t, J = 7.2 Hz), 8.41 (1H, s), 8.66 (1H, s), 9.68 (1H, s)

0 0 1 5

### Example 11 Synthesis of Compound I-246

### Step 1 Synthesis of Compound 50

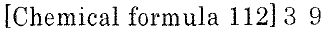

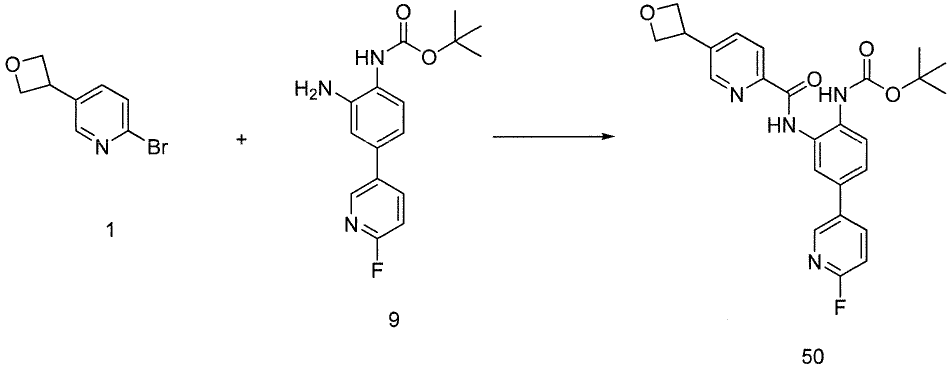

To a solution of Compound 1 (0.400 g, 1.869 mmol) and THF (2.0 mL) wa s added 1.57 mol/L normal butyllithium THF solution (1.428 mL, 2.242 mmol) u nder cooling with a dry ice/acetone bath, the mixture was stirred at -78°C for 1 0 minutes. Dry ice was added to the reaction mixture, and the mixture was sti rred at -78°C for 30 minutes. HATU (924 mg, 2.429 mmol) and Compound 9 (4 53 mg, 1.495 mg) were added to the reaction solution, and the mixture was stirr ed at room temperature for 5 hours. Water (10 mL) was added to the reaction solution, and the mixture was extracted with ethyl acetate (50 mL). The organi c layer was washed with water (10 mL) and brine (10 mL), dried over anhydrou s sodium sulfate, and concentrated under reduced pressure. The residue was pu rified by silica gel chromatography (ethyl acetate/hexane) to give Compound 50 ( 0.102 g, yield 12%) as a white powder.

1H-NMR (δ ppm TMS / d6-DMSO): 1.51 (1H, s), 4.45 (1H, m), 4.67 (2H, t, J = 6.3 Hz), 5.01 (2H, dd, J = 6.1 Hz, 8.3 Hz), 7.31 (1H, dd, J = 2.7 Hz, 8.5 Hz), 7.42 (1H, d, J = 8.4 Hz), 7.54 (1H, dd, J = 2.0 Hz, 8.3 Hz), 8.18-8.23 (2H, m), 8.26-8.30 (2H, m), 8.54 (1H, d, J = 2.0 Hz), 8.64 (1H, s), 9.24 (1H, s), 10.57 (1H, s)

### Step 2 Synthesis of Compound I-246

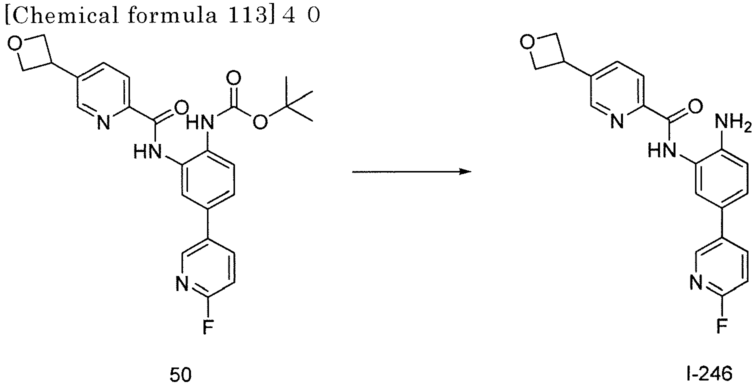

To a solution of Compound 50 (0.100 g, 0.215 mmol) and dioxane (2.0 mL) was added Zinc bromide (242 mg, 1.076 mmol), and the mixture was stirred at 80°C. for 2 hours. After adding saturated sodium hydrogen carbonate aqueous solution (10 mL) and ethyl acetate (10 mL) to the reaction solution, the precipitated solid was removed by filtration. The organic layer of the filtrate was washed with saturated sodium hydrogen carbonate aqueous solution (10 mL) and brine (10 mL), dried over anhydrous sodium sulfate, and concentrated under reduced pressure. The residue was purified by silica gel chromatography (ethyl acetate/hexane) to give Compound I-246 (0.035 g, 46% yield) as a light brown powder.

1H-NMR (δ ppm TMS / d6-DMSO): 4.41-4.48 (1H, m), 4.69 (2H, t, J = 6.3 Hz), 5.01 (2H, dd, J = 6.0 Hz, 8.3 Hz), 5.20 (2H, s), 6.93 (1H, d, J = 8.4 Hz), 7.21 (1H, dd, J = 2.9 Hz, 8.5 Hz), 7.36 (1H, dd, J = 2.2 Hz, 8.3 Hz), 7.82 (1H, d, J = 2.1 Hz), 8.13-8.30 (3H, m), 8.42 (1H, d, J = 2.3 Hz), 8.73 (1H, s), 10.15 (1H, s)

0 0 1 6

### Example 12 Synthesis of Compound 1-218

### Step 1 Synthesis of Compound 53

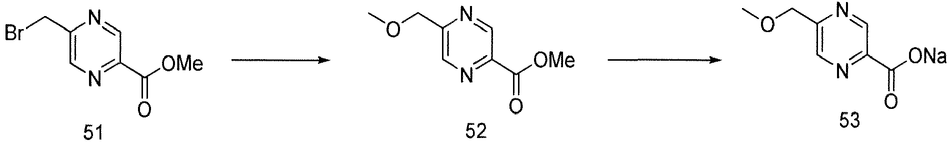

To a solution of Compound 51 (1.49 g, 6.45 mmol) and methanol (15 mL) was added potassium carbonate (1.34 g, 370 mmol), and the mixture was stirred at room temperature for 8 hours. Water was added to the reaction mixture, and the mixture was extracted with ethyl acetate. It was washed with brine and dried over anhydrous magnesium sulfate. The reaction mixture was concentrated under reduced pressure to give Compound 52 (1.34 g).

To a solution of Compound 52 (1.17 g, 6.42 mmol), THF (6 mL) and methanol (6 mL) was added sodium hydroxide aqueous solution (1 mol/L, 6.74 mL, 6.74 mmol), and the mixture was stirred at room temperature for 2 hours. The reaction mixture was concentrated under reduced pressure, ethyl acetate was added to the residue, and the resulting powder was collected by filtration to give Compound 53 as a yellow powder (1.06 g, 5.58 mmol, yield: 87%).

1H-NMR (δ ppm TMS / d6-DMSO): 3.37 (3H, s), 4.54 (2H, s), 8.50 (1H, s), 9.00 (1H, s)

### Step 2 Synthesis of Compound 1-218

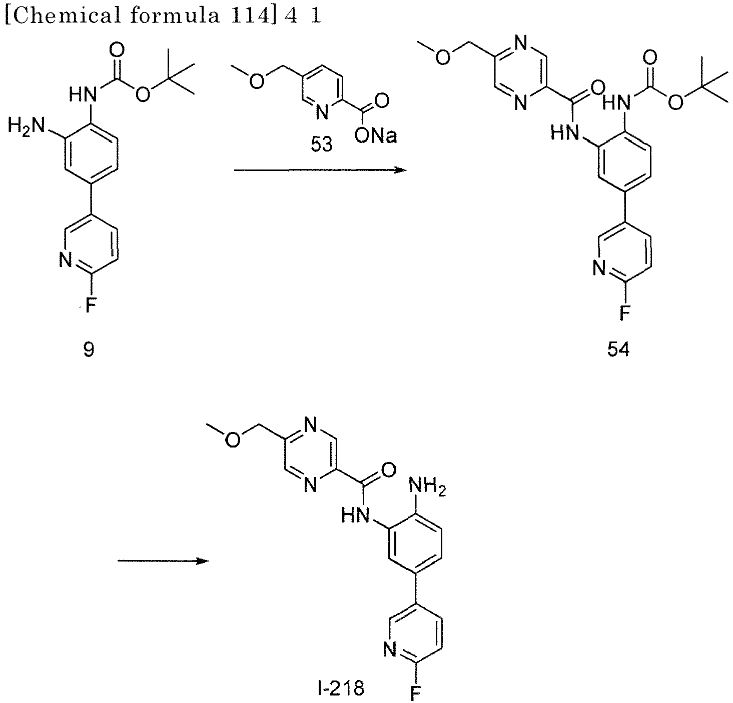

To a suspension of Compound 9 (139 mg, 0.458 mmol), Compound 53 (174 mg, 0.916 mmol) and DMF (1 mL) was added HATU (436 mg, 1.146 mmol), and the mixture was stirred at room temperature overnight. Water was added to the reaction solution, and the precipitated solid was collected by filtration to give Compound 54 (175 mg, yield: 84%) as a yellow powder.

To a suspension of Compound 54 (123 mg, 0.27 mmol) and dichloromethane (1.2 mL) were added 2,6-Lutidine (0.19 mL, 1.63 mmol) and trimethylsilyl trifluoromethanesulfonate (0.25 mL, 1.36 mmol) under ice-cooling. The mixture was stirred at room temperature for 1 hour. Water was added to the reaction mixture, and the mixture was extracted with chloroform. The organic layer was washed with brine, dried over anhydrous sodium sulfate, and concentrated under reduced pressure. Ethyl acetate and hexane were added to the residue, and the resulting powder was collected by filtration to give Compound 1-218 as an orange powder.

1H-NMR (δ ppm TMS / d6-DMSO): 3.45 (3H, s), 4.71 (2H, s), 5.26 (1H, s), 6.91 (1H, d, J = 8.4 Hz), 7.21 (1H, dd, J = 8.4 Hz, 2.8 Hz), 7.39 (1H, J = 8.4 Hz, 2.1 Hz), 7.70 (1H, d, J = 2.0 Hz), 8.15 (1H, td, J = 8.2 Hz, 2.6 Hz), 8.42 (1H, d, J = 2.1 Hz), 8.80 (1H, s), 9.24 (1H, s), 10.2 (1H, s)

0 0 1 7

### Example 13 Synthesis of Compound I-300

### Step 1 Synthesis of Compound 57

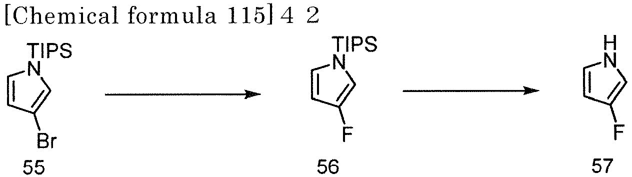

To a solution of Compound 55 (8.00 g, 26.5 mmol) and THF (80 mL) was add normal butyllithium hexane solution (2.8 mol / L, 9.92 mL, 27.8 mmol) at -78°C, and the mixture was stirred at the same temperature for 1 hour. Then, N-fluorobenzenesulfonimide (10.0 g, 31.8 mmol) was added, the mixture was warmed to room temperature, and stirred for 1 hour. Saturated ammonium chloride aqueous solution was added to the reaction mixture, and the mixture was extracted with ethyl acetate. The organic layer was washed with brine and dried over anhydrous magnesium sulfate. The reaction mixture was concentrated under reduced pressure, and the residue was purified by silica gel chromatography (ethyl acetate/hexane) to give Compound 56 (5.70 g, yield: 89%) as a colorless oil.

To a solution of Compound 56 (5.70 g, 23.6 mmol) and dichloromethane (57 mL) was added tetrabutylammonium fluoride (1 mol/L, 23.6 mL, 23.6 mmol), and the mixture was stirred at room temperature for 1 hour. Saturated ammonium chloride aqueous solution was added to the reaction mixture, and the mixture was extracted with ethyl acetate. The organic layer was washed with water and brine, dried over anhydrous sodium sulfate, and concentrated under reduced pressure to give crude Compound 57 (4.32 g, purity: 47%) as yellow oil.

1H-NMR (δ ppm TMS / CDCl3): 5.98-6.00 (1H, m), 6.50-6.54 (2H, m)

### Step 2 Synthesis of Compound 60

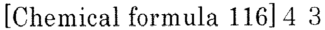

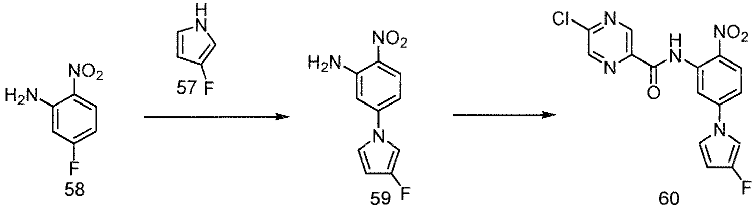

To a suspension of Compound 58 (2.11 g, 13.5 mmol), potassium carbonate (5.60 g, 40.5 mmol) and DMSO (63 mL) was added Compound 57 (3.68 g, 20.3 mmol, purity: 47%). The mixture was stirred at 100°C for 22 hours. Saturated ammonium chloride aqueous solution was added to the reaction mixture, and the mixture was extracted with ethyl acetate. The organic layer was washed with water and brine, and dried over anhydrous magnesium sulfate. The reaction mixture was concentrated under reduced pressure, and the residue was purified by silica gel chromatography (ethyl acetate/hexane) to give Compound 59 (1.19 g, yield: 39%) as a yellow powder.

To a solution of Compound 59 (1.00 g, 4.52 mmol), triethylamine (0.19 mL, 13.6 mmol) and dichloromethane (10 mL) was added 5-chloropyrazine-2-carbonyl chloride (1.09 g, 0.26 mmol) at room temperature for 4 hours. Saturated sodium hydrogen carbonate aqueous solution was added to the reaction mixture, and the mixture was extracted with ethyl acetate. The organic layer was washed with brine, dried over anhydrous sodium sulfate, and concentrated under reduced pressure. The residue was purified by silica gel chromatography (ethyl acetate/hexane) to give Compound 60 (603 mg, yield: 36.9%) as a yellow powder.

1H-NMR (δ ppm TMS / d6-DMSO): 6.25 (1H, s), 7.05 (2H, s), 7.19 (1H, dd, J = 9.4 Hz, 2.4 Hz), 8.40 (1H, d, J = 9.3 Hz), 8.73 (1H, s), 9.16 (1H, d, J = 2.3 Hz), 9.28 (1H, s)

### Step 3 Synthesis of Compound I-300

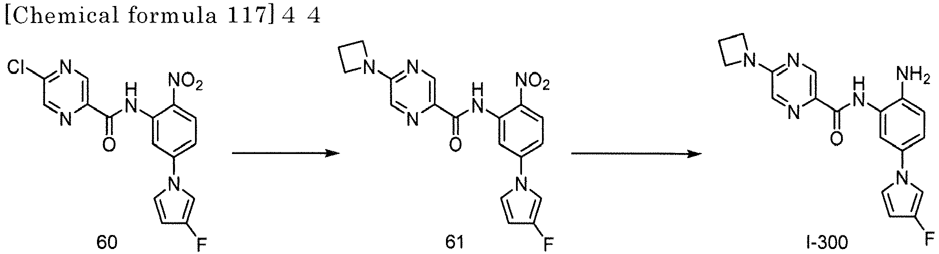

To a suspension of Compound 60 (80 mg, 0.22 mmol), triethylamine (0.06 mL, 0.44 mmol) and 1,4-dioxane (0.8 mL) was added azetidine (0.03 mL, 0.44 mmol), and the mixture was heated under reflux for 2 hours. Water was added to the reaction mixture, and the mixture was extracted with ethyl acetate. The organic layer was washed with brine and dried over anhydrous magnesium sulfate. The reaction mixture was concentrated under reduced pressure to give Compound 61 (79.0 mg, yield: 93.0%) as a yellow powder.

To a suspension of Compound 61 (79 mg, 0.20 mmol), THF (1.6 mL) and methanol (1.6 mL) was added 10% Palladium on carbon powder (132 mg, 0.06 mmol), the inside of the reaction vessel was replaced with hydrogen, and the mixture was stirred at room temperature for 1 hour. The reaction mixture was filtered through Celite (registered trademark) and concentrated under reduced pressure. The residue was purified by silica gel chromatography (ethyl acetate/hexane) to give Compound I-300 (33.0 mg, yield: 45.3%) as a yellow powder.

1H-NMR (δ ppm TMS / d6-DMSO): 2.44 (2H, t, J = 7.5 Hz), 4.19 (4H, t, J = 7.5 Hz), 4.95 (2H, br, s), 6.09 (1H, s), 6.87 (1H, d, J = 8.4 Hz), 6.97 (1H, s), 7.08-7.12 (2H, m), 7.68 (1H, s), 7.87 (1H, s), 8.69 (1H, s), 9.74 (1H, s)

0 0 1 8

### Example 14 Synthesis of Compound 1-267

### Step 1 Synthesis of Compound 64

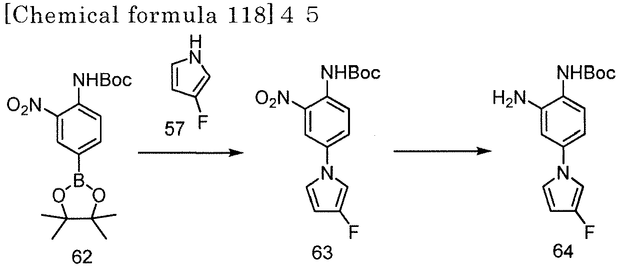

To a suspension of Compound 62 (500 mg, 1.373 mmol), potassium carbonate (569 mg, 4.12 mmol), copper acetate (374 mg, 2.06 mmol) and acetonitrile (15 mL) was added Compound 57 (372 mg, 2.06 mmol, purity: 47%), and the mixture was heated under reflux for 2 hours. Water was added to the reaction mixture, and the mixture was extracted with ethyl acetate. The organic layer was washed with brine and dried over anhydrous magnesium sulfate. The reaction mixture was concentrated under reduced pressure, and the residue was purified by silica gel chromatography (ethyl acetate/hexane) to give Compound 63 (172 mg, yield: 39.0%) as a yellow powder.

To a solution of Compound 63 (46 mg, 0.143 mmol) and methanol (0.5 mL) was added 10% palladium on carbon powder (15 mg, 0.014 mmol), the inside of the reaction vessel was replaced with hydrogen, and the mixture was stirred at room temperature for 1 hour. The reaction mixture was filtered through Celite (registered trademark) and concentrated under reduced pressure to give Compound 64 (39 mg, yield: 93.5%) as a yellow powder.

1H-NMR (δ ppm TMS / d6-DMSO): 1.51 (9H, s), 3.92 (2H, br, s), 6.07 (1H, s), 6.19 (1H, br, s), 6.77-6.81 (5H, m)

### Step 2 Synthesis of Compound I-267

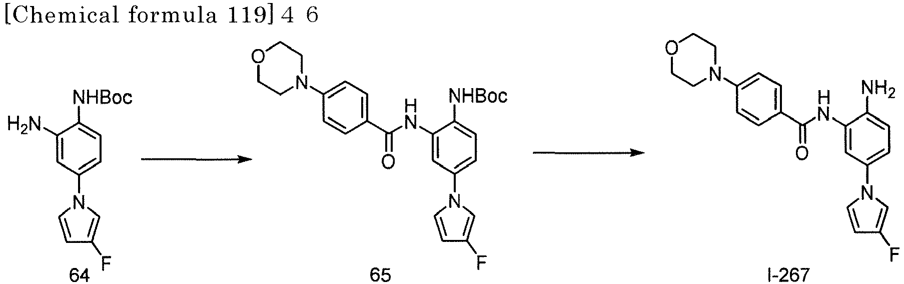

To a suspension of Compound 64 (39.0 mg, 0.137 mmol), 5-morpholine-2-pyrazine carboxylic acid (31.6 mg, 0.151 mmol) and DMF (0.4 mL) were added HATU (62.6 mg, 0.165 mmol) and triethylamine (0.02mL, 0.165mmol), the mixture was stirred at room temperature overnight. Water was added to the reaction mixture, and the mixture was extracted with ethyl acetate. The organic layer was washed with brine and dried over anhydrous magnesium sulfate. The reaction mixture was concentrated under reduced pressure, and the residue was purified by silica gel chromatography (ethyl acetate/hexane) to give Compound 65 (47 mg, yield: 70.9%) as a brown powder.

To a solution of Compound 65 (47 mg, 0.10 mmol) and dichloromethane (1.1 mL) was added hydrochloric acid dioxane solution (4 mol/L, 0.05 mL, 0.20 mmol) under ice-cooling, and the mixture was stirred at room temperature for 1 hour. Saturated sodium hydrogen carbonate aqueous solution was added to the reaction mixture, and the mixture was extracted with ethyl acetate. The organic layer was washed with brine, dried over anhydrous sodium sulfate, and concentrated under reduced pressure. The residue was purified by preparative HPLC to give Compound 1-267 (11 mg, yield: 28%).

1H-NMR (δ ppm TMS / d6-DMSO): 3.70-3.76 (8H, m), 4.97 (2H, br, s), 6.09 (1H, s), 6.88 (1H, d, J = 8.4 Hz), 6.98 (1H, s), 7.09-7.14 (2H, m), 7.69 (1H, s), 8.38 (1H, s), 8.75 (1H, s), 9.77 (1H, s)

0 0 1 9

### Example 15 Synthesis of Compound I-236

### Step 1 Synthesis of Compound I-236

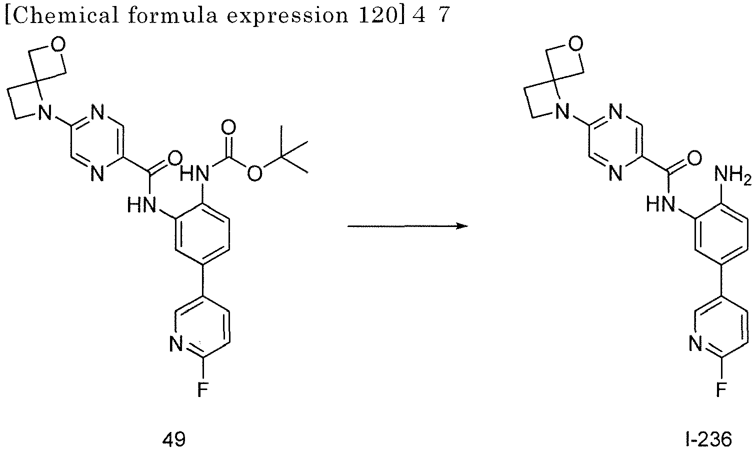

To a solution of Compound 49 (0.112 g, 0.221 mmol) and dioxane (2.2 mL) was added zinc bromide (249 mg, 1.106 mmol), and the mixture was stirred at 80°C for 5 hours. After adding saturated sodium hydrogen carbonate aqueous solution (10 mL) and ethyl acetate (10 mL) to the reaction mixture, the precipitated solid was removed by filtration. The organic layer of the filtrate was washed with saturated sodium hydrogen carbonate aqueous solution (10 mL) and brine (10 mL), dried over anhydrous sodium sulfate, and concentrated under reduced pressure. The residue was purified by silica gel chromatography (ethyl acetate/hexane) to give Compound I-236 (0.021 g, 23% yield) as a yellow powder.

1H-NMR (δ ppm TMS / d6-DMSO): 2.69 (2H, t, J = 7.1 Hz), 3.98 (2H, t, J = 7.2 Hz), 4.69 (2H, d. J = 7.3 Hz), 5.17 (2H, s), 5.27 (2H, d, J = 7.2 Hz), 6.92 (1H, d, J = 8.3 Hz), 7.21 (1H, dd, J = 2.9 Hz, 8.6 Hz), 7.34 (1H, dd, J = 2.1 Hz, 8.3 Hz), 7.79 (1H, d, J = 2.0 Hz), 8.04 (1H, s), 8.15 (1H, td, J = 2.5 Hz, 8.3 Hz), 8.42 (1H, d, J = 2.1 Hz), 8.78 (1H, d, J = 1.1 Hz), 9.77 (1H, s)

0 0 2 0

Using the above general synthetic method or the synthetic method described in Examples, the compounds shown below were also synthesized in the same manner.

**[Table 1]**

| Example No. | Structure | RT(min) | MS |
|---|---|---|---|
| I-001 | 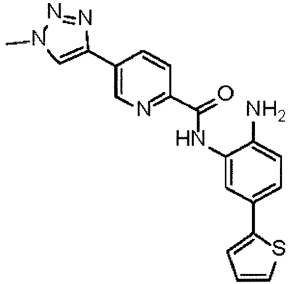 | 1.69 | 377 |
| I-002 | 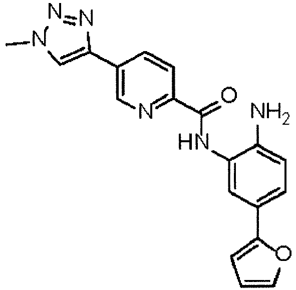 | 1.57 | 361 |
| I-003 | 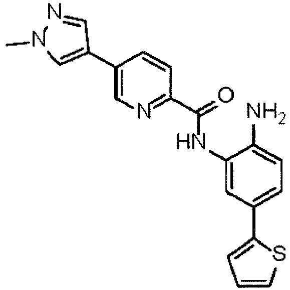 | 1.8 | 376 |
| I-004 | 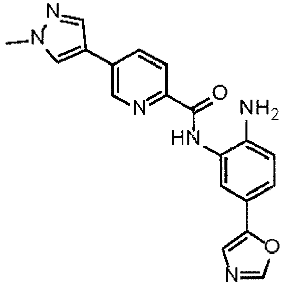 | 1.41 | 361 |

**[Table 2]**

| | | | |
|---|---|---|---|
| I-005 | 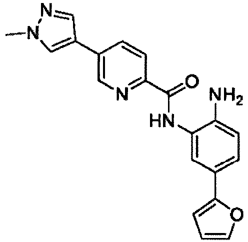 | 1.68 | 360 |
| I-006 | 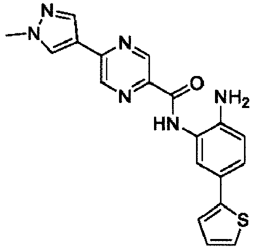 | 1.81 | 377 |
| I-007 | 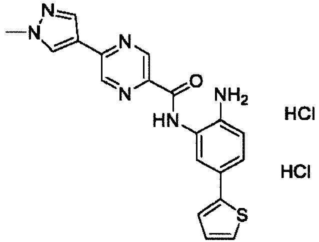 | 1.81 | 377 |
| I-008 | 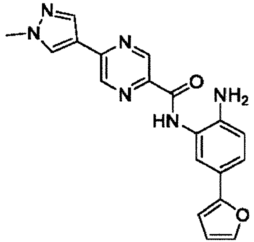 | 1.77 | 361 |
| I-009 | 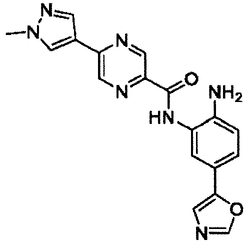 | 1.36 | 362 |

**[Table 3]**

| | | | |
|---|---|---|---|
| I-010 | 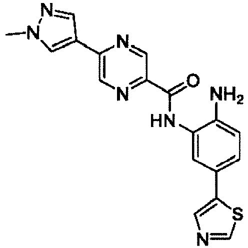 | 1.42 | 378 |
| I-011 | 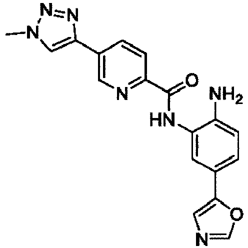 | 1.33 | 362 |
| I-012 | 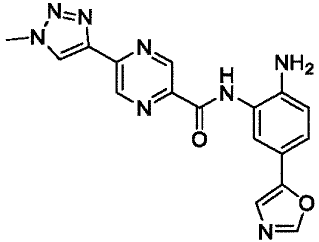 | 1.21 | 363 |
| I-013 | 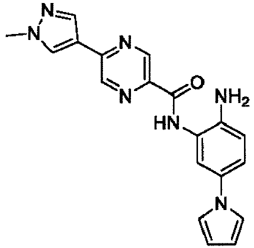 | 1.72 | 360 |
| I-014 | 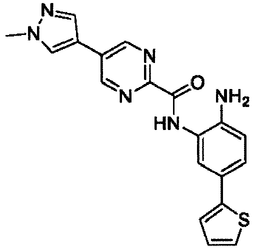 | 1.72 | 377 |

**[Table 4]**

| | | | |
|---|---|---|---|
| I-015 | 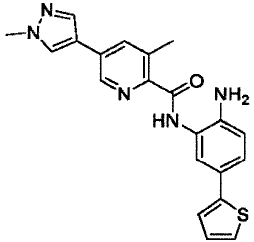 | 2.1 | 390 |
| I-016 | 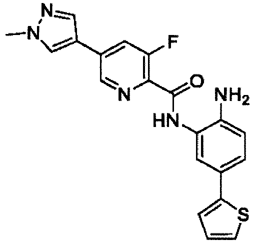 | 1.97 | 394 |
| I-017 | 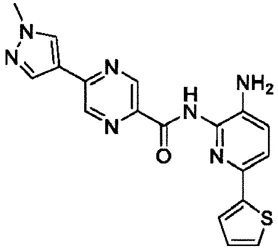 | 1.6 | 378 |
| I-018 | 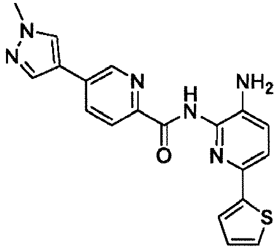 | 1.72 | 377 |
| I-019 | 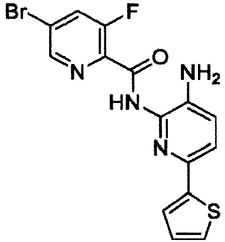 | 1.93 | 395 |

**[Table 5]**

| | | | |
|---|---|---|---|
| I-020 | 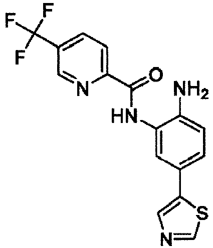 | 1.72 | 365 |
| I-021 | 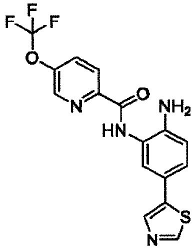 | 1.93 | 381 |
| I-022 | 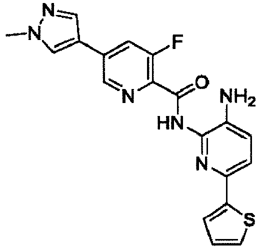 | 1.69 | 395 |
| I-023 | 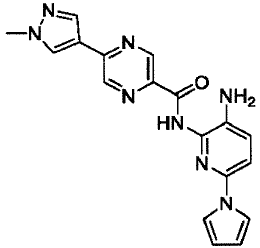 | 1.53 | 361 |
| I-024 | 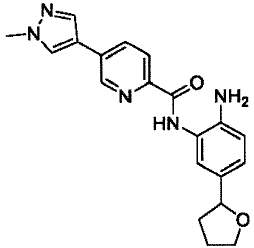 | 1.51 | 364 |

**[Table 6]**

| | | | |
|---|---|---|---|
| I-025 | 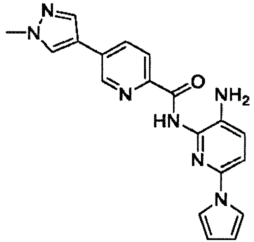 | 1.8 | 360 |
| I-026 | 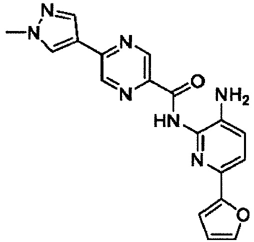 | 1.55 | 362 |
| I-027 | 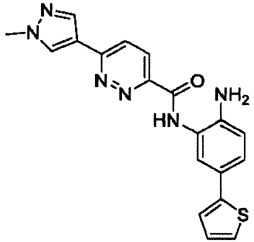 | 1.8 | 377 |
| I-028 | 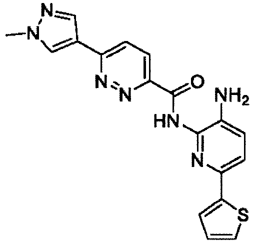 | 1.66 | 378 |
| I-029 | 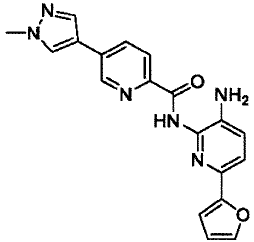 | 1.51 | 361 |

**[Table 7]**

| | | | |
|---|---|---|---|
| I-030 | 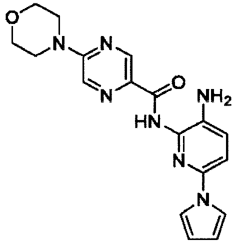 | 1.71 | 366 |
| I-031 | 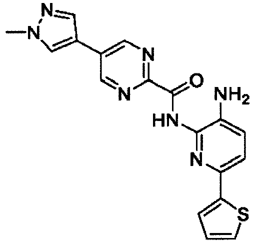 | 1.45 | 378 |
| I-032 | 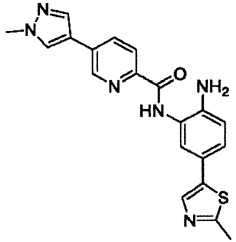 | 1.66 | 391 |
| I-033 | 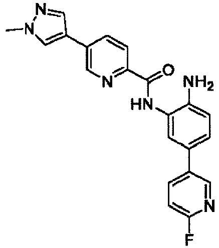 | 1.63 | 389 |
| I-034 | 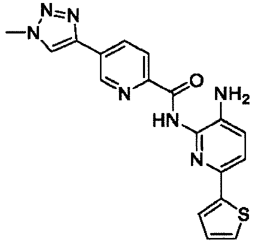 | 1.61 | 378 |

**[Table 8]**

| | | | |
|---|---|---|---|
| I-035 | 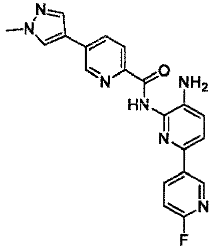 | 1.6 | 390 |
| I-036 | 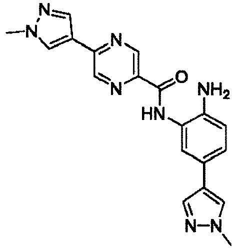 | 1.12 | 375 |
| I-037 | 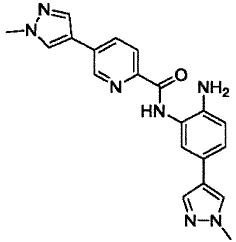 | 1.2 | 374 |
| I-038 | 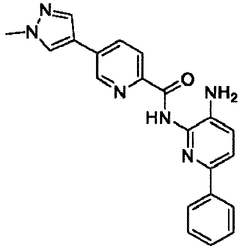 | 1.74 | 371 |
| I-039 | 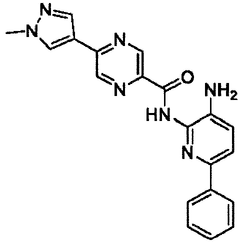 | 1.64 | 372 |

**[Table 9]**

| | | | |
|---|---|---|---|
| I-040 | 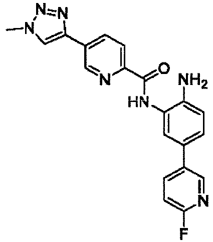 | 1.46 | 390 |
| I-041 | 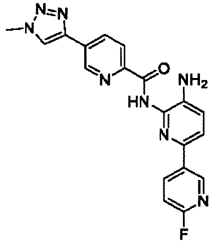 | 1.41 | 391 |
| I-042 | 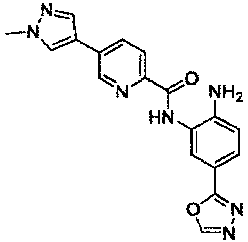 | 1.14 | 362 |
| I-043 | 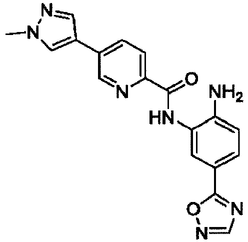 | 1.35 | 362 |
| I-044 | 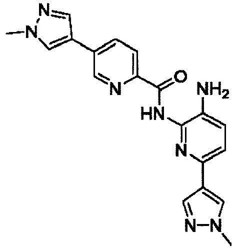 | 1.12 | 375 |

**[Table 10]**

| | | | |
|---|---|---|---|
| I-045 | 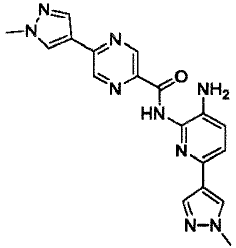 | 1.02 | 376 |
| I-046 | 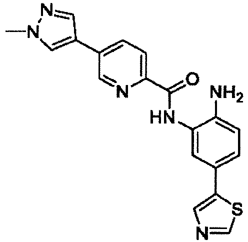 | 1.36 | 377 |
| I-047 | 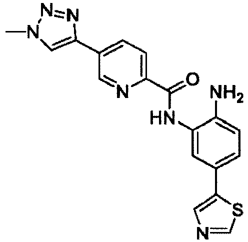 | 1.24 | 378 |
| I-048 | 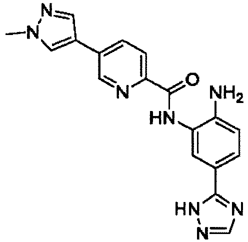 | 0.97 | 361 |
| I-049 | 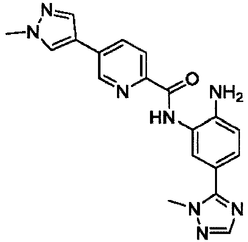 | 1.08 | 375 |

**[Table 11]**

| | | | |
|---|---|---|---|
| I-050 | 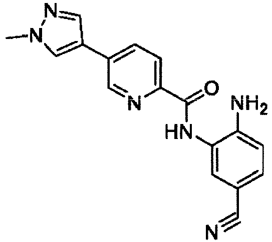 | 1.32 | 319 |
| I-051 | 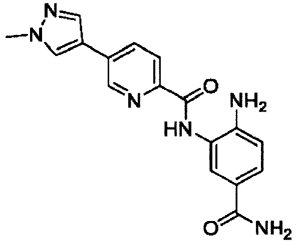 | 0.88 | 337 |
| I-052 | 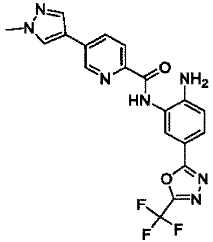 | 1.75 | 430 |
| I-053 | 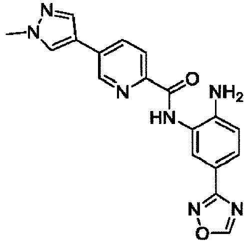 | 1.33 | 362 |
| I-054 | 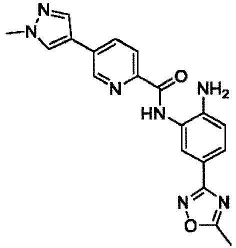 | 1.42 | 376 |

**[Table 12]**

| | | | |
|---|---|---|---|
| I-055 | 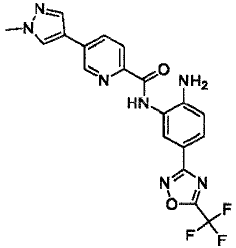 | 1.96 | 430 |
| I-056 | 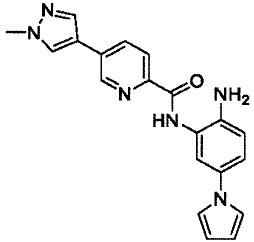 | 1.67 | 359 |
| I-057 | 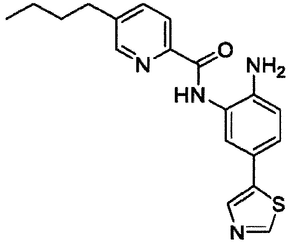 | 2.04 | 353 |
| I-058 | 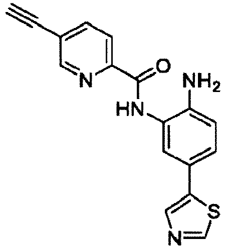 | 1.53 | 321 |
| I-059 | 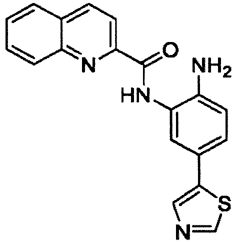 | 1.76 | 347 |

**[Table 13]**

| | | | |
|---|---|---|---|
| I-060 | 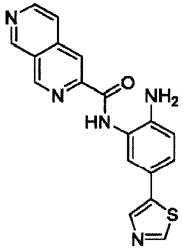 | 1.2 | 348 |
| I-061 | 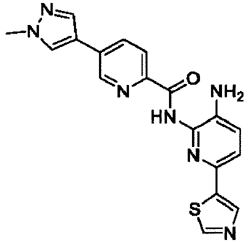 | 1.31 | 378 |
| I-062 | 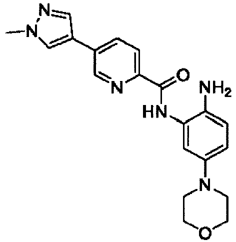 | 0.91 | 379 |
| I-063 | 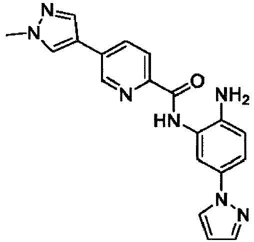 | 1.37 | 360 |
| I-064 | 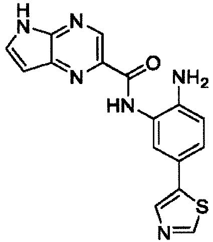 | 1.21 | 337 |

**[Table 14]**

| | | | |
|---|---|---|---|
| I-065 | 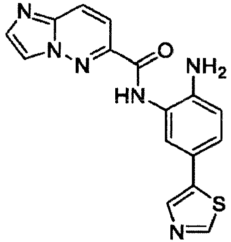 | 0.97 | 337 |
| I-066 | 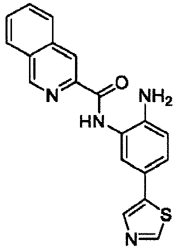 | 1.67 | 347 |
| I-067 | 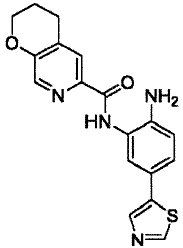 | 1.55 | 353 |
| I-068 | 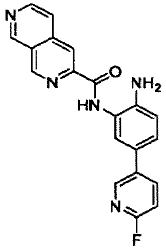 | 1.42 | 360 |
| I-069 | 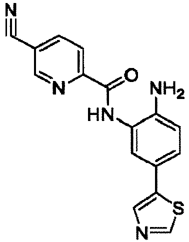 | 1.34 | 322 |

**[Table 15]**

| | | | |
|---|---|---|---|
| I-070 | 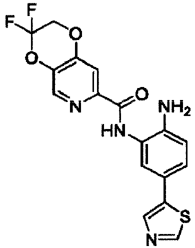 | 1.67 | 391 |
| I-071 | 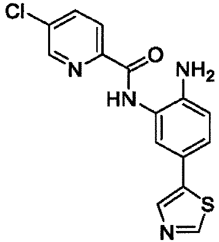 | 1.58 | 331 |
| I-072 | 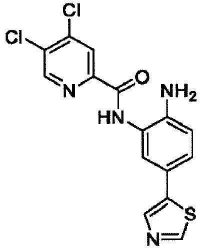 | 1.82 | 365 |
| I-073 | 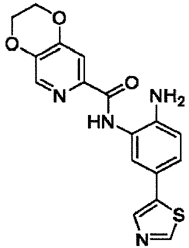 | 1.53 | 355 |
| I-074 | 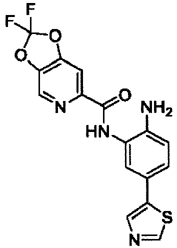 | 1.88 | 377 |

**[Table 16]**

| | | | |
|---|---|---|---|
| I-075 | 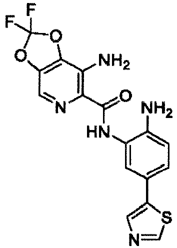 | 1.91 | 392 |
| I-076 | 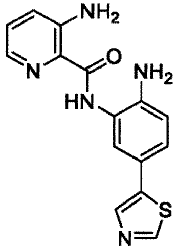 | 1.55 | 312 |
| I-077 | 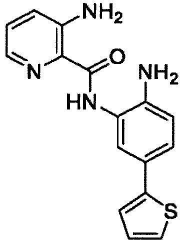 | 2.01 | 311 |
| I-078 | 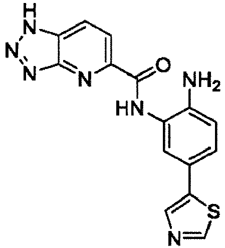 | 1.29 | 338 |
| I-079 | 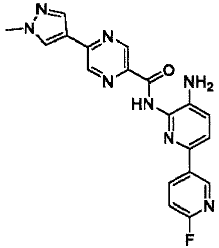 | 1.63 | 391 |

**[Table 17]**

| | | | |
|---|---|---|---|
| I-080 | 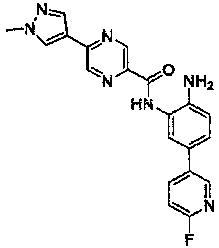 | 1.7 | 390 |
| I-081 | 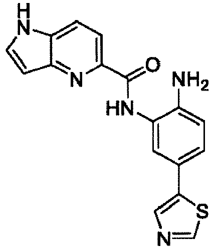 | 1.16 | 336 |
| I-082 | 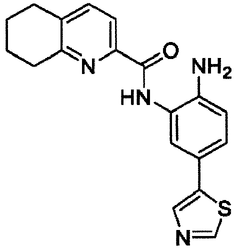 | 1.9 | 351 |
| I-083 | 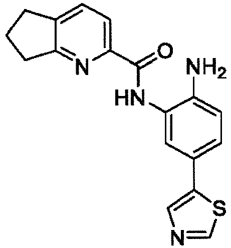 | 1.77 | 337 |
| I-084 | 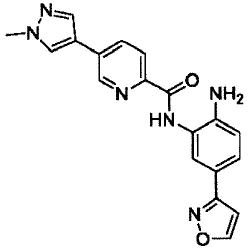 | 1.53 | 361 |

**[Table 18]**

| | | | |
|---|---|---|---|
| I-085 | 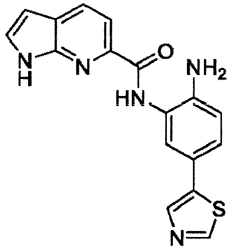 | 1.52 | 336 |
| I-086 | 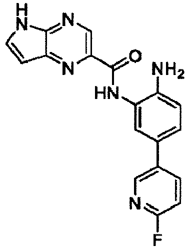 | 1.55 | 349 |
| I-087 | 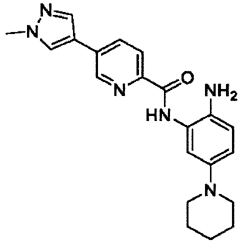 | 1.19 | 377 |
| I-088 | 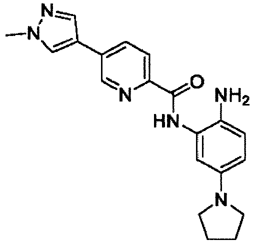 | 1.37 | 363 |
| I-089 | 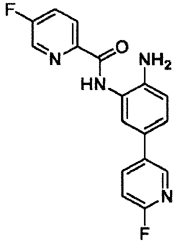 | 1.7 | 327 |

**[Table 19]**

| | | | |
|---|---|---|---|
| I-090 | 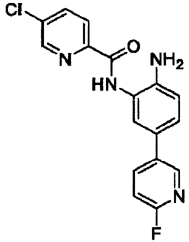 | 1.66 | 379 |
| I-091 | 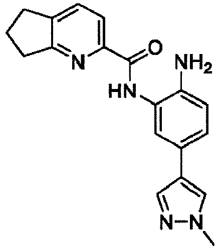 | 1.52 | 334 |
| I-092 | 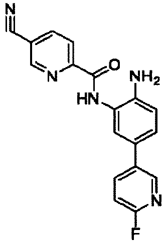 | 1.6 | 334 |
| I-093 | 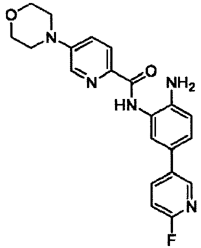 | 1.62 | 394 |
| I-094 | 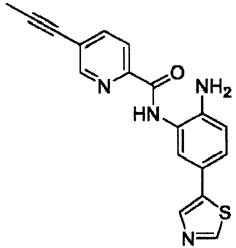 | 1.76 | 335 |

**[Table 20]**

| | | | |
|---|---|---|---|
| I-095 | 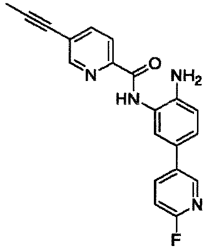 | 1.95 | 347 |
| I-096 | 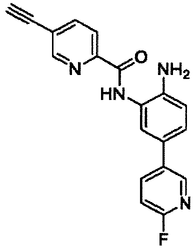 | 1.8 | 333 |
| I-097 | 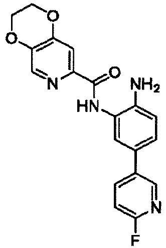 | 1.64 | 367 |
| I-098 | 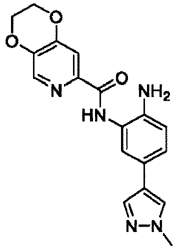 | 1.29 | 352 |
| I-099 | 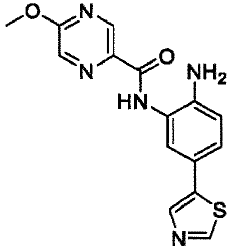 | 1.43 | 328 |

**[Table 21]**

| | | | |
|---|---|---|---|
| I-100 | 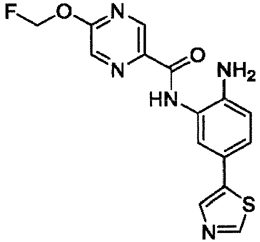 | 1.43 | 346 |
| I-101 | 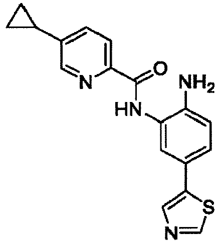 | 1.74 | 337 |
| I-102 | 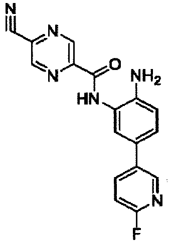 | 1.65 | 335 |
| I-103 | 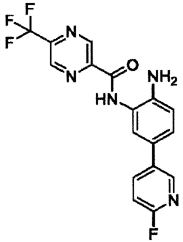 | 1.93 | 378 |
| I-104 | 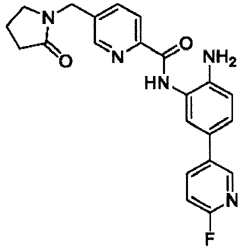 | 1.52 | 406 |

**[Table 22]**

| | | | |
|---|---|---|---|
| I-105 | 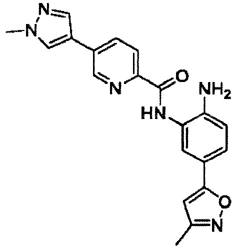 | 1.67 | 375 |
| I-106 | 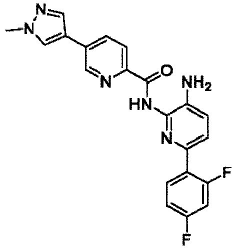 | 1.93 | 407 |
| I-107 | 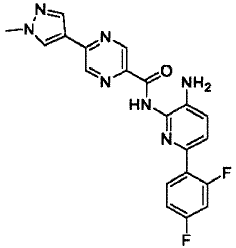 | 1.82 | 408 |
| I-108 | 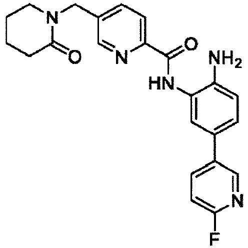 | 1.6 | 420 |
| I-109 | 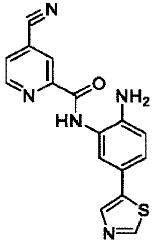 | 1.47 | 322 |

**[Table 23]**

| | | | |
|---|---|---|---|
| I-110 | 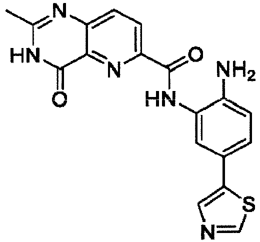 | 1.21 | 379 |
| I-111 | 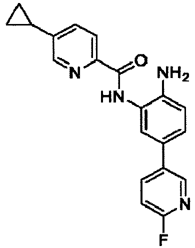 | 1.92 | 349 |
| I-112 | 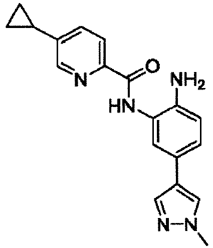 | 1.56 | 334 |
| I-113 | 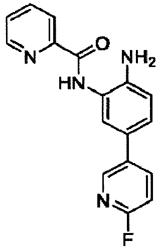 | 1.61 | 309 |
| I-114 | 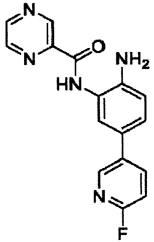 | 1.42 | 310 |

**[Table 24]**

| | | | |
|---|---|---|---|
| I-115 | 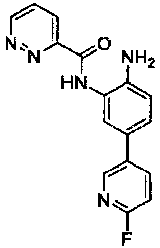 | 1.37 | 310 |
| I-116 | 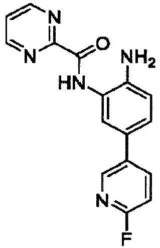 | 1.31 | 310 |
| I-117 | 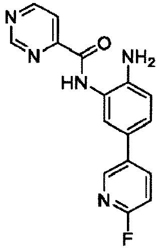 | 1.42 | 310 |
| I-118 | 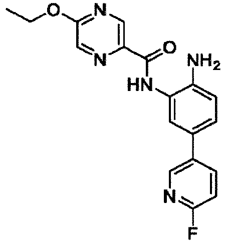 | 1.63 | 354 |
| I-119 | 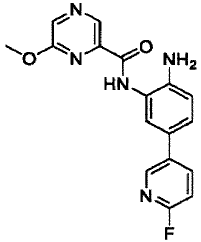 | 1.64 | 340 |

**[Table 25]**

| | | | |
|---|---|---|---|
| I-120 | 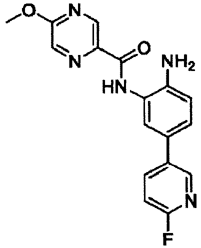 | 1.66 | 340 |
| I-121 | 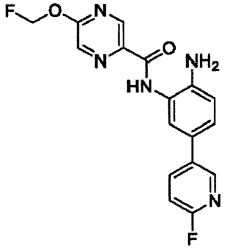 | 1.71 | 358 |
| I-122 | 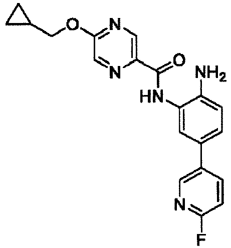 | 2.04 | 380 |
| I-123 | 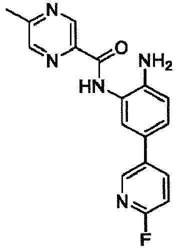 | 1.5 | 324 |
| I-124 | 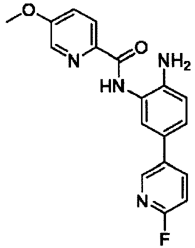 | 1.72 | 339 |

**[Table 26]**

| | | | |
|---|---|---|---|
| I-125 | 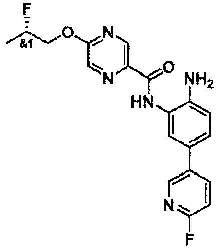 | 1.89 | 386 |
| I-126 | 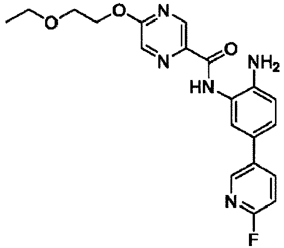 | 1.79 | 398 |
| I-127 | 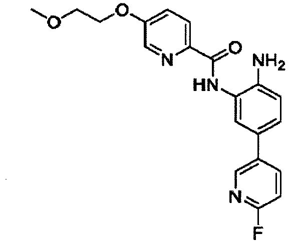 | 1.68 | 383 |
| I-128 | 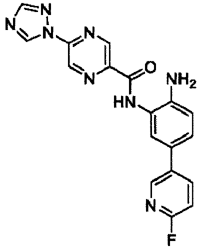 | 1.52 | 377 |
| I-129 | 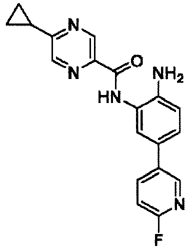 | 1.78 | 350 |

**[Table 27]**

| | | | |
|---|---|---|---|
| I-130 | 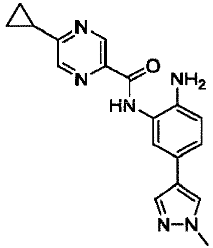 | 1.42 | 335 |
| I-131 | 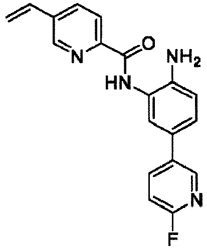 | 1.88 | 335 |
| I-132 | 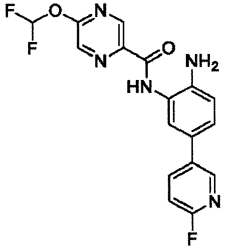 | 1.91 | 376 |
| I-133 | 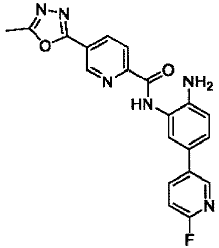 | 1.58 | 391 |
| I-134 | 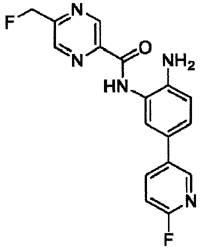 | 1.6 | 342 |

**[Table 28]**

| | | | |
|---|---|---|---|
| I-135 | 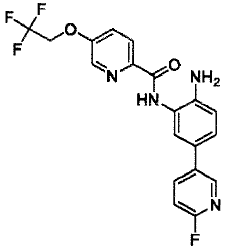 | 2.05 | 407 |
| I-136 | 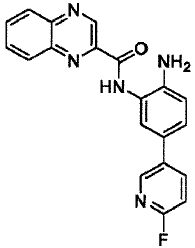 | 1.8 | 360 |
| I-137 | 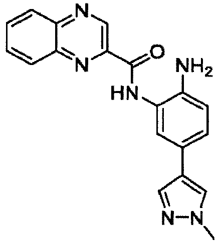 | 1.42 | 345 |
| I-138 | 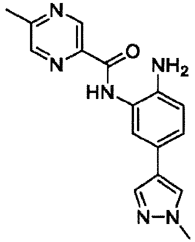 | 1.13 | 309 |
| I-139 | 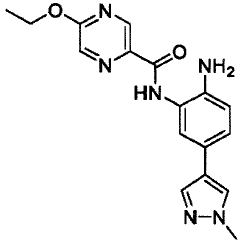 | 1.42 | 339 |

**[Table 29]**

| | | | |
|---|---|---|---|
| I-140 | 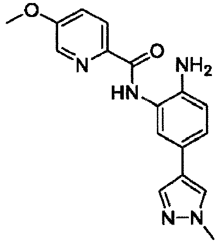 | 1.31 | 324 |
| I-141 | 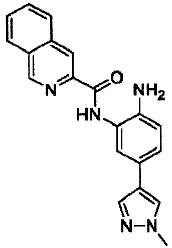 | 1.54 | 344 |
| I-142 | 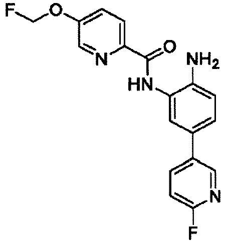 | 1.76 | 357 |
| I-143 | 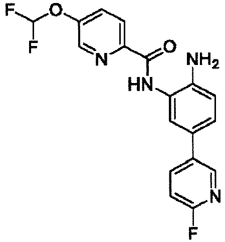 | 1.9 | 375 |
| I-144 | 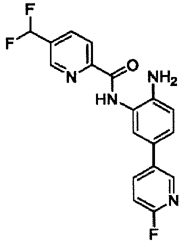 | 1.86 | 359 |

**[Table 30]**

| | | | |
|---|---|---|---|
| I-145 | 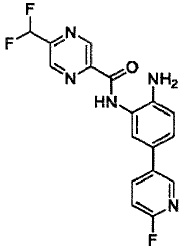 | 1.74 | 360 |
| I-146 | 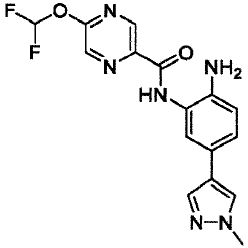 | 1.5 | 361 |
| I-147 | 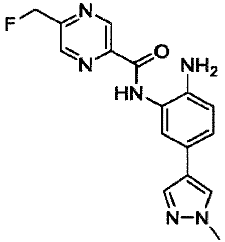 | 1.21 | 327 |
| I-148 | 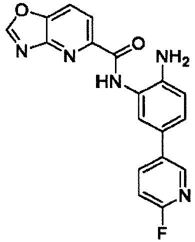 | 1.51 | 350 |
| I-149 | 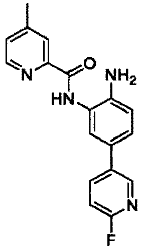 | 1.73 | 323 |

**[Table 31]**

| | | | |
|---|---|---|---|
| I-150 | 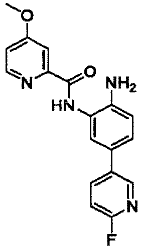 | 1.66 | 339 |
| I-151 | 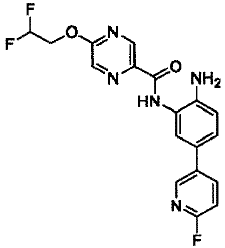 | 1.88 | 390 |
| I-152 | 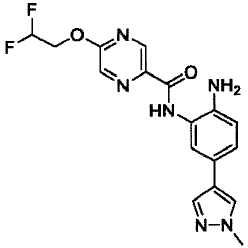 | 1.49 | 375 |
| I-153 | 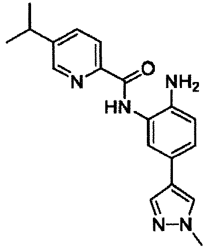 | 1.68 | 336 |
| I-154 | 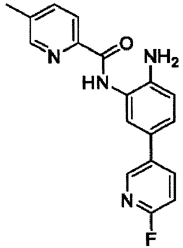 | 1.76 | 323 |

**[Table 32]**

| | | | |
|---|---|---|---|
| I-155 | 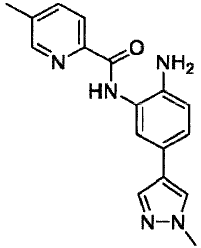 | 1.34 | 308 |
| I-156 | 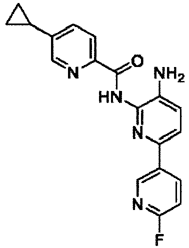 | 1.94 | 350 |
| I-157 | 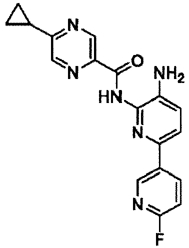 | 1.74 | 351 |
| I-158 | 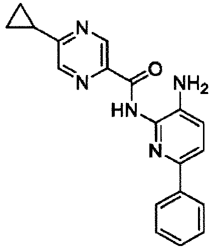 | 1.98 | 332 |
| I-159 | 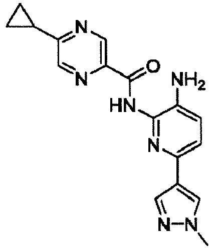 | 1.33 | 336 |

**[Table 33]**

| | | | |
|---|---|---|---|
| I-160 | 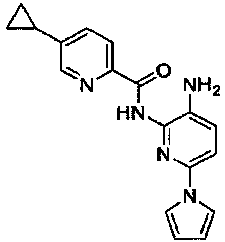 | 2.14 | 320 |
| I-161 | 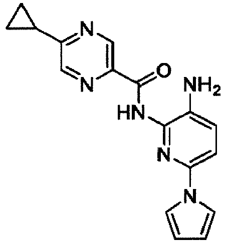 | 1.94 | 321 |
| I-162 | 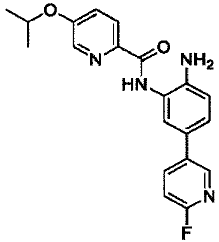 | 2.04 | 367 |
| I-163 | 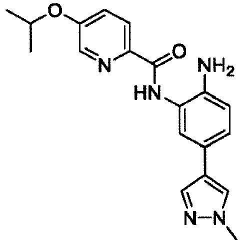 | 1.62 | 352 |
| I-164 | 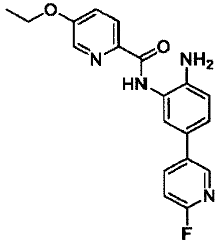 | 1.89 | 353 |

**[Table 34]**

| | | | |
|---|---|---|---|
| I-165 | 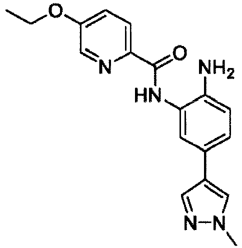 | 1.49 | 338 |
| I-166 | 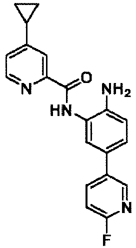 | 1.92 | 349 |
| I-167 | 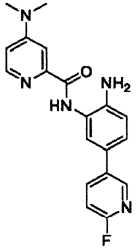 | 1.12 | 352 |
| I-168 | 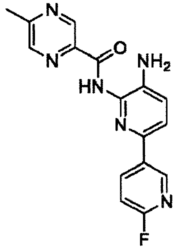 | 1.39 | 325 |
| 1-169 | 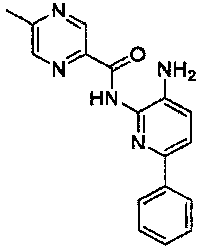 | 1.66 | 306 |

**[Table 35]**

| | | | |
|---|---|---|---|
| I-170 | 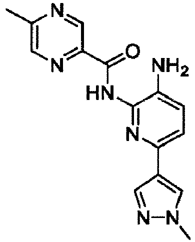 | 0.96 | 310 |
| I-171 | 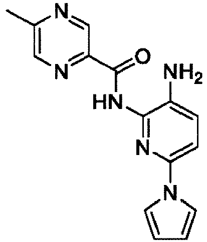 | 1.56 | 295 |
| I-172 | 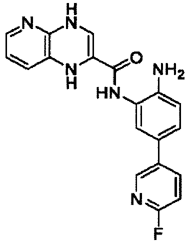 | 1.38 | 363 |
| I-173 | 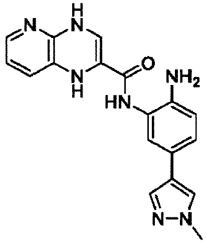 | 1.07 | 348 |
| I-174 | 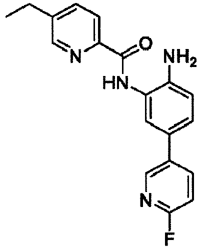 | 1.92 | 337 |

**[Table 36]**

| | | | |
|---|---|---|---|
| I-175 | 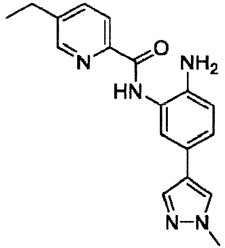 | 1.52 | 322 |
| I-176 | 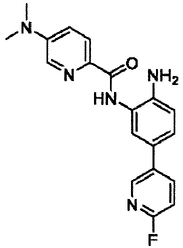 | 1.73 | 352 |
| I-177 | 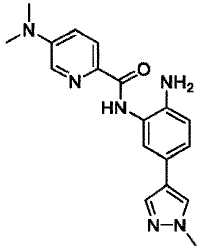 | 1.3 | 337 |
| I-178 | 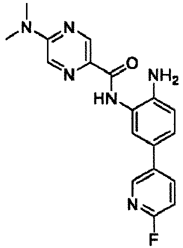 | 1.6 | 353 |
| I-179 | 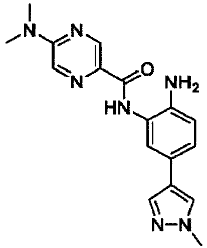 | 1.21 | 338 |

**[Table 37]**

| | | | |
|---|---|---|---|
| I-180 | 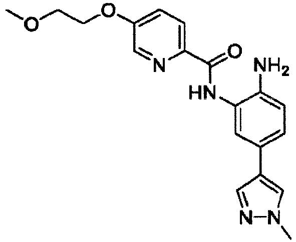 | 1.3 | 368 |
| I-181 | 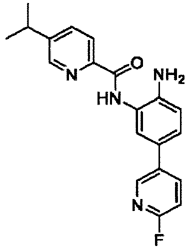 | 2.09 | 351 |
| I-182 | 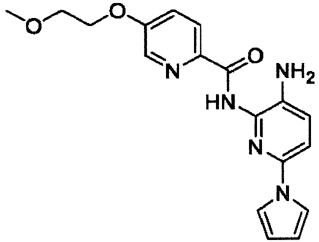 | 1.84 | 354 |
| I-183 | 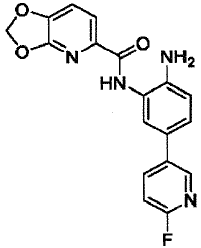 | 1.67 | 353 |
| I-184 | 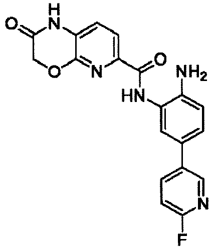 | 1.48 | 380 |

**[Table 38]**

| | | | |
|---|---|---|---|
| I-185 | 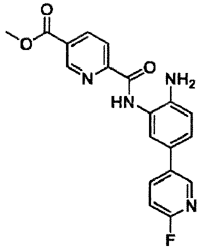 | 1.78 | 367 |
| I-186 | 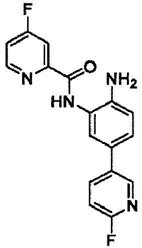 | 1.76 | 327 |
| I-187 | 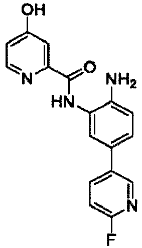 | 1.25 | 325 |
| I-188 | 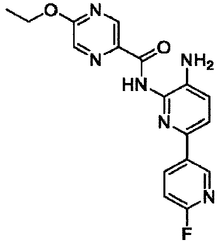 | 1.95 | 355 |
| I-189 | 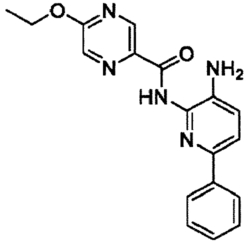 | 1.8 | 336 |

**[Table 39]**

| | | | |
|---|---|---|---|
| I-190 | 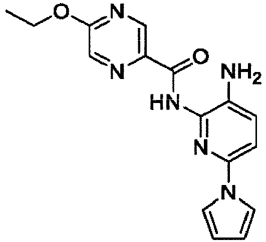 | 2 | 325 |
| I-191 | 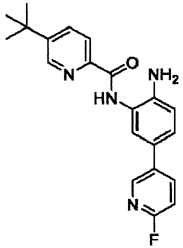 | 2.2 | 365 |
| I-192 | 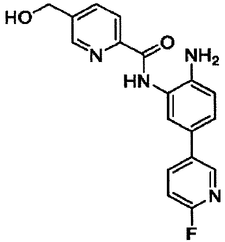 | 1.42 | 339 |
| I-193 | 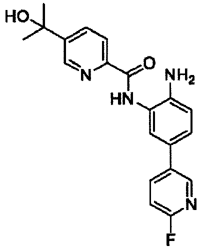 | 1.6 | 367 |
| I-194 | 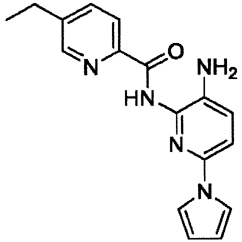 | 2.08 | 308 |

**[Table 40]**

| | | | |
|---|---|---|---|
| I-195 | 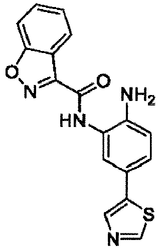 | 1.68 | 337 |
| I-196 | 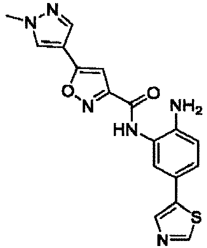 | 1.56 | 367 |
| I-197 | 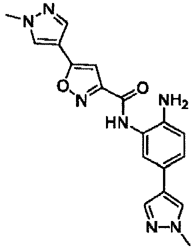 | 1.25 | 364 |
| I-198 | 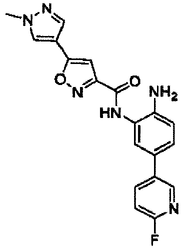 | 1.66 | 379 |
| I-199 | 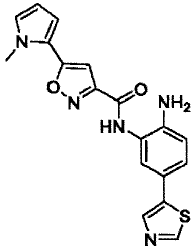 | 1.8 | 366 |

**[Table 41]**

| | | | |
|---|---|---|---|
| I-200 | 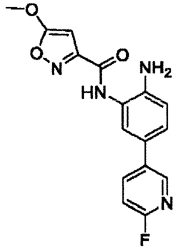 | 1.46 | 329 |

**[Table 42]**

| Example No. | Structure | RT(min) | MS |
|---|---|---|---|
| I-201 | 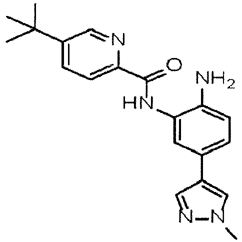 | 1.82 | 350 |
| I-202 | 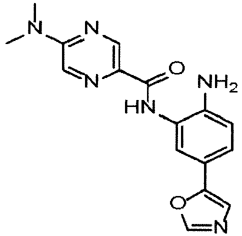 | 1.38 | 325 |
| I-203 | 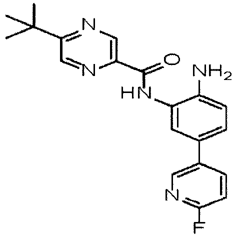 | 2.05 | 366 |
| I-204 | 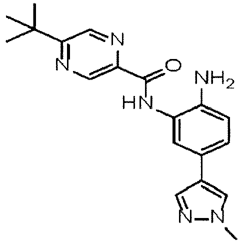 | 1.68 | 351 |
| I-205 | 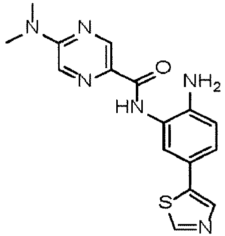 | 1.44 | 341 |

**[Table 43]**

| | | | |
|---|---|---|---|
| I-206 | 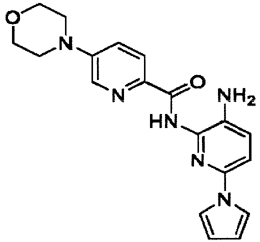 | 1.81 | 365 |
| I-207 | 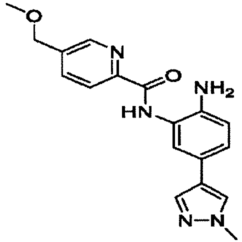 | 1.27 | 338 |
| I-208 | 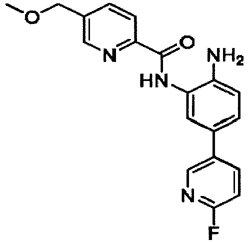 | 1.67 | 353 |
| I-209 | 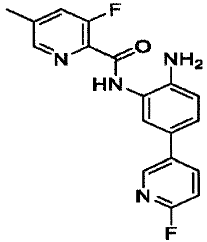 | 1.69 | 341 |
| I-210 | 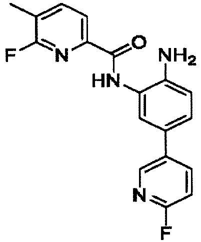 | 1.93 | 341 |

**[Table 44]**

| | | | |
|---|---|---|---|
| I-211 | 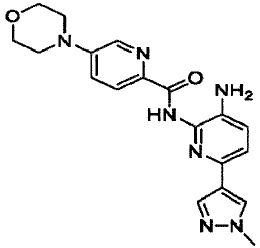 | 1.14 | 380 |
| I-212 | 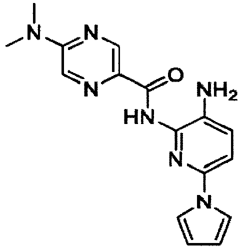 | 1.76 | 324 |
| I-213 | 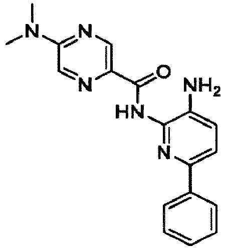 | 1.7 | 335 |
| I-214 | 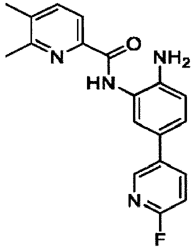 | 1.89 | 337 |
| I-215 | 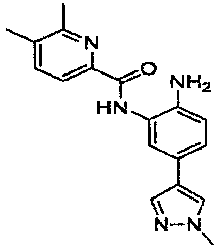 | 1.49 | 322 |

**[Table 45]**

| | | | |
|---|---|---|---|
| 1-216 | 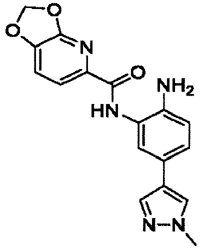 | 1.25 | 338 |
| I-217 | 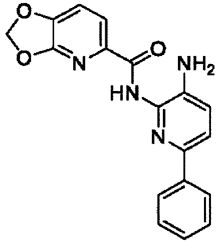 | 1.8 | 335 |
| I-218 | 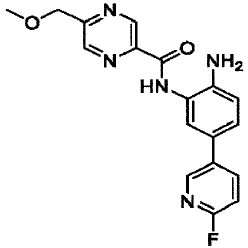 | 1.46 | 354 |
| 1-219 | 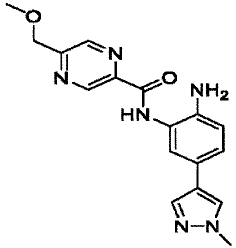 | 1.06 | 339 |
| I-220 | 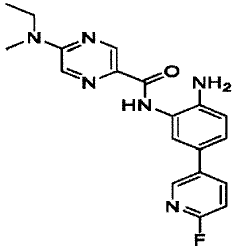 | 1.76 | 367 |

**[Table 46]**

| | | | |
|---|---|---|---|
| I-221 | 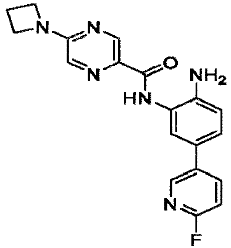 | 1.6 | 365 |
| I-222 | 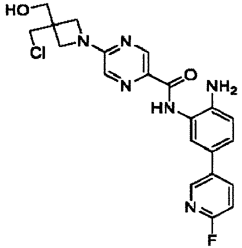 | 1.64 | 443 |
| I-223 | 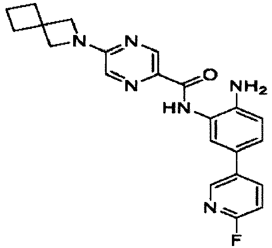 | 1.99 | 405 |
| I-224 | 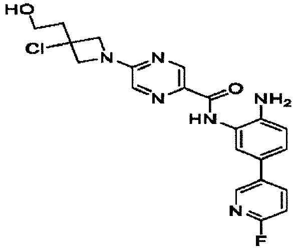 | 1.69 | 443 |
| I-225 | 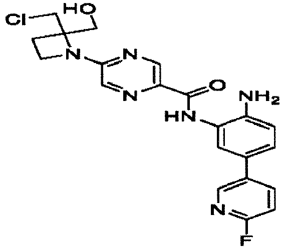 | 1.75 | 443 |

**[Table 47]**

| | | | |
|---|---|---|---|
| I-226 | 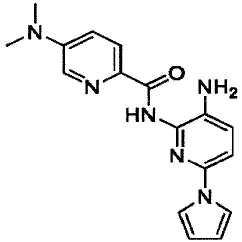 | 1.85 | 323 |
| I-227 | 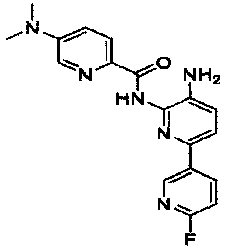 | 1.68 | 353 |
| I-228 | 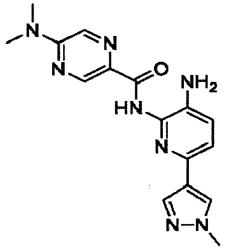 | 1.02 | 339 |
| I-229 | 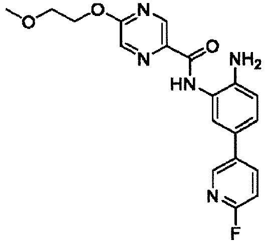 | 1.65 | 384 |
| I-230 | 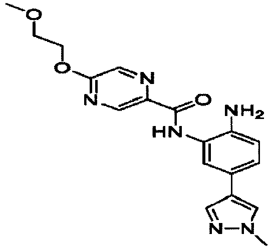 | 1.29 | 369 |

**[Table 48]**

| | | | |
|---|---|---|---|
| I-231 | 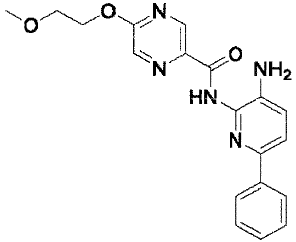 | 1.82 | 366 |
| I-232 | 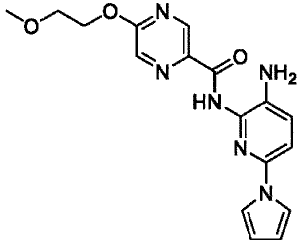 | 1.78 | 355 |
| I-233 | 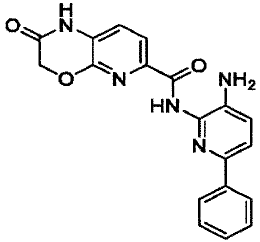 | 1.6 | 362 |
| I-234 | 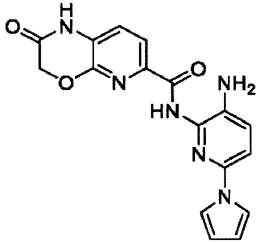 | 1.56 | 351 |
| I-235 | 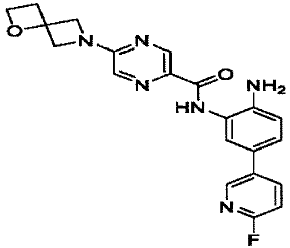 | 1.54 | 407 |

**[Table 49]**

| | | | |
|---|---|---|---|
| I-236 | 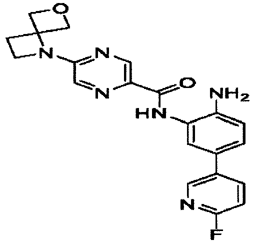 | 1.57 | 407 |
| I-237 | 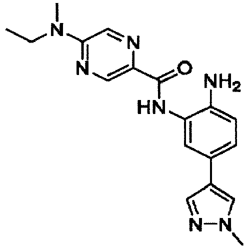 | 1.42 | 352 |
| I-238 | 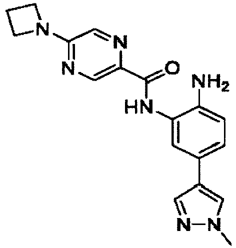 | 1.24 | 350 |
| I-239 | 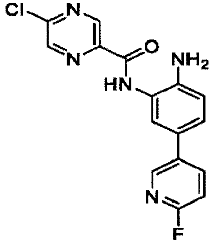 | 1.7 | 344 |
| I-240 | 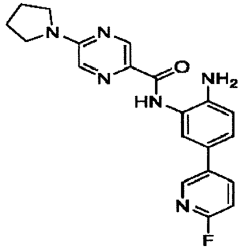 | 1.71 | 379 |

**[Table 50]**

| | | | |
|---|---|---|---|
| I-241 | 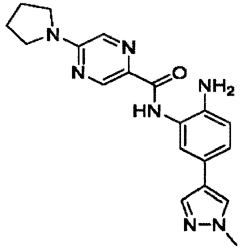 | 1.34 | 364 |
| I-242 | 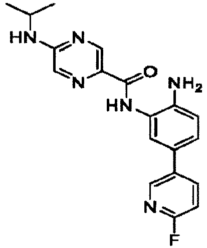 | 1.69 | 367 |
| I-243 | 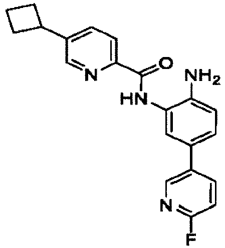 | 2.17 | 363 |
| I-244 | 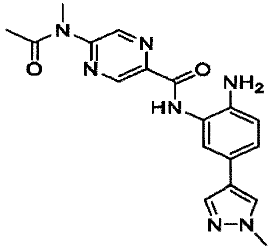 | 1.07 | 366 |
| I-245 | 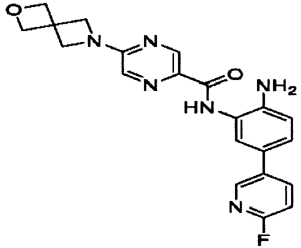 | 1.49 | 407 |

**[Table 51]**

| | | | |
|---|---|---|---|
| I-246 | 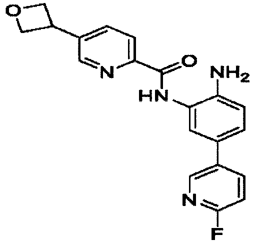 | 1.57 | 365 |
| I-247 | 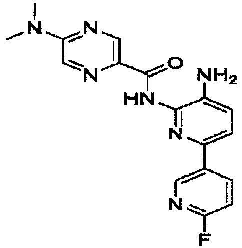 | 1.54 | 354 |
| I-248 | 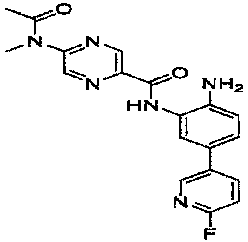 | 1.46 | 381 |
| I-249 | 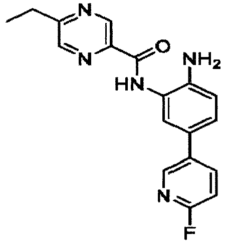 | 1.67 | 338 |
| I-250 | 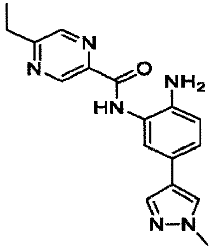 | 1.31 | 323 |

**[Table 52]**

| | | | |
|---|---|---|---|
| I-251 | 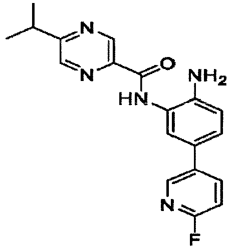 | 1.86 | 352 |
| I-252 | 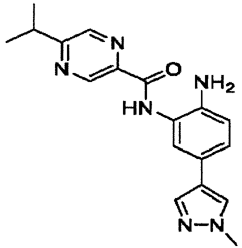 | 1.48 | 337 |
| I-253 | 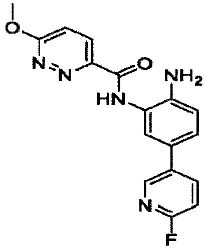 | 1.59 | 340 |
| I-254 | 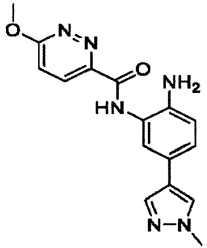 | 1.22 | 325 |
| I-255 | 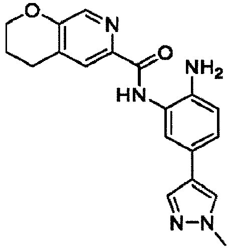 | 1.45 | 350 |

**[Table 53]**

| | | | |
|---|---|---|---|
| I-256 | 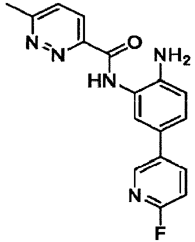 | 1.45 | 324 |
| I-257 | 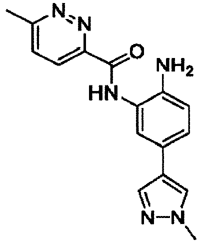 | 1.1 | 309 |
| I-258 | 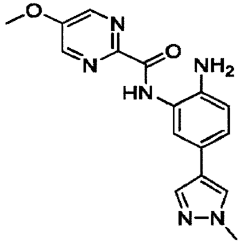 | 1.04 | 325 |
| I-259 | 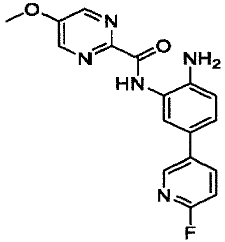 | 1.43 | 340 |
| I-260 | 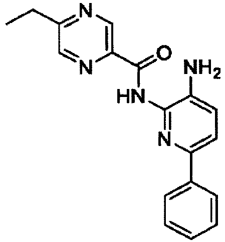 | 1.86 | 320 |

**[Table 54]**

| | | | |
|---|---|---|---|
| I-261 | 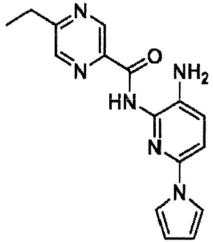 | 1.8 | 309 |
| I-262 | 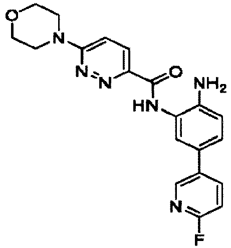 | 1.53 | 395 |
| I-263 | 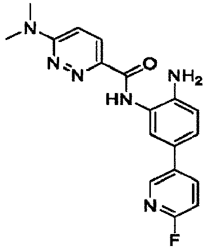 | 1.35 | 353 |
| I-264 | 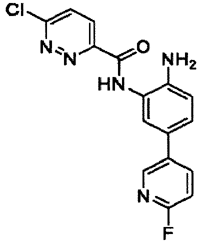 | 1.62 | 344 |
| I-265 | 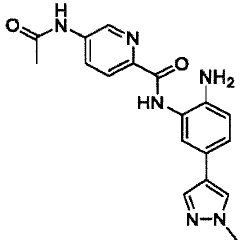 | 1.15 | 351 |

**[Table 55]**

| | | | |
|---|---|---|---|
| I-266 | 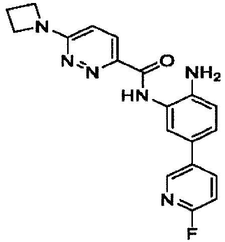 | 1.31 | 365 |
| I-267 | 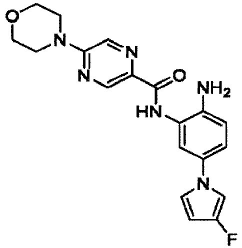 | 1.73 | 383 |
| I-268 | 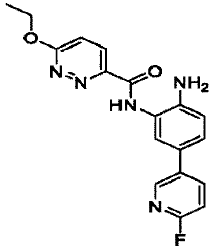 | 1.76 | 354 |
| I-269 | 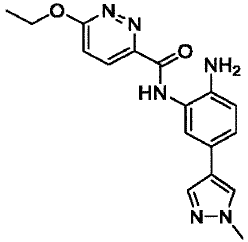 | 1.39 | 339 |
| I-270 | 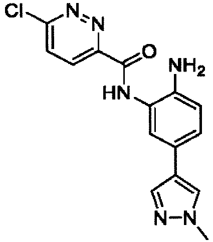 | 1.25 | 329 |

**[Table 56]**

| | | | |
|---|---|---|---|
| I-271 | 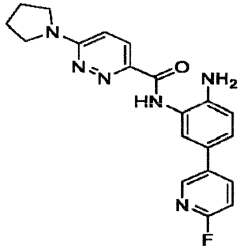 | 1.35 | 379 |
| I-272 | 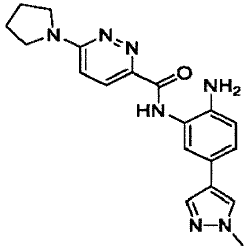 | 1.09 | 364 |
| I-273 | 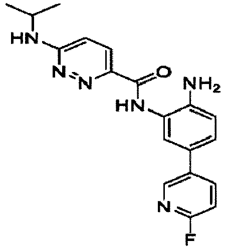 | 1.37 | 367 |
| I-274 | 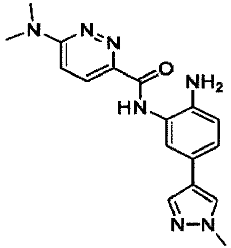 | 0.99 | 338 |
| I-275 | 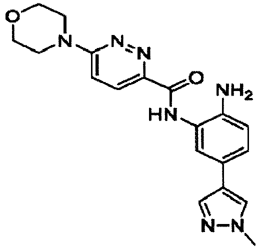 | 1.2 | 380 |

**[Table 57]**

| | | | |
|---|---|---|---|
| I-276 | 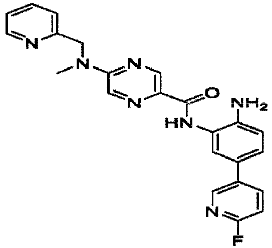 | 1.37 | 430 |
| I-277 | 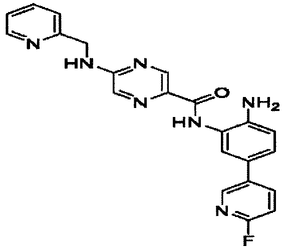 | 1.26 | 416 |
| I-278 | 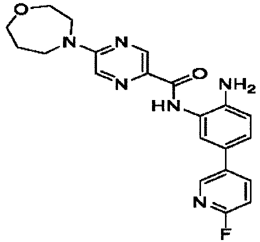 | 1.61 | 409 |
| I-279 | 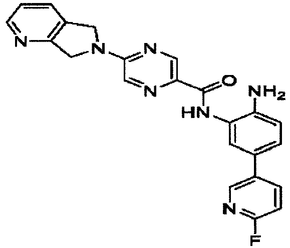 | 1.58 | 428 |
| I-280 | 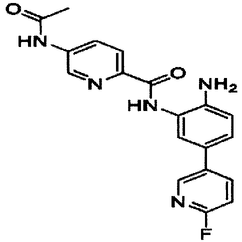 | 1.49 | 366 |

**[Table 58]**

| | | | |
|---|---|---|---|
| I-281 | 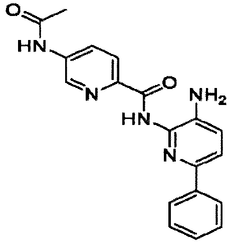 | 1.61 | 348 |
| I-282 | 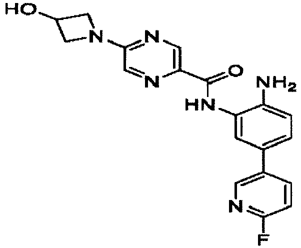 | 1.38 | 381 |
| I-283 | 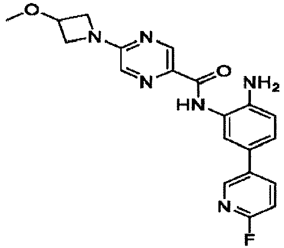 | 1.58 | 395 |
| I-284 | 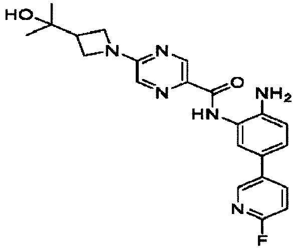 | 1.56 | 423 |
| I-285 | 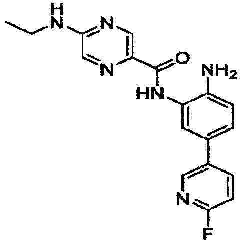 | 1.55 | 353 |

**[Table 59]**

| | | | |
|---|---|---|---|
| I-286 | 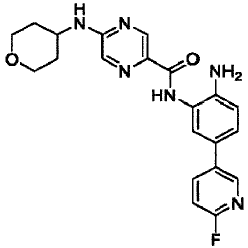 | 1.43 | 409 |
| I-287 | 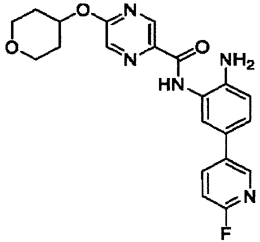 | 1.7 | 410 |
| I-288 | 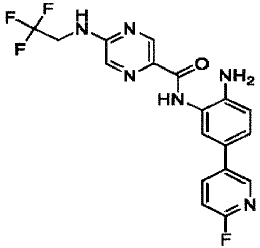 | 1.67 | 407 |
| I-289 | 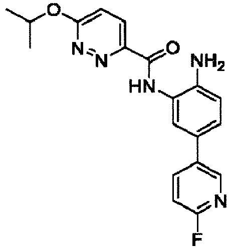 | 1.83 | 368 |
| I-290 | 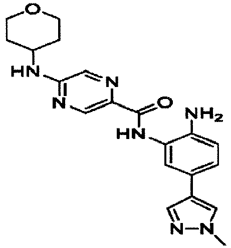 | 1.06 | 394 |

**[Table 60]**

| | | | |
|---|---|---|---|
| I-291 | 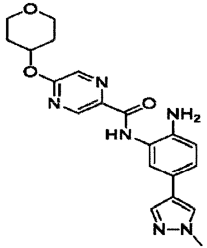 | 1.31 | 395 |
| I-292 | 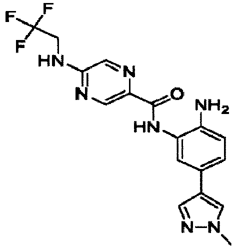 | 1.28 | 392 |
| I-293 | 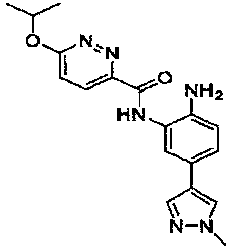 | 1.45 | 353 |
| I-294 | 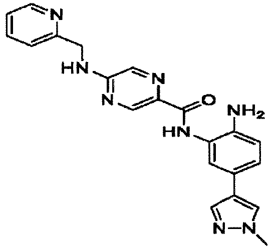 | 0.96 | 401 |
| I-295 | 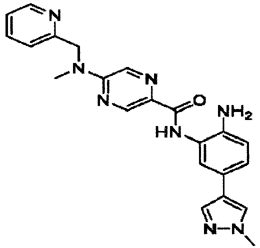 | 1.09 | 415 |

**[Table 61]**

| | | | |
|---|---|---|---|
| I-296 | 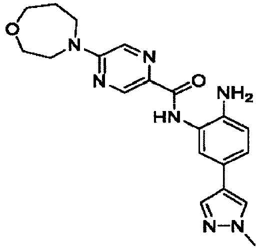 | 1.27 | 394 |
| I-297 | 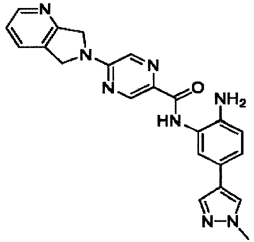 | 1.26 | 413 |
| I-298 | 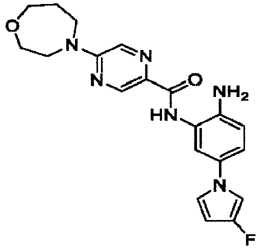 | 1.83 | 397 |
| I-299 | 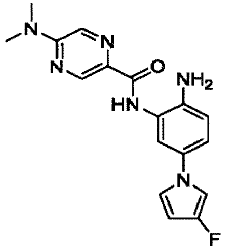 | 1.81 | 341 |
| I-300 | 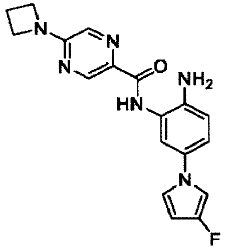 | 1.8 | 353 |

**[Table 62]**

| | | | |
|---|---|---|---|
| I-301 | 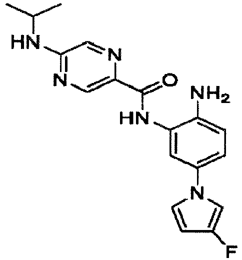 | 1.91 | 355 |
| I-302 | 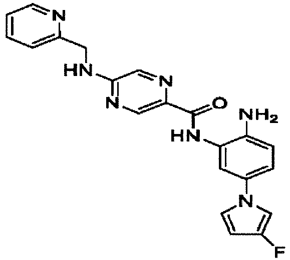 | 1.45 | 404 |
| I-303 | 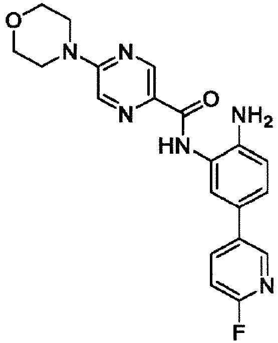 | 1.51 | 353 |

**[Table 63]**

| | | | |
|---|---|---|---|
| I-304 | 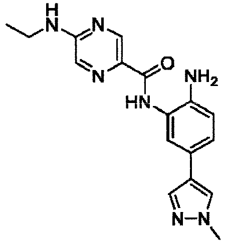 | 1.21 | 338 |
| I-305 | 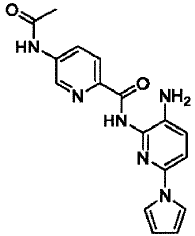 | 1.58 | 337 |
| I-306 | 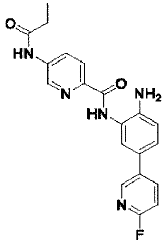 | 1.61 | 380 |
| I-307 | 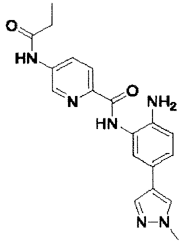 | 1.24 | 365 |
| I-308 | 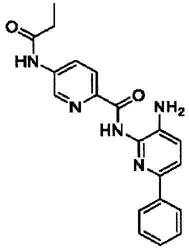 | 1.73 | 362 |

**[Table 64]**

| | | | |
|---|---|---|---|
| I-309 | 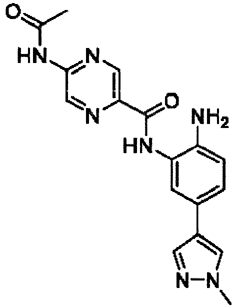 | 1.13 | 352 |
| I-310 | 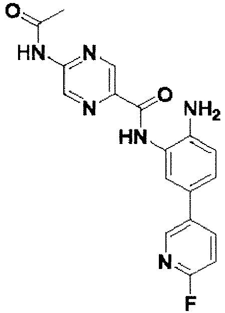 | 1.46 | 367 |
| I-311 | 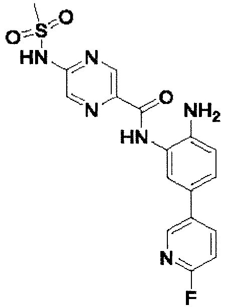 | 1.44 | 403 |
| I-312 | 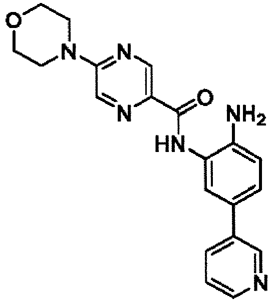 | 0.79 | 377 |

0 0 2 1

The following is a description of a biological test example of the compound of the present invention.

The compound represented by the Formula (I) according to the present invention has an HDAC2 inhibitory activity, and in the evaluation method described below, the IC50 is preferably 5000 nM or less, more preferably 1000 nM or less, still more preferably 100 nM or less.

0 0 2 2

### (TEST EXAMPLE 1: Measurement of HDAC2 enzyme inhibitory activity)

The HDAC2 enzyme inhibitory activity was measured by the method described in the following test example.

To each well of a 384-well plate (Corning, 4514), DMSO solution of the test compound was dispensed at 50nL/well from a final concentration of 99 *µ* M to 8 points (n = 2) in the 3-fold dilution series using Echo555 (Beckman Coulter). 2.5 *µ* l of 0.005 mg/mL recombinant enzyme HDAC2 (HDAC2 Human recombinant C-terminal FLAG-tag, BPS Bioscience, 50052) was added to each well, 2.5 µl of 0.6 *µ* M H3K9ac peptide (Lys(Ac)9-Histone H3 (1-21)-GGK(Biotin), AnaSpec, 64361) was added, mixed, and reacted in a wet box at room temperature for 3 hours. 50 mM Tris HCL pH8.0, 137 mM sodium chloride, 2.7 mM potassium chloride, 1 mM magnesium chloride, 0.01% Tween20, and 0.1% BSA which were diluted with ultrapure water were used as a reaction buffer at this time. After the reaction, 5 *µ* L of 10 *µ* M SAHA (suberoylanilide hydroxamic acid) as a reaction terminator and the 5µL of a mixture of 2.5ng of H3K9me0 antibody (ActiveMotif, 61399) labeled in-house with europium cryptate (Cisbio, 65EUSABA) and 125 nM streptavidin-XL665 (Cisbio, 610SAXLA) as an HTRF (time-resolved fluorescence) reagent were added to each well, mixed, and allowed to stand in a wet box at room temperature for reaction. A detection buffer used at this time was 50 mM Tris HCL pH 8.0, 0.8 M potassium fluoride, and 0.1% BSA which were diluted with ultrapure water. After reacting for 2 hours or more, HTRF was measured using a multi-plate reader such as SpectraMax Paradigm (MOLECULAR DEVICE) or RUBYstar (BMG LABTECH). Ratio (665 nm/615 - 620 nm x 10000) was calculated from the fluorescence values of two wavelengths, 615 - 620 nm and 665 nm, which were excited at 320 - 340 nm, and the energy transition was obtained. The test compound IC50 value was calculated using TIBCO Spotfire with the DMSO treatment group (n = 32) as the control (+) and the SAHA treatment group (n = 16) as the control (-).

**[Table 65]**

| Example No. | HDAC2 IC50 (uM) | Example No. | HDAC2 IC50 (uM) | Example No. | HDAC2 IC50 (uM) | Example No. | HDAC2 IC50 (uM) |
|---|---|---|---|---|---|---|---|
| I-001 | 0.056 | I-064 | 0.096 | I-121 | 0.24 | I-175 | 0.17 |
| I-002 | 0.054 | I-065 | 0.33 | I-122 | 0.25 | I-176 | 0.079 |
| I-003 | 0.31 | I-066 | 0.11 | I-123 | 0.14 | I-177 | 0.17 |
| I-004 | 0.064 | I-067 | 0.12 | I-124 | 0.16 | I-178 | 0.046 |
| I-005 | 0.22 | I-068 | 0.14 | I-125 | 0.18 | I-179 | 0.081 |
| I-006 | 0.054 | I-069 | 0.16 | I-126 | 0.14 | I-180 | 0.21 |
| I-007 | 0.096 | I-070 | 0.24 | I-127 | 0.11 | I-181 | 0.14 |
| I-008 | 0.12 | I-071 | 0.16 | I-128 | 0.18 | I-182 | 0.35 |
| I-009 | 0.061 | I-072 | 0.48 | I-129 | 0.094 | I-183 | 0.048 |
| I-010 | 0.031 | I-073 | 0.12 | I-130 | 0.14 | I-184 | 0.049 |
| I-011 | 0.057 | I-074 | 0.27 | I-131 | 0.18 | I-185 | 0.14 |
| I-012 | 0.094 | I-079 | 0.48 | I-132 | 0.43 | I-186 | 0.49 |
| I-013 | 0.1 | I-080 | 0.048 | I-133 | 0.11 | I-191 | 0.24 |
| I-014 | 0.2 | I-081 | 0.28 | I-134 | 0.21 | I-192 | 0.22 |
| I-016 | 0.16 | I-082 | 0.34 | I-135 | 0.47 | I-193 | 0.17 |
| I-017 | 0.22 | I-083 | 0.16 | I-138 | 0.2 | I-195 | 0.29 |
| I-020 | 0.26 | I-084 | 0.31 | I-139 | 0.18 | I-196 | 0.055 |
| I-021 | 0.22 | I-086 | 0.18 | I-140 | 0.22 | I-197 | 0.24 |
| I-022 | 0.47 | I-089 | 0.4 | I-141 | 0.23 | I-198 | 0.24 |
| I-023 | 0.18 | I-090 | 0.29 | I-142 | 0.18 | I-199 | 0.2 |
| I-025 | 0.37 | I-092 | 0.25 | I-143 | 0.39 | | |
| I-026 | 0.32 | I-093 | 0.13 | I-144 | 0.23 | | |
| I-027 | 0.13 | I-094 | 0.23 | I-145 | 0.34 | | |
| I-028 | 0.23 | I-096 | 0.27 | I-146 | 0.38 | | |
| I-030 | 0.19 | I-097 | 0.16 | I-147 | 0.29 | | |
| I-032 | 0.14 | I-098 | 0.24 | I-148 | 0.15 | | |
| I-033 | 0.088 | I-099 | 0.072 | I-150 | 0.31 | | |
| I-034 | 0.28 | I-100 | 0.1 | I-151 | 0.22 | | |
| I-036 | 0.16 | I-101 | 0.092 | I-152 | 0.25 | | |
| I-037 | 0.18 | I-102 | 0.45 | I-153 | 0.21 | | |
| I-039 | 0.42 | I-104 | 0.085 | I-154 | 0.12 | | |
| I-040 | 0.063 | I-108 | 0.084 | I-155 | 0.22 | | |
| I-046 | 0.054 | I-109 | 0.19 | I-161 | 0.35 | | |
| I-047 | 0.03 | I-111 | 0.15 | I-162 | 0.2 | | |
| I-056 | 0.12 | I-112 | 0.2 | I-163 | 0.21 | | |
| I-057 | 0.32 | I-113 | 0.32 | I-164 | 0.21 | | |
| I-058 | 0.13 | I-114 | 0.26 | I-165 | 0.2 | | |
| I-060 | 0.047 | I-115 | 0.44 | I-169 | 0.36 | | |
| I-061 | 0.21 | I-118 | 0.14 | I-171 | 0.43 | | |
| I-063 | 0.27 | I-120 | 0.24 | I-174 | 0.12 | | |

**[Table 66]**

| Example No. | HDAC2 IC50(uM) | Example No. | HDAC2 IC50(uM) | Example No. | HDAC2 IC50(uM) |
|---|---|---|---|---|---|
| I-201 | 0.26 | I-238 | 0.15 | I-271 | 0.09 |
| I-202 | 0.06 | I-239 | 0.23 | I-272 | 0.23 |
| I-203 | 0.24 | I-240 | 0.08 | I-273 | 0.23 |
| I-204 | 0.34 | I-241 | 0.14 | I-274 | 0.24 |
| I-205 | 0.06 | I-242 | 0.04 | I-275 | 0.26 |
| I-206 | 0.50 | I-243 | 0.26 | I-276 | 0.04 |
| I-207 | 0.25 | I-244 | 0.47 | I-277 | 0.04 |
| I-208 | 0.15 | I-245 | 0.07 | I-278 | 0.05 |
| I-209 | 0.15 | I-246 | 0.11 | I-279 | 0.05 |
| I-210 | 0.19 | I-247 | 0.38 | I-280 | 0.05 |
| I-212 | 0.83 | I-248 | 0.22 | I-281 | 0.23 |
| I-213 | 0.31 | I-249 | 0.07 | I-282 | 0.05 |
| I-216 | 0.10 | I-250 | 0.17 | I-283 | 0.05 |
| I-217 | 0.34 | I-251 | 0.11 | I-284 | 0.08 |
| I-218 | 0.15 | I-252 | 0.17 | I-285 | 0.04 |
| I-219 | 0.31 | I-253 | 0.15 | I-286 | 0.07 |
| I-220 | 0.05 | I-254 | 0.34 | I-287 | 0.17 |
| I-221 | 0.05 | I-255 | 0.41 | I-288 | 0.23 |
| I-222 | 0.08 | I-256 | 0.26 | I-289 | 0.20 |
| I-223 | 0.09 | I-257 | 0.57 | I-290 | 0.11 |
| I-224 | 0.10 | I-258 | 0.87 | I-291 | 0.31 |
| I-225 | 0.09 | I-259 | 0.34 | I-292 | 0.17 |
| I-226 | 0.45 | I-260 | 0.47 | I-293 | 0.34 |
| I-228 | 0.63 | I-261 | 0.26 | I-294 | 0.07 |
| I-229 | 0.13 | I-262 | 0.10 | I-295 | 0.08 |
| I-230 | 0.25 | I-263 | 0.12 | I-296 | 0.07 |
| I-231 | 0.85 | I-264 | 0.27 | I-297 | 0.10 |
| I-232 | 0.32 | I-265 | 0.09 | I-298 | 0.13 |
| I-233 | 0.28 | I-266 | 0.14 | I-299 | 0.12 |
| I-234 | 0.24 | I-267 | 0.10 | I-300 | 0.07 |
| I-235 | 0.06 | I-268 | 0.13 | I-301 | 0.31 |
| I-236 | 0.07 | I-269 | 0.18 | I-302 | 0.08 |
| I-237 | 0.16 | I-270 | 0.97 | I-303 | 0.07 |

Compound I-304: IC50 (*µ*M): 0.07
Compound I-305: IC50 (*µ*M): 0.13
Compound I-306: IC50 (*µ*M): 0.048
Compound I-307: IC50 (*µ*M): 0.077
Compound I-308: IC50 (*µ*M): 0.28
Compound I-309: IC50 (*µ*M): 0.12
Compound I-310: IC50 (*µ*M): 0.092
Compound I-311: IC50 (*µ*M): 0.15
Compound I-312: IC50 (*µ*M): 0.045

0 0 2 3

### (TEST EXAMPLE 2: Platelet toxicity test using human blood progenitor cells)

Human bone marrow CD34 + progenitor cells (Lonza, 2M-101B) stored at -80°C were thawed, spun down at 150 g for 10 minutes at 20°C, and a medium (StemSpan Expansion Supplement, STEM CELL TECHNOLOGIES, ST-02696) to which cytokines (StemSpan SFEM II, STEM CELL TECHNOLOGIES, ST-09655) was added was added to adjust to 2 × 10⁴/well, the mixture was seeded in 12well non-coat dish (Corning, 3513), was incubated for 10 days at 37 °C, 5% CO₂. After 10 days, the cells were collected and spun down at 150 g for 10 minutes at 20°C, and a medium (StemSpan Expansion Supplement) containing cytokines (StemSpan SFEM II) was added so that the number of cells became 2 × 10⁵ cells/ml. 50 *µ*l of the cell suspension was seeded to each well of a 96-well plate (Corning, 3596) and allowed to stand overnight in an incubator at 37°C, 5% CO₂. The next day, 50 *µ*l of the test compound was treated to each well and allowed to stand for 48 hours in the incubator at 37°C, 5% CO2 (n = 2). At this time, the test compound dissolved in 100% DMSO was serially diluted with DMSO at a 3-fold ratio, and then a medium containing cytokine was added to adjust the final concentration to 25, 8.33, 2.78, 0.93, 0.31, 0.1 *µ*M. After that, 10 *µ*l of WST (Cell count reagent SF, Nacalai Tesque Inc., 07553-44) was added to each well, and the mixture was allowed to stand in the incubator for 4 hours at 37 ° C, 5% CO2, and then the multi-label reader 2030 ARVO X4 (PerkinElmer) was used to measure the absorbance. At this time, the absorbance at 450 nm and the absorbance at 620 nm were obtained, and the difference between 450 nm and 620 nm was used as the measured value. The measured values were analyzed by Excel and TIBCO Spotfire, and IC50 values were calculated. At this time, for control (-), the well without cells was set as n=6 for one row, and the average value was calculated, and for control (+), the well in which DMSO was added to the cells so as to have the same concentration as the compound treatment group was set as n=6 for one row, and the average value was calculated and corrected for each plate. For the test compound, the average value of n=2 was used.

The experimental results were shown below.

**[Table 67]**

| Example No. | Blood toxicity IC50 (uM) | Example No. | Blood toxicity IC50 (uM) |
|---|---|---|---|
| I-003 | >25 | I-126 | >25 |
| I-006 | >25 | I-127 | >25 |
| I-017 | >25 | I-129 | >25 |
| I-023 | >25 | I-130 | >25 |
| I-028 | >25 | I-132 | >25 |
| I-036 | >25 | I-139 | >25 |
| I-080 | >25 | I-141 | >25 |
| I-092 | >25 | I-142 | >25 |
| I-093 | >25 | I-144 | >25 |
| I-112 | >25 | I-157 | >25 |
| I-118 | >25 | I-158 | >25 |
| I-121 | >25 | I-160 | >25 |
| I-122 | >25 | I-161 | >25 |
| I-124 | >25 | I-198 | >25 |
| I-125 | >25 | | |

0 0 2 4

### (TEST EXAMPLE 3: Evaluation of HDAC2 selectivity)

0 0 2 5

### TEST EXAMPLE 3-1: HDAC2 Knockout K562 Cell production method

K562 cells were cultured in RPMI1640 medium (SIGMA, R8758) containing 10% FBS (Hyclone, SH30070.03) and 1% penicillin streptomycin (Nacalai Tesque, 26252-94). CompoZr (registered trademark) Zinc Finger Nuclease (Merck) targeting HDAC2 sequences was transfected into K562 cells by electroporation using Nucleofector (Lonza). The cells after transfection were subjected to limiting dilution, and DNA sequencing was performed on the cell clones in which mutations were detected by the PCR method. Cell clones lacking a base number that was not a multiple of 3 in both alleles were identified, and a single-cell cloning was performed on the cell clones to produce HDAC2 knockout K562 cells. It was confirmed that the expression of HDAC2 was not detected by Western blotting in the prepared cells.

0 0 2 6

### TEST EXAMPLE 3-2: Detection of H3K9 histone acetylation (AlphaLISA method)

By evaluating the H3K9 histone acetylation-enhancing activity of the test compound using HDAC2 knockout K562 cells, the inhibitory activity against HDACs other than HDAC2 was evaluated.

HDAC2 knockout K562 cells stored in liquid nitrogen were thawed and cultured in RPMI1640 medium (Nacalai Tesque, 30264-85) for 2 to 4 days. Using Echo555 (Beckman Coulter), the test compound was dispensed to the assay plate (Corning, 4513) at 37.5 nL/well, and then HDAC2 knockout K562 cells were seeded to 125 cells/4.5 *µ*L/well, and stirred. At this time, the test compound was treated at 8 points (n = 2) in the 3-fold dilution series from the maximum concentration of 83.3 *µ* M. After culturing at 37°C and 5% CO₂ overnight, 1.5 *µ*L/well of Lysis buffer (PerkinElmer, AL009F1) was added, and after incubating at room temperature for 15 minutes, 3 *µ*L/well of Extraction buffer (PerkinElmer, AL009F2) was added and the mixture was incubated for 10 minutes at room temperature. Then, 5x antibody solution (prepared by diluting anti-H3K9 Acceptor beads (PerkinElmer, AL114M) 75-fold with a detection buffer prepared by diluting 10×Cell-Histone Detection buffer (PerkinElmer, AL009F3) 10-fold with ultrapure water, and diluting Biotinylated anti H3 antibody (PerkinElmer, AL118M) diluted 150 times with the detection buffer) was added at 3 µL/well, and after incubating at room temperature for 60 minutes, then 5x donor beads solution (prepared by diluting Streptavidin Donor beads (PerkinElmer, 6760002) 75 fold with the detection buffer) was added at 3 *µ*L/well, and the mixture was incubated again at room temperature for 60 minutes, and then the emission value was measured using PHERAstar (BMG LABTECH) (excitation = 680 nm, emission = 570 nm). The maximum inhibition rate of the test compound was calculated with 0% inhibition in the DMSO-treated group (n = 16) and 100% inhibition in the 30 *µ*M SAHA-treated group (n = 35). For cell toxicity, cell viability rate was evaluated by the PrestoBlue method.

0 0 2 7

The experimental results were shown below.
Compound I-017: 5%
Compound I-023: 8%
Compound I-028: 8%
Compound I-030: 9%
Compound I-036: 8%
Compound I-080: 7%
Compound I-092: 6%
Compound I-118: 8%
Compound I-122: 3%
Compound I-124: 9%
Compound I-125: 6%
Compound I-126: 5%
Compound I-127: 7%
Compound I-132: 6%
Compound I-141: 6%
Compound I-142: 8%
Compound I-144: 7%
Compound I-157: 7%
Compound I-158: 7%
Compound I-160: 6%
Compound I-161: 9%
Compound I-179: 9%
Compound I-198: 5%
Compound I-250: 5%
Compound I-254: 3%
Compound I-280: 8%

0 0 2 8

### TEST EXAMPLE 4: In vivo acetylation evaluation method

The target tissue was cut out from the animal to which each compound was administered, and immediately frozen in liquid nitrogen. The tissues were homogenized in a mixed extract of 15 mM HEPES (pH 7.5), 100 mM NaCl, 0.1% NP-40, protease inhibitor cocktail using a Douncns homogenizer, permeated through a cell strainer, and then the supernatant was removed using a centrifuge. After adding the extract again and washing the pellet, 20 mM Tris-HCl (pH 8.0), 100 mM NaCl, 1 mM EDTA (pH 8.0), 1% Triton-X 100, 1% SDS, protease inhibitor cocktail was added to the pellet, the mixed solution was sonicated using an ultrasonic homogenizer (BRANSON). BCA protein assay (Thermo Fisher Scientific) was performed on the obtained sample to quantify the amount of protein. Acetylated histones in the sample were detected by Wes (Bio-Techne) using anti-acetyl histone antibodies.

### (TEST EXAMPLE 5: CYP inhibition test)

Using commercially available pooled human liver microsomes, and using 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation (CYP3A4), which are typical substrate metabolic reactions of human main five CYP isoforms (CYP1A2, 2C9, 2C19, 2D6, and 3A4), as indicators, an inhibitory degree of each metabolite production amount by the compound of the present invention is assessed.

The reaction conditions are as follows: substrate, 0.5 µmol/L ethoxyresorufin (CYP1A2), 100 µmol/L tolbutamide (CYP2C9), 50 µmol/L S-mephenitoin (CYP2C19), 5 µmol/L dextromethorphan (CYP2D6), 1 µmol/L terfenedine (CYP3A4); reaction time, 15 minutes; reaction temperature, 37°C; enzyme, pooled human liver microsomes 0.2 mg protein/mL; concentrations of the compound of the present invention, 1, 5, 10, 20 µmol/L (four points).

Each five kinds of substrates, human liver microsomes, and compound of the present invention in 50 mmol/L Hepes buffer are added to a 96-well plate at the composition as described above, and NADPH, as a cofactor is added to initiate metabolism reactions. After the incubation at 37°C for 15 minutes, a methanol/acetonitrile = 1/1 (v/v) solution is added to stop the reaction. After the centrifugation at 3000 rpm for 15 minutes, resorufin (CYP1A2 metabolite) in the supernatant is quantified by a fluorescent multilabel counter or LC/MS/MS and hydroxytolbutamide (CYP2C9 metabolite), 4' hydroxymephenytoin (CYP2C19 metabolite), dextrorphan (CYP2D6 metabolite), and terfenadine alcohol metabolite (CYP3A4 metabolite) are quantified by LC/MS/MS. The dilution concentration or the dilution solvent are changed as necessary.

The sample adding only DMSO as a solvent to a reaction system instead of a solution dissolving a compound of the present invention is adopted as a control (100%). Remaining activity (%) is calculated and IC₅₀ is calculated by reverse presumption by a logistic model using a concentration and an inhibition rate.

### (TEST EXAMPLE 6: CYP3A4 (MDZ) MBI test)

CYP3A4 (MDZ) MBI test is a test of investigating mechanism based inhibition potential on CYP3A4 by the enhancement of inhibitory activity caused by a metabolic reaction of the compound of the present invention. CYP3A4 inhibition was evaluated using pooled human liver microsomes by 1-hydroxylation reaction of midazolam (MDZ) as a marker reaction.

The reaction conditions are as follows: substrate, 10 µmol/L MDZ; pre-reaction time, 0 or 30 minutes; substrate reaction time, 2 minutes; reaction temperature, 37°C; pooled human liver microsomes, at pre-reaction time 0.5 mg/mL, at reaction time 0.05 mg/mL (at 10-fold dilution); concentrations of the compound of the present invention at pre-reaction time, 1, 5, 10, 20 µmol/L (four points).

Pooled human liver microsomes and a solution of the compound of the present invention in K-Pi buffer (pH 7.4) as a pre-reaction solution were added to a 96-well plate at the composition of the pre-reaction. A part of pre-reaction solution was transferred to another 96-well plate, and 1/10 diluted by K-Pi buffer containing a substrate. NADPH as a co-factor was added to initiate a reaction as a marker reaction (preincubation 0 min). After a predetermined time of a reaction, methanol/acetonitrile=1/1 (v/v) solution was added to stop the reaction. After centrifuged at 3000 rpm for 15 minutes, 1-hydroxymidazolam in the supernatant was quantified by LC/MS/MS. The dilution concentration or the dilution solvent were changed as necessary.

The sample adding DMSO as a solvent to a reaction system instead of a solution dissolving the compound of the present invention was adopted as a control (100 %). Remaining activity (%) was calculated at each concentration of the compound of the present invention compared to a control, and IC value was calculated by reverse-presumption by a logistic model using a concentration and an inhibition rate. Shifted IC value was calculated as "IC of preincubation at 0 min/ IC of preincubation at 30min". When a shifted IC was 1.5 or more, this was defined as positive. When a shifted IC was 1.0 or less, this was defined as negative.

The experimental results were shown below.
Compound I-080: negative
Compound I-092: negative
Compound I-118: negative
Compound I-125: negative
Compound I-130: negative
Compound I-148: negative
Compound I-152: negative
Compound I-155: negative
Compound I-177: negative
Compound I-178: negative
Compound I-179: negative

### (TEST EXAMPLE 7: BA test)

Materials and methods for experiments to evaluate oral absorption
(1) Experimental animals: mise or rats were used.
(2) Rearing condition: mice or rats were allowed free access to solid feed and sterilized tap water.
(3) Setting of dosage and grouping: Oral administration and intravenous administration were performed with the predetermined dosage. Grouping was set as below. (Dosage was changed per compound)
   Oral administration 2 to 60 µmol/kg or 1 to 30 mg/kg (n = 2 to 3)
   Intravenous administration 1 to 30 µmol/kg or 0.5 to 10 mg/kg (n = 2 to 3)
(4) Preparation of administration solutions: Oral administration was performed as suspension or solution. Intravenous administration was performed after solubilization.
(5) Routes of administration: Oral administration was performed mandatory into the stomach by oral sonde. Intravenous administration was performed from caudal vein by syringes with needle.
(6) Evaluation items: Blood was collected serially and concentration of a compound of the present invention in plasma was measured by LC/MS/MS.
(7) Statistical analysis: About transition of concentration of a compound of the present invention in plasma, the area under the plasma concentration versus time curve (AUC) was calculated by non-linear least-squares method program, and bioavailability (BA) of a compound of the present invention was calculated from dosages and AUCs of the oral administration group and the intravenous administration group. The dilution concentration or the dilution solvent were changed as necessary.

The experimental results were shown below.
Compound I-003: 33.8%
Compound I-006: 35.6%
Compound I-017: 19.6%
Compound I-023: 43.7%
Compound I-080: 38.4%
Compound I-092: 22.4%
Compound I-118: 22.7%
Compound I-125: 32.1%
Compound I-130: 37.2%

### (TEST EXAMPLE 8: Clearance test)

### Materials and methods for experiments

(1) Experimental animals: SD rats were used.
(2) Rearing condition: SD rats were allowed free access to solid feed and sterilized tap water.
(3) Setting of dosage and grouping: Intravenous administration is performed with the predetermined dosage. Grouping is set as below.
   Intravenous administration 1 µmol/kg (n = 2)
(4) Preparation of administration solutions: Administration is performed after solubilization by using dimethyl sulfoxide/propylene glycol = 1/1 as the solvent.
(5) Routes of administration: Intravenous administration is performed from caudal vein by syringes with needle.
(6) Evaluation items: Blood is collected serially and concentration of a compound of the present invention in plasma is measured by LC/MS/MS.
(7) Statistical analysis: About transition of concentration of a compound of the present invention in plasma, Total Clearance (CLtot) of a compound of the present invention is calculated by the moment analysis method. The dilution concentration or the dilution solvent are changed as necessary.

### (TEST EXAMPLE 9: Metabolism Stability Test)

Using commercially available pooled human hepatic microsomes, a compound of the present invention is reacted for a constant time, and a remaining rate is calculated by comparing a reacted sample and an unreacted sample, thereby, a degree of metabolism in liver is assessed.

Using commercially available pooled human hepatic microsomes, a compound of the present invention is reacted for a constant time, and a remaining rate is calculated by comparing a reacted sample and an unreacted sample, thereby, a degree of metabolism in liver is assessed.

A reaction is performed (oxidative reaction) at 37 °C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution is added to 100 µL of a methanol/acetonitrile = 1/1 (v/v), mixed and centrifuged at 3000 rpm for 15 minutes. The compound of the present invention in the supernatant is quantified by LC/MS/MS or Solid Phase Extraction (SPE)/MS, and a remaining amount of the compound of the present invention after the reaction is calculated, letting a compound amount at 0 minute reaction time be 100%. The hydrolysis reaction is carried out in the absence of NADPH, and the glucuronidation reaction is carried out in the presence of 5 mmol / L UDP-glucuronic acid instead of NADPH, and the same operation is carried out thereafter. The dilution concentration and dilution solvent are changed as necessary.

The experimental results were shown below.
Compound I-017: 81.8%
Compound I-023: 95.3%
Compound I-030: 101%
Compound I-080: 84.8%
Compound I-092: 103%
Compound I-125: 80.4%
Compound I-130: 92.9%
Compound I-148: 94.9%
Compound I-152: 97.9%
Compound I-169: 91.4%
Compound I-177: 92%
Compound I-179: 93.5%

### (TEST EXAMPLE 10: Fluctuation Ames Test)

Mutagenicity of compounds of the present invention is evaluated.

A 20 µL of freezing-stored Salmonella typhimurium (TA98 strain, TA100 strain) is inoculated on 10 mL of a liquid nutrient medium (2.5% Oxoid nutrient broth No.2), and this is incubated at 37°C for 10 hours under shaking. The 7.70 to 8.00 mL of TA98 bacterial solution is centrifuged (2000 × g, 10 minutes) and TA98 is suspended in Micro F buffer (K₂HPO₄: 3.5 g/L, KH₂PO₄: 1 g/L, (NH₄)₂SO₄: 1 g/L, trisodium citrate dehydrate: 0.25 g/L, MgSO₄ • 7H₂O: 0.1 g/L) with the same volume as the bacterial solution used for centrifugation after removing the culture medium. The TA98 suspension is mixed with 120 mL Exposure medium (Micro F buffer containing Biotin: 8 µg/mL, histidine: 0.2 µg/mL, glucose: 8 mg/mL). The 3.10 to 3.42 mL of TA100 bacterial solution is mixed with 120 to 130 mL Exposure medium to prepare a test bacterial suspension. Each 12 µL of DMSO solution of the compound of the present invention (several stage dilution from maximum dose 50 mg/mL at 2 to 3 fold ratio), DMSO as a negative control, and 50 µg/mL of 4-nitroquinoline 1-oxide DMSO solution for the TA98 strain and 0.25 µg/mL of 2-(2-furyl)-3-(5-nitro-2-furyl)acrylamide DMSO solution for the TA100 strain in the assay without metabolic activation, 40 µg/mL of 2-aminoanthracene DMSO solution for the TA98 strain and 20 µg/mL of 2-aminoanthracene DMSO solution for the TA100 strain in the assay with metabolic activation as a positive control, and 588 µL of the test bacterial suspension (498 µL and 90 µL of S9 mixture in the case of metabolic activation assay) are mixed, and this is incubated at 37°C for 90 minutes under shaking. A 460 µL of the mixture is mixed with 2300 µL of Indicator medium (Micro F buffer containing 8 µg/mL biotin, 0.2 µg/mL histidine, 8 mg/mL glucose, 37.5 µg/mL bromocresol purple), each 50 µL is dispensed to microplate 48 wells/dose, and this is incubated at 37°C for 3 days.

Since the wells containing the bacteria which gained growth ability by point mutation in amino acid (histidine) synthesizing enzyme gene turns from purple to yellow due to a pH change, the number of yellow wells in 48 wells is counted per dose, and is compared with the negative control group. (-) means negative in mutagenicity and (+) means positive in mutagenicity respectively. The dilution concentration and dilution solvent are changed as necessary.

### (TEST EXAMPLE 11: hERG Test)

For the purpose of assessing risk of an electrocardiogram QT interval prolongation of the compound of the present invention, effects of the compound of the present invention on delayed rectifier K⁺ current (I_{Kr}), which plays an important role in the ventricular repolarization process, was studied using CHO cells expressing human ether-a-go-go related gene (hERG) channel.

After a cell was retained at a membrane potential of -80 mV by whole cell patch clamp method using an automated patch clamp system (QPatch; Sophion Bioscience A/S) and gave a leak potential of -50 mV, IKr induced by depolarization pulse stimulation at +20 mV for 2 seconds, and further, repolarization pulse stimulation at -50 mV for 2 seconds, was recorded. A vehicle, which was the 0.1% dimethyle sulfoxide solution in extracellular solution (NaCl: 145 mmol/L, KCl: 4 mmol/L, CaCl₂: 2 mmol/L, MgCl₂: 1 mmol/L, glucose:10 mmol/L, HEPES (4-(2-hydroxyethyl)-1-piperazineethanesulfonic acid): 10 mmol/L, pH7.4), or the compound of the present invention had been dissolved at an objective concentration in the extracellular solution was applied to the cell at room temperature for 7 minutes or more. From the recording I_{Kr}, an absolute value of the tail peak current was measured based on the current value at the resting membrane potential using analysis software (QPatch assay software; Sophion Bioscience A/S). Further, the % inhibition of tail peak current for the compound of the present invention relative to the tail peak current after application of the vehicle was calculated to assess influence of the compound of the present invention on I_{Kr}. The dilution concentration and dilution solvent were changed as necessary.

The experimental results were shown below.
Compound I-003: 17.1%
Compound I-006: 14.3%
Compound I-003: 17.1%
Compound I-080: 9.5%
Compound I-092: 30%
Compound I-118: 23.3%
Compound I-125: 18.6%
Compound I-130: 3.3%

### (TEST EXAMPLE 12: Solubility test)

The solubility of the compound of the present invention is determined under 1% DMSO addition conditions. A 10 mmol/L solution of the compound is prepared with DMSO, and 2 µL of the solution of the compound of the present invention is added to 198 µL of JP-1 fluid and JP-2 fluid respectively. The mixture is shaken for 1 hour at room temperature, and the mixture is filtered under suction. The filtrate is ten or hundred-fold diluted with methanol/water = 1/1(v/v) or acetonitrile/methanol/water=1/1/2(V/V/V) and the compound concentration in the filtrate is measured with LC/MS or Solid Phase Extraction (SPE)/MS by the absolute calibration method. The dilution concentration and dilution solvent are changed as necessary.

### The composition of JP-1 fluid is as follows.

2.0 g of sodium chloride and 7.0 mL of hydrochloric acid are dissolved in water to reach 1000 mL.

The composition of JP-2 fluid is as follows.

1 volume of water is added 1 volume of the solution that 3.40g of potassium dihydrogen phosphate and 3.55g of sodium dihydrogen phosphate anhydrous are dissolved in water to be 1000mL.

### (TEST EXAMPLE 13: Powder solubility test)

Appropriate quantity of the compound of the present invention is put in a suitable container and 200 µL of JP-1 fluid (2.0 g of sodium chloride and 7.0 mL of hydrochloric acid are dissolved in water to reach 1000 mL) JP-2 fluid (1 volume of water is added to 1 volume of the solution in which 3.40 g of potassium dihydrogen phosphate and 3.55 g of anhydrous disodium hydrogen phosphate are dissolved in water to reach 1000 mL) or 20 mmol/L sodium taurocholate (TCA)/JP-2 fluid (JP2 fluid is added to 1.08g of TCA to reach 100mL) is independently added to each container. When total amount is dissolved after adding the test reagent, the compound of the present invention is added appropriately. After sealing and shaking at 37 °C for 1 hour, solution is filtrated and 100 µL of methanol is added to 100 µL of each filtrate to dilute two-fold. The dilution rate is changed as necessary. After checking that there is no bubble and precipitate, the container is sealed and shaken. The compound of the present invention is measured using HPLC by absolute calibration curve method. The dilution concentration and dilution solvent are changed as necessary.

### (TEST EXAMPLE 14: Ames Test)

The mutagenicity of the compound of the present invention is evaluated by an Ames test using Salmonella typhimurium TA98 strain, TA100 strain, TA1535 strain, TA1537 strain and Escherichia coli WP2uvrA strain as test strains. 0.5 mL of S9mix under metabolic activation conditions, 0.5 mL of phosphate buffer under nonmetabolic activation conditions is mixed with 0.1 mL of DMSO solution of the compound of the present invention, and layered on a minimal glucose agar plate with 2 mL of soft agar containing histidine and biotin or tryptophan for multiple layers. At the same time, the same procedure is carried out for negative control substances (DMSO) and positive control substances (2-(2-furyl)-3-(5-nitro-2-furyl)acrylamide, sodium azide, 9-aminoacridine, or 2-aminoanthracene). After incubating at 37°C for 48 hours, the emerging reverse mutation colonies are counted and evaluated in comparison with the negative control group. When the number of reverse mutation colonies increases in a concentration-dependent manner and becomes twice the number of colonies in the negative control group or more than twice the number of colonies in the negative control group, it is judged as positive (+). The dilution concentration and the dilution solvent are changed as necessary.

0 0 2 9

### (TEST EXAMPLE 15: Photohemolysis test)

The compound of the present invention is dissolved at a desired concentration, mixed with 0.1 to 0.0008% of an erythrocyte suspension (2.5 v/v%) prepared from sheep defibrillated blood on a microplate, and light irradiation (10 J / cm², 290 to 400 nm) in the UVA and UVB regions was performed using an ultraviolet fluorescent lamp (GL20SE lamp, Sankyo Electric and FL20S-BLB lamp, Panasonic). After the light irradiation is completed, the mixed solution is collected and centrifuged. After centrifuging, the supernatant is collected and transferred to a microplate, and then the absorbance (540 or 630 nm) of the supernatant is measured and a judgment is made based on the absorbance. Absorbances at 540 and 630 nm are indicators of biological membrane damage (photohemolysis rate%) and lipid membrane peroxidation (methemoglobin production), respectively. It is judged to be (-) when the photohemolysis rate is less than 10% and the change in the absorbance at 630 nm was less than 0.05. It is judged to be (+) when the photohemolysis rate is 10% or more and the change in the absorbance at 630 nm is 0.05 or more.

0 0 3 0

### (TEST EXAMPLE 16: P-gp substrate test)

The compound of the present invention is added to one side of a transwell (registered trademark, CORNING) in which human MDR1-expressing cells or parent cells are cultured in a single layer, and reacted for a certain period of time. For MDR1-expressing cells and parent cells, the membrane permeability coefficients from the Apical side to the Basolaternal side (A→B) and from the Basolaternal side to the Apical side (B→A) are calculated, and Efflux Ratio (ER; Ratio of membrane permeability coefficients of B→A and A→B) value of the MDR1-expressing cells and the parent cells are calculated. The Efflux Ratio (ER value) of the MDR1-expressing cell and the parent cell are compared to determine whether the compound of the present invention is a P-gp substrate or not.

The compound of the present invention can be administered as a pharmaceutical composition by any conventional route, in particular enterally, for example, orally, for example, in the form of tablets or capsules, or parenterally, for example, in the form of injectable solutions or suspensions, topically, for example, in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets, granules, or capsules containing excipients, disintegrants, binders, lubricants and the like and active ingredients. Compositions for injection can be solutions or suspension, may be sterilized, and may contain preservatives, stabilizers, buffering agents, and the like.

### [INDUSTRIAL APPLICABILITY]

The compounds of the present invention have HDAC2 inhibitory activity, are considered to be useful as a therapeutic and/or prophylactic agent for a disease or condition associated with HDAC2.

## Claims

1. A compound represented by Formula (I): 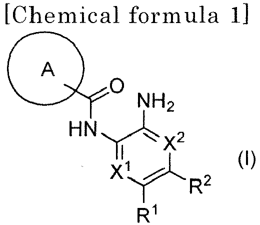 wherein the group represented by Formula: 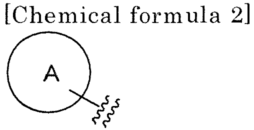 is a group represented by Formula: 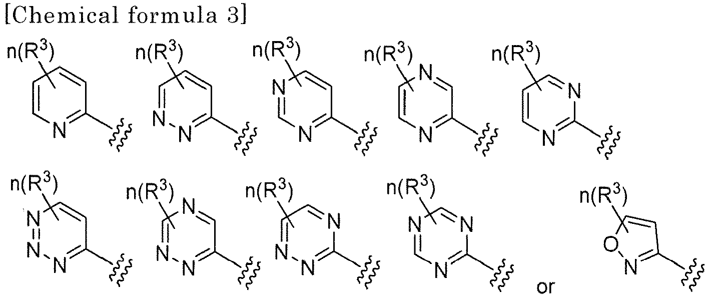 wherein R³ is each independently substituted with Substituent group D or unsubstituted amino, halogen, cyano, hydroxy, substituted with Substituent group A or unsubstituted alkyl, substituted with Substituent group A or unsubstituted alkenyl, substituted with Substituent group A or unsubstituted alkynyl, substituted with Substituent group A or unsubstituted alkyloxy, substituted with Substituent group A or unsubstituted alkyloxycarbonyl, substituted with Substituent group A or unsubstituted alkylcarbonyloxy, substituted with Substituent group B or unsubstituted monocyclic non-aromatic carbocyclyl, substituted with Substituent group B or unsubstituted monocyclic non-aromatic heterocyclyl (provided that piperazinyl and methylpiperazinyl are excluded), substituted with Substituent group B or unsubstituted monocyclic aromatic carbocyclyl, or substituted with Substituent group B or unsubstituted monocyclic aromatic heterocyclyl,
n is an integer from 0 to 4, and
two R³s bonded to an adjacent carbon atom may be taken together to form (a) a non-aromatic carbocycle optionally substituted with halogen, (b) a non-aromatic heterocycle optionally substituted with one or more substituents selected from the group consisting of halogen; alkyl; and oxo, (c) an aromatic carbocycle optionally substituted with halogen, or (d) an aromatic heterocycle optionally substituted with halogen,
provided that when R¹ is substituted with Substituent group C or unsubstituted thienyl, (i) R³ is not alkyloxyalkyl and hydroxyalkyl, or (ii) two R³s bonded to adjacent carbon atoms is not taken together to form the rings (a) to (d) above,
Substituent group A: halogen, cyano, hydroxy, amino, alkylamino, alkyloxy, a non-aromatic carbocycle, and a non-aromatic heterocycle optionally substituted with oxo,
Substituent group B: oxo, halogen, cyano, hydroxy, alkyl, haloalkyl, hydroxyalkyl, oxo non-aromatic heterocyclylalkyl, alkenyl, alkynyl, alkyloxy, haloalkyloxy, non-aromatic carbocyclylalkyloxy, non-aromatic heterocyclyloxy, amino, and alkylamino,
Substituent group D: alkyl, haloalkyl, aromatic carbocyclylalkyl, aromatic heterocyclylalkyl, non-aromatic carbocyclylalkyl, non-aromatic heterocyclylalkyl, aromatic carbocyclyl, aromatic heterocyclyl, non-aromatic carbocyclyl, non-aromatic heterocyclyl, and alkylcarbonyl,
or the group represented below: 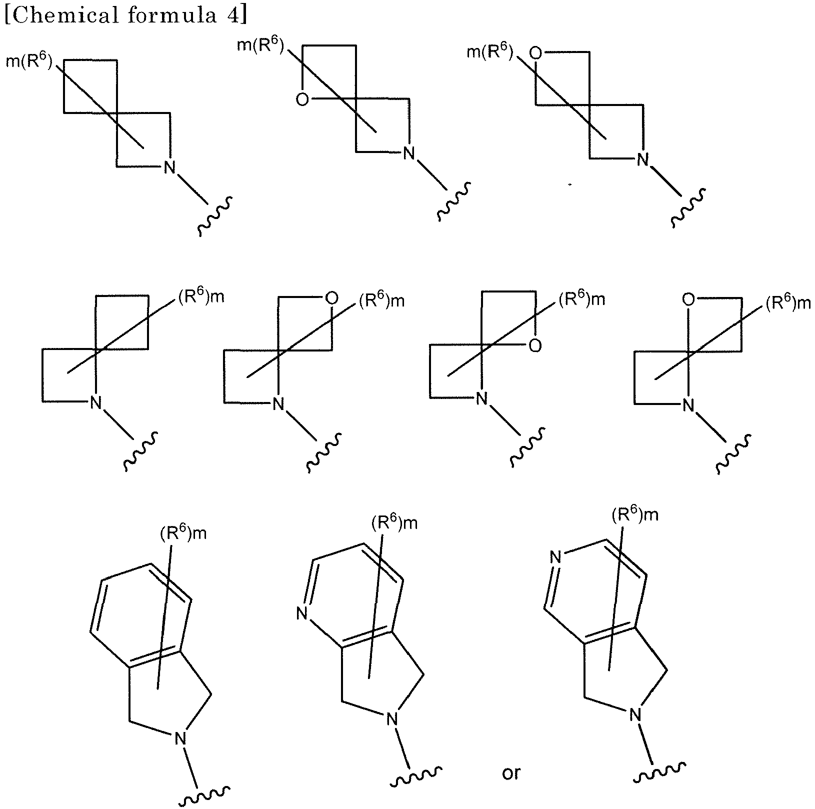
wherein R⁶ is each independently halogen, cyano, hydroxy, alkyl, haloalkyl, alkyloxy, haloalkyloxy, amino, or alkylamino, and
m is an integer from 0 to 2,
X¹ is N or CR⁴,
X² is N or CR⁵,
R⁴ and R⁵ are each independently a hydrogen atom, halogen, alkyl, haloalkyl, alkyloxy or haloalkyloxy,
R¹ is substituted with Substituent group C or unsubstituted non-aromatic carbocyclyl, substituted with Substituent group C or unsubstituted non-aromatic heterocyclyl, substituted with Substituent group C or unsubstituted aromatic carbocyclyl, or substituted with Substituent group C or unsubstituted aromatic heterocyclyl,
provided that when R¹ is substituted with Substituent group C or unsubstituted aromatic carbocyclyl, either X1 or X2 is N,
Substituent group C: halogen, cyano, hydroxy, alkyl, haloalkyl, alkyloxy, haloalkyloxy, amino and alkylamino, and
R² is a hydrogen atom, halogen, alkyl, haloalkyl, alkyloxy or haloalkyloxy,
when R¹ is substituted with Substituent group C or unsubstituted aromatic carbocyclyl, a substituent on the aromatic carbocyclyl and R² may be taken together to form a substituted or unsubstituted carbocycle or a substituted or unsubstituted heterocycle,
provided that the following compound is excluded: 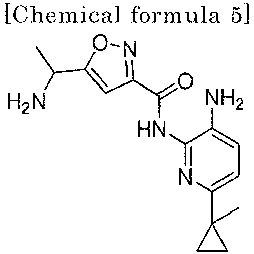 or a pharmaceutically acceptable salt thereof.

2. The compound according to Claim 1, wherein R¹ is substituted with Substituent group C or unsubstituted aromatic carbocyclyl or substituted with Substituent group C or unsubstituted aromatic heterocyclyl,
or a pharmaceutically acceptable salt thereof.

3. The compound according to Claim 1, wherein R¹ is substituted with Substituent group C or unsubstituted aromatic heterocyclyl,
or a pharmaceutically acceptable salt thereof.

4. The compound according to Claim 1, wherein R¹ is substituted with Substituent group C or unsubstituted pyridyl or substituted with Substituent group C or unsubstituted imidazolyl,
or a pharmaceutically acceptable salt thereof.

5. The compound according to any one of claims 1 to 4, wherein R² is a hydrogen atom,
or a pharmaceutically acceptable salt thereof.

6. The compound according to any one of claims 1 to 5, wherein X² is CR⁵, or a pharmaceutically acceptable salt thereof.

7. The compound according to any one of claims 1 to 6, wherein X¹ is CR⁴, or a pharmaceutically acceptable salt thereof.

8. The compound according to any one of claims 1 to 7, wherein each of R⁴ and R⁵ is a hydrogen atom,
or a pharmaceutically acceptable salt thereof.

9. The compound according to any one of claims 1 to 8, wherein n is 0 or 1, or a pharmaceutically acceptable salt thereof.

10. The compound according to any one of claims 1 to 8, wherein the group represented by Formula: 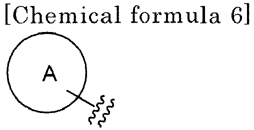 is a group represented by Formula: 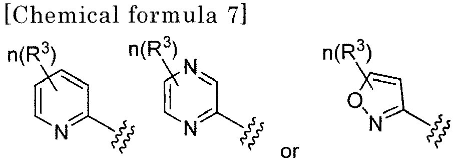 wherein n is an integer of 0 or 1, and the other symbols have the same meaning as Claim 1,
or a pharmaceutically acceptable salt thereof.

11. The compound according to any one of claims 1 to 8, wherein the group represented by Formula: 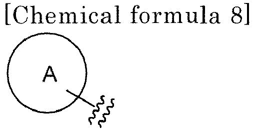 is a group represented by Formula: 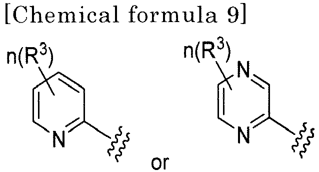 wherein n is an integer of 0 or 1, and the other symbols have the same meaning as Claim 1,
or a pharmaceutically acceptable salt thereof.

12. The compound according to any one of claims 1 to 8, wherein the group represented by Formula: 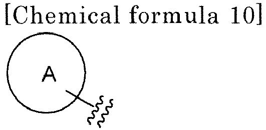 is a group represented by Formula: 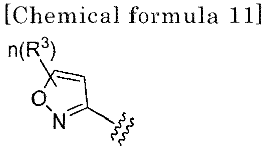 wherein n is an integer of 0 or 1, and the other symbols have the same meaning as Claim 1,
or a pharmaceutically acceptable salt thereof.

13. The compound according to any one of claims 1 to 12, wherein R³ is each independently cyano, alkyl, haloalkyl, alkyloxy, haloalkyloxy, alkyloxyalkyloxy, non-aromatic carbocyclyl, non-aromatic heterocyclyl, or alkyl aromatic heterocyclyl, or a pharmaceutically acceptable salt thereof.

14. The compound according to any one of claims 1 to 13, wherein R³ is each independently cyano, alkyl, haloalkyl, alkyloxy, haloalkyloxy, alkyloxyalkyloxy, non-aromatic carbocyclyl, or alkyl aromatic heterocyclyl,
or a pharmaceutically acceptable salt thereof.

15. A pharmaceutical composition comprising the compound according to any one of claims 1 to 14, or a pharmaceutically acceptable salt thereof.

16. The pharmaceutical composition according to Claim 15, wherein the composition is an HDAC2 inhibitor.

17. An HDAC2 inhibitor comprising the compound according to any one of claims 1 to 14, or a pharmaceutically acceptable salt thereof.
